# EUROPEAN PATENT SPECIFICATION

(11) **EP 2 841 068 B1**
(45) Date of publication and mention of the grant of the patent: **28.11.2018**
(21) Application number: 13722318.6
(22) Date of filing: 23.04.2013
(51) Int. Cl.: A61K 31/4412, A61K 31/53

(54) **CRHR1 ANTAGONISTS FOR USE IN THE TREATMENT OF PATIENTS HAVING CRH OVERACTIVITY**
CRHR1-ANTAGONISTEN ZUR VERWENDUNG BEI DER BEHANDLUNG VON PATIENTEN MIT CRH-ÜBERAKTIVITÄT
ANTAGONISTES DE CRHR1 POUR UNE UTILISATION DANS LE TRAITEMENT DE PATIENTS PRÉSENTANT UNE HYPERACTIVITÉ DE LA CRH

(30) Priority: 23.04.2012 GB 201207102; 23.04.2012 EP 12165231
(43) Date of publication of application: 04.03.2015
(73) Proprietor: HMNC Value GmbH, 80807 München (DE)
(72) Inventor: HOLSBOER, Florian, 80805 Munich (DE)
(74) Representative: Maiwald Patent- und Rechtsanwaltsgesellschaft mbH
(86) International application number: PCT/EP2013/058413
(87) International publication number: WO 2013/160317

(56) References cited:
- WO-A1-95/33750
- WO-A1-02/072202
- WO-A1-2004/094420
- WO-A1-2007/137227
- US-A- 6 060 478
- US-A- 6 107 301
- US-A1- 2007 281 919
- US-A1- 2008 194 589
- US-B1- 7 067 664
- LIU Z ET AL: "Association of corticotropin-releasing hormone receptor1 gene SNP and haplotype with major depression", NEUROSCIENCE LETTERS, LIMERICK, IE, vol. 404, no. 3, 1 September 2006 (2006-09-01), pages 358-362, XP027885387, ISSN: 0304-3940 [retrieved on 2006-09-01]
- BOGUSLAWA BUDZISZEWSKA ET AL: "Regulation of the Human Corticotropin-Releasing-Hormone Gene Promoter Activity by Antidepressant Drugs in Neuro-2A and AtT-20 Cells", NEUROPSYCHOPHARMACOLOGY, vol. 29, no. 4, 1 April 2004 (2004-04-01), pages 785-794, XP055077200, ISSN: 0893-133X, DOI: 10.1038/sj.npp.1300379
- YUHPYNG L. CHEN ET AL: "Synthesis and SAR of 2-Aryloxy-4-alkoxy-pyridines as Potent Orally Active Corticotropin-Releasing Factor 1 Receptor Antagonists", JOURNAL OF MEDICINAL CHEMISTRY, vol. 51, no. 5, 1 March 2008 (2008-03-01), pages 1377-1384, XP055077125, ISSN: 0022-2623, DOI: 10.1021/jm070578k
- Brendon Binneman ET AL: "A 6-week randomized, placebo-controlled trial of CP-316,311 (a selective CRH1 antagonist) in the treatment of major depression", The American journal of psychiatry, 1 May 2008 (2008-05-01), pages 617-620, XP055143057, United States DOI: 10.1176/appi.ajp.2008.07071199 Retrieved from the Internet: URL:http://www.ncbi.nlm.nih.gov/pubmed/184 13705

### SEQUENCE LISTING

<110> HolsboerMaschmeyer NeuroChemie GmbH
<120> CRHR1 antagonists for use in the treatment of patients having CRH overactivity
<130> H 7835/MH
<150> GB 1207102.3
   <151> 2012-04-23
<150> EP 12165231.7
   <151> 2012-04-23
<160> 30
<170> PatentIn version 3.5
<210> 1
   <211> 401
   <212> DNA
   <213> Homo sapiens
<400> 1
   caagaaagag agtaataaaa ataaccacaa tgagggctct cattaatact ggatcttatg 60
   gaaaccaatt gttcagtccc tcaacaaaag accagatggg caggaagcta aatatacacc 120
   atgcactaaa cattatgagt atcatagttt acaagtcaaa gggggctcta ttgaagatag 180
   ttctattttc cctctatatt atctgctaga caatacctga taacattatc caagtaaatg 240
   acaacttgat aaatagtaat ttccaatggt gaacagaggt gacatttcct cattacaaaa 300
   atattttctt tggcagatga gattaactga ataagaaatc cactgacact gaaatcacag 360
   agccaaattc cctatcacag cacttatcac attgcgttag g 401
<210> 2
   <211> 401
   <212> DNA
   <213> Homo sapiens
<400> 2
   ttccttgtag cggggagaga gactcaggga aggcagggtg atacctgagt tggggcttaa 60
   agcaaggtag ggtgtgtgtg gtgatggcaa aataggtagg aagacagcac gggcaaagtc 120
   ctggaggcaa ggacagaaag aggaagtggc aggaagtgag gctggggaaa tgagtagggg 180
   tcaatcatga tgtttctggt atagggaaga gtttggaatg catcctctag gccatacgcc 240
   attgggggct tttaagaaag acagtgatgt tggtttgatt tgcattttat atagactttt 300
   ctggcagctg agaggaaggt ggttttgaga atcacaaagc tgcgggaaga tcagtcagga 360
   gggttctaga ataatccagg caagagctga tggggactga g 401
<210> 3
   <211> 401
   <212> DNA
   <213> Homo sapiens
<400> 3
   acaggggtgg ctactctttc tccagaaata ggtgtcctgt ggggcatttt gaagtagaat 60
   gttgatagtt gctttcaatt ttagactggt aaataagaat tgggcatttg aatttcaata 120
   tactcactgt gtaactgtta ttgagtatgc tttaagtgac ctataatact gcttcattta 180
   actttattgt cctaataact ctcttagagt gacaataact taggttagcc acttgcctag 240
   ggttctgaaa ccaagtaaat ggtggagctg gaattgctgt tcttgtcagt cattagacta 300
   gatcggtttt cttcttccta caaattttat atactaaaaa attttgaaaa aagacatttt 360
   tctttgggaa aaatagggaa tgtcagatcc ctttggagat g 401
<210> 4
   <211> 401
   <212> DNA
   <213> Homo sapiens
<400> 4
   ctcgcagcaa ccaagcctgc ccaagccggg gaaacctggg gagcaaacct tcacctgcac 60
   tgtacatcag agaccagttg gccctatttt ggctcctgtg gacaggtaag tatccctttt 120
   gactcatccc ccaaatatca ggtgagccag gaaaataagg cctttggctt agacagtcaa 180
   ttcaaagtct gccatagcat acctaattac atccctattg ccccttttct aggtcgtttc 240
   tcctctaaca cgattttatt tttctgtcag ccattttatt ttatttctca ccttgaaata 300
   tatgttttct ttgcagtttt tgctttggct tcctgctaac tctatttggg caattgttta 360
   aggctgaaca cttggttatg agaggtaccc tgttgtgttg a 401
<210> 5
   <211> 401
   <212> DNA
   <213> Homo sapiens
<400> 5
   tcatcaagtc tcctttttct ctaggaaaaa taacattgtc aaggttatta acagtcaata 60
   agctgtcata ggctcagcat ggatggggat attgggtttc cttgtgctta tatgaaagat 120
   gggaaaatcc gaagttcttt tcaccctgat atggaaaata cccaacatga ggagaagcag 180
   cagctatatg attctgagca cagaatggga gtaagaatag ggtcatgctg tactgattat 240
   ctgctaataa aatgcaaaag tgttaggtaa tttcatcaat atccagttaa tactaatata 300
   gttaatattt catgactggg taatatttta taatgataaa tatttttata gatcttagct 360
   ctttttattc tcatatcaac tgtatgaaat cagtgattgg t 401
<210> 6
   <211> 385
   <212> DNA
   <213> Homo sapiens
<400> 6
   ctggggacct cagggagagg tacgcaggtt gccatggctg cgtctgcagt ccacctgcct        60
   ttccacgcca gggagtcagt gatgtggagc cccctgggcc ccagtggaag cagcgatcag       120
   actatgtgtc cttgaaataa tgtttattcc acgctgtccc gacagccccc tctgcaggtc       180
   ccctcggtgt actctgaggt gggaaaccct ccctgggggc ggtgaagggg aactcgggcc       240
   accccaccag ccagcagatg ctccagcagc cagagcccca gcctggagct gaggctcttc       300
   ctggggctcg ccgggcccct gcaggctttt cggaccctca gccagcccgg cttcctctgc       360
   tttgggcagc agcaagctgg ccctt                                             385
<210> 7
   <211> 401
   <212> DNA
   <213> Homo sapiens
<400> 7
   ttcctgtgcc tcagctcctc tgtagaatgg tgctggcaat acagtttgcc tcattgggct        60
   cttgtaagct ttaaataggt tattatacat aaagagctaa tagtgatgcc tgtagccgtt       120
   gtctaagtgc tagctctgat gatggtgaca aagaagtaat agcaatcagt ggtttagatt       180
   aaaccatttt aggcataaac cgttctgcta gaatccaagg ggagattttt tcccatcaag       240
   gagacatagc ttgttgggaa gataagacat acccaattgc agaagtaatt aattaattct       300
   tttttttttt tttttttttt tttttgcgat ggagtttcgc tcttgttgcc caggctggag       360
   tgcaatagca tgatctcggc tcaccacaac ctctgcctcc t                           401
<210> 8
   <211> 401
   <212> DNA
   <213> Homo sapiens
<400> 8
   ataggcccta tacagctctc aatttcttta atcaatcttc ctagcagccc gtgagaaata        60
   ttactgtctt cagcttccta aaggagaaaa cagaggcctg gagggattaa aagacttttc       120
   taagatttta gagggcatgt tagggttcag gcccagggct gtctaaccca aggcctaatt       180
   ccttctatta catccatcat acatgagtga gcactgggca tgaggatacg tcagtgaaag       240
   gggccctgta acatggacct tacattttgg ctgggggaga caggcaatga atacatagga       300
   ccatgttggg aagtgctaag tactctgatg ataacacagc agggtgaggt gacagaggtc       360
   tagggagagt ggtgttcagc aaaaacttct ctggggagag a                           401
<210> 9
   <211> 401
   <212> DNA
   <213> Homo sapiens
<400> 9
   aaaatttacc aggtttaaaa aaaaaaaaac tcaaatgata tttcagaaac ctaccccttt      60
   caaaacaagg aggagaaaaa tcttctccac aaaagcacat attgaaaaaa tattttgggg     120
   gcaaggcctg aaagggttgg cagtgtgcag ttctgttatt attcccgtgg ccattttatg     180
   ggcctcagca aaacactggg atcattatct gtcttctggt tactccagga gagctagcca     240
   tcacaaccca atggaagaga cttcagagaa acccacacag gcaccagaag tccttccctt     300
   tcatctgcca ctgtggggtt ttgtcctcat ctattacaat gttgtccaat ctcagactgc     360
   attcagaaca aaggctctca gactgaggat gagttcttgg a                          401
<210> 10
   <211> 401
   <212> DNA
   <213> Homo sapiens
<400> 10
   ccaaataatt gttattgttg ttttaacatg gcaatcacgt tatttgccat atgtgaaaaa      60
   gaatatttaa aatgcttttt aaaactatgt atgtaaaaga atgtttaaat tgttttaaaa     120
   atatgttata tctaccttgg caccatcctt gctgttgaga aatgactttt acctgcttac     180
   ttagaaggaa atgtcagaag cagaagtaca tttgaatacg attatttgaa agcttcatcc     240
   atttttcaaa gaatgtatac agtaacacta aatagaaagc atagtttatc aactcttcac     300
   taagaacagt ctagcaagta tatcagagtg gctgtggttc cagttggact aacctaatca     360
   tttatgaaaa ggtgataata agcttggacc aagagcaccc a                          401
<210> 11
   <211> 401
   <212> DNA
   <213> Homo sapiens
<400> 11
   cagatgttat gtgaaactct ggagaaatag tagcaagcaa gactcacatg cctcctgccc 60
   tcacagagct ccatgatctg gtgaaagtgc cagatattta aacccatgga tgtgtgcaca 120
   caaaataaca attctctcaa gcgttgtgaa gaaaagtcac agagcactac aaaagcatgt 180
   aagagtgagg caaaacctat cgtgttagga cagggaaggc ttctgtgagc taacctgaag 240
   gatgagtagg ggtgagccag atgaaaaggc gagagaaaaa cattctgagc agagactgcc 300
   actgagtgca tcccagtttt cccaacatct taacactgta taatgactac actggatttt 360
   cttcatcctg gatccatggt tagacatgtt aatatgcctt c 401
<210> 12
   <211> 401
   <212> DNA
   <213> Homo sapiens
<400> 12
   cccagtctgt ggtatttttt tatagcagca caaacagact aacacaagag gtggatagga 60
   tttgcgagca tggaccttgg aggtttgtgg cctcaattta aagtgagtac attcacccag 120
   ctggtgtttt tctcttgctg cttgggcaca gagatggagt aaatgggtct aatcaaggat 180
   aaagggagag ccaaagagat agtaatattt gaaaggaagt gtttttaatg atgtgccatg 240
   taatctgagc tgggtcagga atgaagtgaa aaactaagag atgatggatg atgatagggg 300
   ctgtgaaagg aaaacaaatc ttggggcccc caaatcacta agctaaagga gaaagtcaag 360
   ctgggaactg tttagggcaa tcctgcctcc cattttattc a 401
<210> 13
   <211> 401
   <212> DNA
   <213> Homo sapiens
<400> 13
   tattactgct gagaaaactg ggtttgataa actaaagatg cccatgtata tcagtcatgc 60
   tcctggtgag aacaggtggc tcactgcata atgagaggaa tattcaatta actatttaca 120
   aagctatgga tgacatgtag ggaagccaca gagagagtac agtatctaga gctagtaaga 180
   gtagaaggcc atcactgtcc acaggcctaa aggaggtaga gcagtcaaag gaaacaagag 240
   acaagggagg ctgcgaggac agggccacct ggcagagcca taaccttaaa ctaggtagtc 300
   acttcttggc aactctgcag gtagggagcc aacctcactt ttaaccctcc ctctgatgcc 360
   cagctggttt accccattgg tgaaaatcag tgggtgaggg a 401
<210> 14
   <211> 401
   <212> DNA
   <213> Homo sapiens
<400> 14
   catgaaaaga tacttaacat tgtaacatct ttgcattagg gaactgcaaa tcaaaatcat      60
   aacaaaatag tactgcatgc tcattaggat gactataatc caaaagaata aaaaagaaaa     120
   taacaggtgt tggtagggat acagatatag agaaactgga gctctcatgc cttgctgggg     180
   ggcatgtaaa atgattctgc cgctttggaa aacagtttgg tggttcctca aaaagttaaa     240
   catataatcc aacaattcca cccaaaagaa ttgaaagcag ggtctagtac accaacgttc     300
   atagcagctt tattcacatc aagccaaagg tggaagcagc ccaaatgtct actgatggat     360
   gagttgatac acaaaatgtg gtatatatat gcaatggaat a                         401
<210> 15
   <211> 401
   <212> DNA
   <213> Homo sapiens
<400> 15
   gccacttgaa tgccccaaaa tggagagatg ggcgtgggaa gagaaagaca cctcagcaac      60
   acagagctga gaaaacactg tgagttttat ttaattccta cttaccgtta ttttgcatag     120
   taaacaaaag ggatattttt gaaaatccct ttggataatt tctgccacct aaaattctga     180
   gcattttgac tcactgcctt ataaaaagaa tcaattaatt gaataagaga agggattctc     240
   ccctgatctt ttcaagaatc cttaaaaggc acatttctca ctaaggatct tgaaagtgta     300
   tttctagcca atcccaggag tcactgctca gagatttaca tttcacaaat gtaatcaaca     360
   gcctaagcag aatattgacg tttggactgc agagctctgc t                         401
<210> 16
   <211> 400
   <212> DNA
   <213> Homo sapiens
<400> 16
   agggtcccca aatatttcca tttgagatga caaagtgctc ttcagtcatt tagcttactc 60
   ttcagttcag atgacttatc atcttgattt cagagagttc atatatgtct gttttaaaaa 120
   actggttcaa aaagtctgaa gttacgaaac taaaccaaat atgcattact ctcatgtcaa 180
   attacaagct cttagctgcg gggatttttt cacatgcagc ctggagccct tgaaaacctc 240
   tgttttctgt tagactctcc agggtacaca gaagttgcct cattatttta gttaatggtg 300
   actgcaaata agccccccaa gtcatttaac tatgtgctta ccactgcttt aaaagaaccc 360
   caagttaggt cctcatgtag gtaaaggagc tcccttcaca 400
<210> 17
   <211> 401
   <212> DNA
   <213> Homo sapiens
<400> 17
   acttgggccc aaaggcattc aactagaaag ctggtaataa taacagcgac agtttattga 60
   gtcttagtgt ttctgagaac ttttctaagt actttacaca tattaaattt ttaaatcttc 120
   acattagtcc tgtgaggaag gtactattgt tatgtctgta ttacccatgg ggatactgac 180
   gcacaaagaa gtcaagtaat atatttaaga ttctagtaag tgcagagccc aggtgcatgc 240
   agtgcctggg ctctgccacc catgcagtgc tgactagggc ttccacccat ggattttttt 300
   tttttttttt tttttttgag acagagtttc gctcttgttg cccaggctgg agtgcaatgg 360
   catgatctcg gctcaccaca acctccgcct ctcgggttca a 401
<210> 18
   <211> 401
   <212> DNA
   <213> Homo sapiens
<400> 18
   cccataataa tgaaggattg gacctgataa tctatcaggt acattttagc ctgaaattta 60
   tttgtacaca cgcacaaaca cagacatgtg cacacacaca tacacatata tatataacat 120
   ttataaattt taaaacataa agctatacta gaaatgaaag cttatatatt gaactgcccc 180
   acctttctat ttgcagccag ctaccacccc agtctaatgt ttcactttat ataaattcat 240
   ttattctttt actcatttca aatatatgat gatgtaacta taaaatcaac atttagtcac 300
   tctgaataac ccaaaatagc aaataattta aaaatcactt ccacttgact ttagaatcta 360
   ttacatgcat tgtttttcca gaaaatttac ctcataatta t 401
<210> 19
   <211> 401
   <212> DNA
   <213> Homo sapiens
<400> 19
   ctacttatat gattagagaa caagaatact agggggaaaa tcagcatgca tataatctaa        60
   gaaattgtca ttataatttt aaaatccttt gcaaaatcag taaatatgag tttaacttat       120
   ataatgatac acacacacac tgatatgatg ctttattgtc taaacactgg ctgcttgtgg       180
   agacgtattc tggtaacaaa aaatatagca tcttaaaatt gatgctagca ttgtatatcc       240
   aaatagagag taaatgcaac cagaatattt tttatatgtt taacattgta gtgttgctga       300
   catcattata tatttggtta tgttaatctc aaaatgcaca atatagctgt atgatctgta       360
   taatgcaaaa aaatgtagag cttcattttg atatttatta t                           401
<210> 20
   <211> 401
   <212> DNA
   <213> Homo sapiens
<400> 20
   ctggaaggga acaatggaag aggtgcatta gtcacattcc aaaatgcagg aagcaataac        60
   atgtggcact attgtcattt atgtagcacc ctaaatactg ggacaaatga catagatgcc       120
   cttctgtgat tactaaactc ccccacagtg tctcagaagg aagagctttt gacaggaaat       180
   catcaagatc tgatgacatt agagagcaat taacattctc ttcaaccatg aactaattgc       240
   ctcattcaca tttttctagc catcctagga agcagataat aagcagcaat tgtcctgccc       300
   aggaattctg acttgtgtaa tttgtaaagc ttttctttgt atctatttct ttcctgtggc       360
   catctttttg tttttggact gtttggtaac agtaagtggg t                           401
<210> 21
   <211> 401
   <212> DNA
   <213> Homo sapiens
<400> 21
   atcattcagt attaagagag aaatgaatac attttcagat atacaagaat tcagtttacc        60
   tcccacagaa tctctgaaaa aaatattaga ttactatagt taaaaaggaa aataaaataa       120
   gtcctgttag aaataattgg taaaaaagca aaggtgatga aaacttattg aaatatatta       180
   ttaaagtaat tgttaaaaat atacactaaa tctagaatat ataaatgtag cagttgttaa       240
   ggggaagggg aaatagaagt ggaagaaaat gaacattaga acaaatgttt agcagtggga       300
   ttattttatt ggaagtctaa tgtaagaagt atattctcca gggaggtatt tcaaggacat       360
   atgaatagta aagggataat aaaacaactc tataaggtag t                           401
<210> 22
   <211> 401
   <212> DNA
   <213> Homo sapiens
<400> 22
   cacacacaac gctgggccca gtaaataagt tttgtttttt cccagggaaa agttgaacaa        60
   caatggtgag accaggaagg ctctccgttc acaggaaata ctgtgtcacc gctcggccgc       120
   aggctgtgtg aggtcacggg cgacgctcgg gtcacgtgtg gcggctcctg ttcacagtgc       180
   cgtgtgtgat aaactgggac cttctggtga ggggagactg gcggggggtg gggagggcaa       240
   ggagtgggaa agtcgcctat aaatgtttaa caaaagatcc gcaatgggaa caggaacttg       300
   cattctttct ttcaatggac aaagcttcca catcaagata cgcttgtgtg ctgggaccaa       360
   atgccacagt gcggcgaaac tcgtgagcac aagtcctgcg t                           401
<210> 23
   <211> 300
   <212> DNA
   <213> Homo sapiens
<400> 23
   acaacagggt atcctagccc agcaaaattg actcataaat ttaatgatca cgcaattggt        60
   aattctaaat ccagtcagaa gtctacattc tgtgtccaca gtgtcatgtc tagatgttgg       120
   tccagtctcc catggactgt gccttgttat ttgttttctc tttgctaagc cacatcccct       180
   gagggctctg tttatgctca ctgcaaaatc tttgactttt taacttactg ggcatattgt       240
   cttcctactt ttgttctctt ctgttatttt atttacttga ctctgacatg tctcattccc       300
<210> 24
   <211> 401
   <212> DNA
   <213> Homo sapiens
<400> 24
   tttcaatggg actggttgga cagtgggtgc agcccatgaa gggcaagcca aagcaggccg 60
   gggcatcacc tcacccggga agcacaaggg gtcaggcgat ttctctttcc tagtcaaggg 120
   aagccatggc agactgcacc tggaaaaacg agacacttcc acccaaatac tgcgtttttc 180
   gcaaggtctt agcaactaac agacaaggag attctctccc gtgcctggct cggctggtcc 240
   cacacccacg gtgacttgtt cactgctagc acagcagttt gagatcgaac tacgaggcaa 300
   caacctggct aagggagggg catctgccat tgctgaggct tgagtaggta aacaaagtgg 360
   ccaggaagct cgaactgggt ggagcccact gcagcttagc a 401
<210> 25
   <211> 298
   <212> DNA
   <213> Homo sapiens
<400> 25
   actttaggga ctttgagtga tggacaaccc cctatcagat atcatcagcc tgaaacatcc 60
   ttatcttggc attaaattag aaggaacccc agaccctgcg taccagaatt gttagaatca 120
   cagtctcagt aaagaaccaa ctcctgatca cttctctaaa ggaaagttct agaagtctgc 180
   acactctgca gtcactttca attctatcca agtgtacact tagaactcta gaaaacacta 240
   cggacagtct tcagccaggt aaagcctaaa accagcaaag aacagggaga gtgaggga 298
<210> 26
   <211> 401
   <212> DNA
   <213> Homo sapiens
<400> 26
   cggattatca cagttctcaa aagaggagta tgcatttgct tgctccaatt cctcttcttc 60
   tacactctct taagcattcc tcaaccagtc taatatctca tagttcccca aaactgctct 120
   gttcaagacc attagtaaga tctttgatgt taatctgtgg accgtatctc tgtccttatt 180
   ttacttgaag cccaacagca aataaaaaag ttgttctcct ctcctccctg ctacactttc 240
   cttatgtggc ttgctgggct cctcagtccc ctgtgaaaaa ctctgacatg gagatactgc 300
   agaccagtag aagggctggg cagacactat acagaaacag tatgccctac atgctccttg 360
   gctaaatctc tagaattttt ttcagaactc atccacaaat t 401
<210> 27
   <211> 401
   <212> DNA
   <213> Homo sapiens
<400> 27
   attttatctc atttacttat taaatcaaac caatatttta tgaagtgatt ccagtattgg      60
   aataaaaatg taattcttta atcattaaaa aatctttatg aataccttac atcaactgta     120
   ggggaccaac cagggaaaag cagggagact tgtagaatct acacctccag aacaaccgac     180
   ctccatcttc tggacaactc atcttctaaa gtgcaggaca gactagttgg gggagaaagg     240
   aggaaatgaa agagatagac taaaagggag ggagagaaca gatatttttt aagtacctgt     300
   tatgttctgg atacagcaca gagtacattg tatctattat tataaggcat aaagaaagat     360
   ttctcaggtt tttggagtca gattgcaata taaaataata g                         401
<210> 28
   <211> 401
   <212> DNA
   <213> Homo sapiens
<400> 28
   taacttcaaa ttgttttgca aaatctctgt cataaaaatg cttaccaaca aatactgata      60
   ctaaatttag atgtgggggt attagttata atcctgaagt gggaggggga acttcttaat     120
   tccaatttag ttctaagaga aggaagagta tttaggccca gagaaggtta cgcttaaagg     180
   tctgatagtg ttttctttga aaaatatgtc tcaaactaga gaataaaact aattatctca     240
   tctaagttac ctagagacat ttatgctcat cagtttgata aaggactgca agtagacaca     300
   gaagctgtat tttcagtctt gaacccagca atagtacatt aacaagattg gggcaaggca     360
   aagggacttt tgtggcacaa gatacaatat atggattgcg t                         401
<210> 29
   <211> 401
   <212> DNA
   <213> Homo sapiens
<400> 29
   ctcttcaaag gcctttgccc ttgggtacca caggttctga gacaagaggg ctatggagag      60
   cccccattat agctggagcc tcctgccctg cccaaaggtg tgacttgaag ggtggaattt     120
   caggcagcgt ggctcgcccc agggaggcaa agaggccagg ggaatcttca aaggccctgg     180
   gctcatccca gctaggaggc aggcacagtc ataaccctaa tccagtgaac tcagccctca     240
   tcctgactct catggtattc tgtcccaggg agcctctttc cagctttctt agaagcttta     300
   atgtcagcac ttgcagggcc ttagaaactg cacgctacct cttcatttca tacatgagga     360
   aactgaggcc cagggtggac acagggctgc ccagcgagtt a                         401
<210> 30
   <211> 401
   <212> DNA
   <213> Homo sapiens
<400> 30
   attataaagc aaagcactaa cctcatagaa tacctgagtc aagttccctg tgttctcatt      60
   ttctagcctc ttctaccaga cactatgaaa aataacagcc ccatctctcc agaaaatctt     120
   aggagatata ggcgtgctga atttaaggtg tctgtggcac atgcaagtgg atcagccact     180
   gggctgtcca gaatgcaaga cagaactcag agttggggac ataaacttgg cagtcatctg     240
   tgtaaaagag aaaaggtagg taaagtccca caaggatggg ttggcctaca gaaggcacag     300
   agagaagaga gcctggttta gcatgggcta cgatcagagt ccctgggttc aaatcttggc     360
   tccacccatt tctatctggt tgctctaggg catgttacct a                         401

## Description

### Field of the Invention

The present invention relates to a corticotropin releasing hormone receptor type 1 (CRHR1) antagonist for use in the treatment of depressive symptoms and/or anxiety symptoms in patients having corticotropin releasing hormone (CRH) overactivity.

### Background of the Invention

While current antidepressant drugs are effective treatments of depression and anxiety symptoms in a number of psychiatric disorders, a large fraction of patients only show partial remission of symptoms or do not respond at all (Trivedi et al., Am J Psychiatry, 2006). This may be due to the fact that these drugs do not target the inherent pathophysiologic disturbances leading to clinical condition in these patients. A number of novel antidepressant strategies, derived from both animal studies and human studies have been tested, but so far with little success. One of these approaches is the use of corticotropin releasing hormone receptor type 1 (CRHR1) antagonists. A wealth of data ranging from molecular studies in experimental animals to open label studies in human patients indicate that CRHR1 antagonists are a promising novel approach in the treatment of depression and anxiety (Ising et al., Exp Clin Psychopharmacol, 2007; Holsboer et al., CNS Spectr, 2001; Paez-Pereda et al., Expert Opin Invest Drugs, 2011; Chen et al., J. Med. Chem., 2008).

WO 95/033750 A1 and US 7,067,664 B1 disclose corticotropin releasing factor antagonists and their use to treat central nervous system disorders and other disorders.

However, so far all randomized clinical trials have failed to demonstrate the superiority of this drug to placebo (Coric et al., Depress Anxiety, 2010; Binneman et al., Am J Psychiatry, 2008).

Hence, there is still a need for antidepressant drugs effective in the treatment of depression and/or anxiety symptoms in a number of psychiatric disorders.

It has now been found that despite the so far unsuccessful clinical trials, a specific group of patients, i.e. patients suffering from central corticotropin releasing hormone (CRH) overactivity, profits from the treatment with CRHR1 antagonists.

### Summary of the Invention

One aspect of the invention relates to a corticotropin releasing hormone receptor type 1 (CRHR1) antagonist for use in the treatment of depressive symptoms and/or anxiety symptoms in a patient having corticotropin releasing hormone (CRH) overactivity, wherein the treatment comprises detecting whether or not the patient has CRH overactivity by determining the status of one or more biomarkers indicative for CRH overactivity, wherein the biomarker or a set of biomarkers is obtained by a genome wide screening for single nucleotide polymorphisms in patients with depressive and/or anxiety symptoms and/or the biomarker is REM density.

The CRHR1 antagonist is a compound of formula (I) 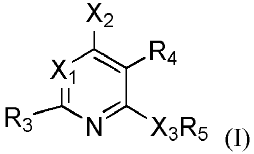 or a pharmaceutically acceptable salt thereof, wherein
X₁ is -CR₆;
X₂ is -NR₁R₂, -CR₁R₂R₉, -C(=CR₂R₁₀)R₁, -NHCHR₁R₂, -OCHR₁R₂, -SCHR₁R₂, -CHR₂OR₁₀, -CHR₂SR₁₀, -C(S)R₂ or -C(O)R₂;
X₃ is NH, O, S, -N(C₁-C₂ alkyl) or -C(R₁₁R₁₂), wherein R₁₁ and R₁₂ are each, independently, hydrogen, trifluoromethyl or methyl, or one of R₁₁ and R₁₂ is cyano and the other is hydrogen or methyl;
R₁ is C₁-C₆ alkyl which may optionally be substituted with one or two substituents R₇ independently selected from the group consisting of hydroxy, fluoro, chloro, bromo, iodo, CF₃, C₃-C₈ cycloalkyl, C₁-C₄ alkoxy, -O-CO-(C₁-C₄ alkyl), -O-CO-NH(C₁-C₄ alkyl), -O-CO-N(C₁-C₄ alkyl)(C₁-C₂ alkyl), -NH(C₁-C₄ alkyl), -N(C₁-C₂ alkyl)(C₁-C₄ alkyl), -S(C₁-C₄ alkyl), -N(C₁-C₄ alkyl)CO(C₁-C₄ alkyl), - NHCO(C₁-C₄ alkyl), -COO(C₁-C₄ alkyl), -CONH(C₁-C₄ alkyl), -CON(C₁-C₄ alkyl)(C₁-C₂ alkyl), CN, NO₂, -SO(C₁-C₄ alkyl) and -SO₂(C₁-C₄ alkyl), and wherein said C₁-C₆ alkyl and the (C₁-C₄) alkyl moieties in the foregoing R₇ groups may optionally contain one carbon-carbon double or triple bond;
R₂ is C₁-C₁₂ alkyl, C₁-C₁₂ alkoxyalkyl, aryl or -(C₁-C₄ alkylene)aryl wherein said aryl is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiazolyl, benzisoxazolyl, benzimidazolyl, indolyl, oxadiazolyl or benzoxazolyl;
   3- to 8-membered cycloalkyl or -(C₁-C₆ alkylene)cycloalkyl, wherein one or two of the ring carbons of said cycloalkyl having at least 4 ring members and the cycloalkyl moiety of said -(C₁-C₆ alkylene)cycloalkyl having at least 4 ring members may optionally be replaced by an oxygen or sulfur atom or by N-Rs wherein R₈ is hydrogen or C₁-C₄ alkyl;
   and wherein each of the foregoing R₂ groups may optionally be substituted with from one to three substituents independently selected from chloro, fluoro and C₁-C₄ alkyl, or with one substituent selected from bromo, iodo, C₁-C₆ alkoxy, -O-CO-(C₁-C₆ alkyl), -O-CO-N(C₁-C₄ alkyl)(C₁-C₂ alkyl), -S(C₁-C₆ alkyl), CN, NO₂, -SO(C₁-C₄ alkyl), and -SO₂(C₁-C₄ alkyl), and wherein said C₁-C₁₂ alkyl and/or the C₁-C₄ alkylene moiety of said -(C₁-C₄ alkylene)aryl may optionally contain one carbon-carbon double or triple bond;
   or -NR₁R₂ or -CR₁R₂R₉ may form a saturated 5- to 8-membered ring which may optionally contain one or two carbon-carbon double bonds and/or in which one or two of the ring carbons may optionally be replaced by an oxygen, nitrogen or sulfur atom and which may be substituted with at least one substituent;
R₃ is methyl, ethyl, fluoro, chloro, bromo, iodo, cyano, methoxy, OCF₃, methylthio, methylsulfonyl, CH₂OH, or CH₂OCH₃;
R₄ is hydrogen, C₁-C₄ alkyl, fluoro, chloro, bromo, iodo, C₁-C₄ alkoxy, trifluoromethoxy, -CH₂OCH₃, -CH₂OCH₂CH₃, -CH₂CH₂OCH₃, -CH₂OCF₃, CF₃, amino, nitro, -NH(C₁-C₄ alkyl), -N(CH₃)₂, -NHCOCH₃ -NHCONHCH₃, -SOₙ(C₁-C₄ alkyl) wherein n is 0, 1 or 2, hydroxy, -CO(C₁-C₄ alkyl), -CHO, cyano or - COO(C₁-C₄ alkyl) wherein said C₁-C₄ alkyl may optionally contain one double or triple bond and/or may optionally be substituted with one substituent selected from hydroxy, amino, -NHCOCH₃, -NH(C₁-C₂ alkyl), -N(C₁-C₂ alkyl)₂, - COO(C₁-C₄ alkyl), -CO(C₁-C₄ alkyl), C₁-C₃ alkoxy, C₁-C₃ thioalkyl, fluoro, chloro, cyano and nitro;
R₅ is phenyl, naphthyl, thienyl, benzothienyl, pyridyl, quinolyl, pyrazinyl, pyrimidyl, furanyl, benzofuranyl, benzothiazolyl, or indolyl,
   wherein each of the above groups R₅ is substituted with from one to three substituents independently selected from fluoro, chloro, C₁-C₆ alkyl and C₁-C₆ alkoxy, or with one substituent selected from hydroxy, iodo, bromo, formyl, cyano, nitro, trifluoromethyl, amino, (C₁-C₆ alkyl)O(C₁-C₆)alkyl, -NHCH₃, - N(CH₃)₂, -COOH, -COO(C₁-C₄ alkyl), -CO(C₁-C₄ alkyl), -SO₂NH(C₁-C₄ alkyl), - SO₂N(C₁-C₄ alkyl)(C₁-C₂ alkyl), -SO₂NH₂, -NHSO₂(C₁-C₄ alkyl), -S(C₁-C₆ alkyl) and SO₂(C₁-C₆ alkyl), and wherein the C₁-C₄ alkyl and the C₁-C₆ alkyl moieties of the foregoing R₅ groups may optionally be substituted with one or two fluoro groups or with one substituent selected from hydroxy, amino, methylamino, dimethylamino and acetyl;
R₆ is hydrogen, methyl, fluoro, chloro, bromo, iodo, cyano, hydroxy, -O(C₁-C₄ alkyl), -C(O)(C₁-C₄ alkyl), -C(O)O(C₁-C₄ alkyl), -OCF₃, CF₃, -CH₂OH, - CH₂OCH₃ or -CH₂OCH₂CH₃;
R₉ is hydrogen, hydroxy, fluoro, or methoxy;
R₁₀ is hydrogen or C₁-C₄ alkyl.

In one embodiment of the CRHR1 antagonists of the invention, the 5- to 8-membered ring formed by -NR₁R₂ or -CR₁R₂R₉ of the compound of formula (I) as defined above is substituted with at least one substituent selected from C₁-C₄ alkyl or with a 4-8 membered ring, which may be saturated or may contain one to three double bonds and in which one carbon atom may be replaced by CO or SO₂ and one to four carbon atoms may optionally be replaced by nitrogen.

In a specific embodiment of the CRHR1 antagonists of the invention, X₂ of the compound of formula (I) as defined above is -NHCHR₁R₂, -OCHR₁R₂ or -NR₁R₂.

Optionally, in the compounds of formula (I) as defined above -NHCHR₁R₂ is - NHCH(CH₂OCH₃)₂, -NHCH(CH₂OCH₃)(CH₂CH₃), -NHCH(CH₂CH₃)₂,, - NHCH(CH₂CH₂OCH₃)₂ or -NHCHR₁R₂, wherein R₁ is ethyl and R₂ is oxadiazolyl substituted with methyl, or -NR₁R₂ is -N(CH₂CH₃)(CH₃), -N(CH₂CH₂CH₃)₂, - N(CH₂CH₂CH₃)(CH₂-cyclopropyl), -N(CH₂CH₃)(CH₂CH₂CH₂CH₃), - N(CH₂CH₂OCH₃)₂, or -N(CH₂CH₂OCH₃)(CH₂CH₂CH₃), or -OCHR₁R₂ is - OCH(CH₂CH₃)₂, -OCH(CH₂CH₃)CH₃, -OCH(CH₂CH₃)(CH₂CH₂CH₃), - OCH(CH₂CH₃)(CH₂OCH₃).

In another embodiment of the CRHR1 antagonists of the invention, R₃ and R₄ of the compound of formula (I) as defined above are methyl.

In a further embodiment of the CRHR1 antagonists of the invention, X₃ of the compound of formula (I) as defined above is O.

Another embodiment of the invention relates to the CRHR1 antagonists of formula (I) as defined above, wherein R₅ is phenyl substituted with from one to three substituent(s) independently selected from the group CH₃, CH₂CH₃, OCH₃, Cl, F, CF₃.

A specific embodiment of the invention relates CRHR1 antagonist for use in the treatment of depressive symptoms and/or anxiety symptoms in patients having corticotropin releasing hormone (CRH) overactivity, wherein the CRHR1 antagonist is a compound of the formula (VI) 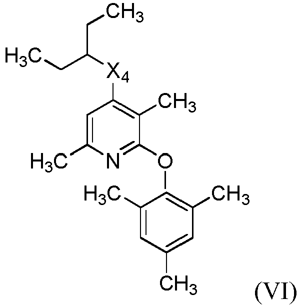 or a pharmaceutically acceptable salt thereof, wherein
X₄ is O or NH.

Another specific embodiment of the invention relates to a CRHR1 antagonist for use in the treatment of depressive symptoms and/or anxiety symptoms in patients having corticotropin releasing hormone (CRH) overactivity, wherein the CRHR1 antagonist is selected from the group consisting of CP-316,311 and CP-376,395.

In an exemplary embodiment of the present invention, the CRHR1 antagonist is CP 316,311.

### Brief Description of the Drawings

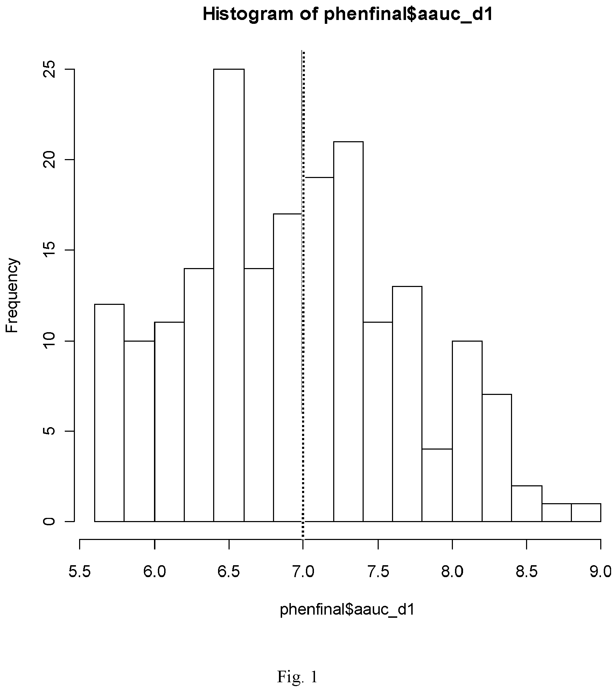

**Figure 1****:** Graph of the phenotypic distribution of ln(AAUC) at in-patient admission. The X-axis shows the In of the AUC of the ACTH response and the Y-axis the frequency in total N/bin. The dashed vertical indicates the cut-off for being classified as a low vs. high responder. AAUC is the area under the curve of the ACTH response.

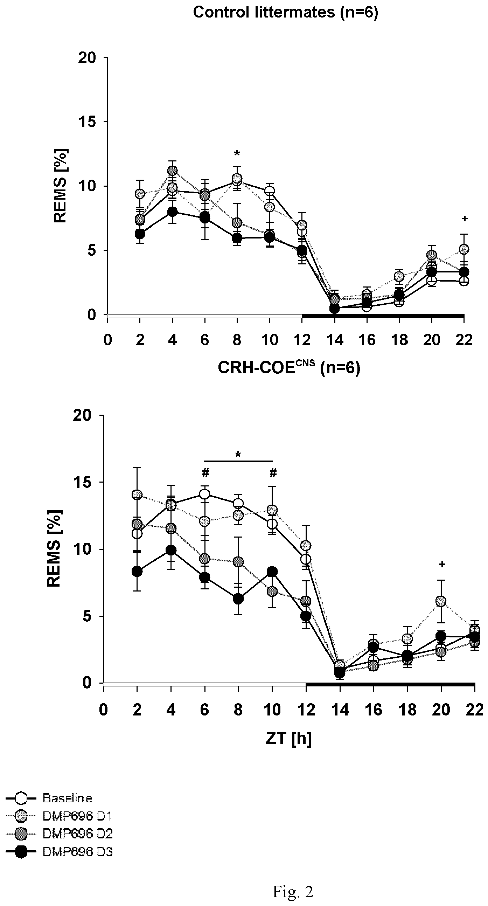

**Figure 2****:** Increased REMS activity in CRH-COE^{CNS} mice is suppressed by DMP696 (50mg/kg/d) application via drinking water. Treatment day one, light green; treatment day 2, dark green; treatment day three, orange. Symbols indicate significant differences between baseline and treatment day one (+), two (#) or three (*). Light and dark bar on the x-axis indicate light and dark period, respectively.

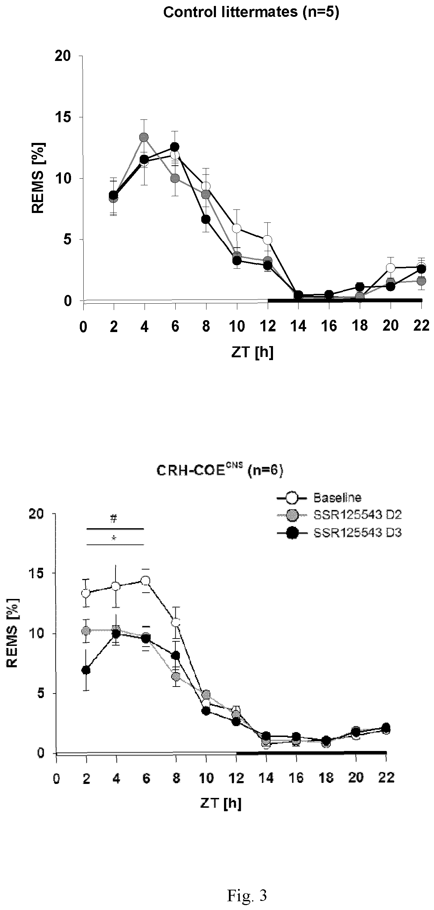

**Figure 3****:** Increased activity of REMS in CRH-COE^{CNS} is suppressed by application of the CRH-R1 antagonist SSR125543 (50mg/kg/d) via drinking water. Baseline day, black; treatment day two, dark green; treatment day three, orange. Symbols indicate significant differences between baseline and treatment day two (#) or three (*). Light and dark bar on the x-axis indicate light and dark period, respectively.

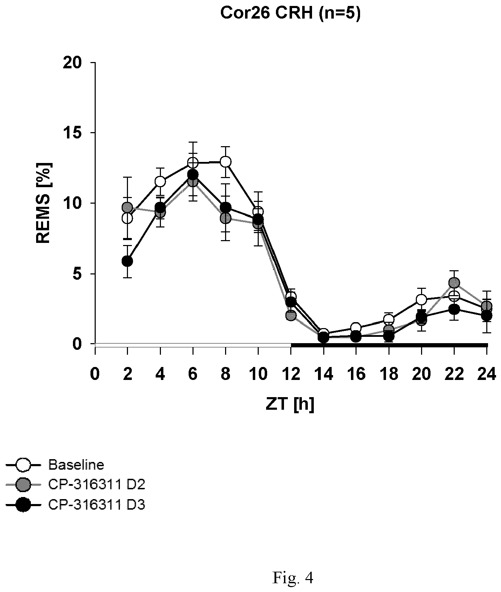

**Figure 4****:** REMS activity in Cor26 CRH mice is suppressed by application of the CRH-R1 antagonist CP-316311 (50mg/kg/d) via drinking water. Baseline day, black; treatment day two, dark green; treatment day three, orange.

### Definitions

Where the term "comprise" or "comprising" is used in the present description and claims, it does not exclude other elements or steps. For the purpose of the present invention, the term "consisting of' is considered to be an optional embodiment of the term "comprising of'. If hereinafter a group is defined to comprise at least a certain number of embodiments, this is also to be understood to disclose a group which optionally consists only of these embodiments.

Where an indefinite or a definite article is used when referring to a singular noun such as "a" or "an" or "the", this includes a plural form of that noun unless specifically stated. Vice versa, when the plural form of a noun is used it refers also to the singular form. For example, when SNPs are mentioned, this is also to be understood as a single SNP.

Furthermore, the terms first, second, third or (a), (b), (c) and the like in the description and in the claims are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The term "corticotropin releasing hormone receptor 1" of "CRHR1" of "CRF₁" refers to the receptor which binds to corticotropin-releasing hormone.

The term "CRHR1 antagonist" refers to a compound capable of binding directly or indirectly to a CRH receptor 1 so as to modulate the receptor mediated activity.

CRHR1 antagonists are well known in the literature and are e.g. described in WO 94/13676, EP 0 773 023, WO 2004/047866, WO 2004/094420, WO 98/03510, WO 97/029109, WO 2006/044958, WO 2001/005776 and WO 95/033750. Exemplary CRHR1 antagonists comprise NBI30775/R121919 (Neurocrine), CP316.311 (Pfizer), CP154,526 (Pfizer), Emicerfont (Glaxo), ONO-2333Ms (Ono Pharmaceutical), Pexacerfont (Bristol-Myers-Squibb), SSR125543 (Sanofi-Aventis), NBI-34101 (Neurocrine) and TAI041 (Taisho).

Most of the non-peptidic CRHR1 antagonists can be described by or adhere to a pharmacophore model that comprises or features a lipophilic top group, a heterocyclic core containing an invariable hydrogen bond acceptor, which is almost always a heterocyclic nitrogen, and a lipophilic, usually aromatic, bottom group.

Class I CRHR1 antagonists as used herein may be characterized in that the heterocyclic hydrogen bond acceptor and the bottom group are connected by a two-atom linker as exemplified by CRHR1 antagonists R-121919, NBI-30545, CP-154526, DMP696, pexacerfont (BMS-562086), emicerfont (GW876008), or verucerfont (GSK561679). Class II CRF1R antagonists as used herein may be characterized by a two-atom linker between hydrogen bond acceptor and the bottom group as present in CRHR1 antagonist SSR125543A.

"Downstream target" or "molecule which is downstream of the CRHR1 receptor" as used herein may denote a molecule such as an endogenous molecule (e.g. peptide, protein, lipid, nucleic acid or oligonucleotide) that is regulated by CRHR1 directly or indirectly. The direct or indirect regulation may comprise direct or indirect modulation of the activity and/or expression level and/or localization, degradation, stability of the downstream target.

The term "normal CRH activity" refers to a range of CRH activity which can be found in human beings who are not affected by a condition which is associated with an increase of CRH activity (such as depression). A value indicative for normal CRH activity is usually considered to be indicative or predictive for a patient not responding to a treatment with a CRHR1 antagonist.

Further definitions of the terms will be given below in the context of which the terms are used.

### Detailed Description of the Invention

While so far all randomized clinical trials have failed to demonstrate the superiority of CRHR1 antagonist to placebo in the treatment of depression and anxiety, it has now been found that a certain patient group, i.e. patients with corticotropin releasing hormone (CRH) overactivity, is responsive to the treatment with corticotropin releasing hormone receptor type 1 (CRHR1) antagonists.

Hence, the present invention relates to CRHR1 antagonists for use in the treatment of depressive symptoms and/or anxiety symptoms in said novel patient group, i.e. patients having CRH overactivity.

The terms "CRH overactivity", "CRH system overactivity", "CRH hyperactivity", "CRH hyperdrive" or "central CRH hyperdrive" are used herein interchangeable. An indication for CRH overactivity may be an increase in activity or concentration of CRH or of one or several molecules downstream of the CRHR1 receptor, that are activated or whose concentration is increased based on the activation of CRHR1 receptor upon CRH binding. A further indication for CRH overactivity may be a decrease in activity or concentration of one or several molecules downstream of the CRHR1 receptor, that are inactivated or whose concentration is decreased based on the activation of CRHR1 receptor upon CRH binding. A value indicative for CRH overactivity is usually considered to be indicative or predictive for a patient responding to a treatment with a CRHR1 antagonist. CRH activity vs. CRH overactivity may be defined relatively to the whole group, e.g. by using a median split of the area under the curve of the ACTH response in the dex/CRH test. Responses in the upper median may be categorized as being predictive of CRH overactivity, while responses in the lower median are indicative of normal CRH activity.

A "value indicative for CRH activity", a "value indicative for CRH overactivity" and/or a "value indicative for normal CRH activity" can be obtained by determining the concentration or activity of CRH and/or of a downstream target of the CRHR1 receptor. The analysis is usually set up in a way that it can be excluded that the modulation of activity or concentration of a downstream target of the CRHR1 receptor is due to another disturbance than CRH activity. For instance, the concentrations or activities of adrenocorticotrophin (ACTH) and/or cortisol are useful biomarkers for determining a value indicative for CRH overactivity. Typically, the CRH overactivity in each patient may be determined by measuring the ACTH and/or cortisol level response to a combined dexamethasone suppression/CRH stimulation test as described in Heuser et al. (J Psychiatr Res., 1994).

The neuroendocrine response to the dex/CRH test may be analyzed using the total area under the curve (AUC) of the ACTH response. Patients suffering from depression normally show an increased release of cortisol and of adrenocorticotropic hormone (ACTH) in response to the combined treatment with dexamethasone and CRH as performed during the test, thus indicating a dysregulation of the hypothalamic-pituitary-adrenal (HPA) axis. Patients with a high HPA axis dysregulation show AUC values of cortisol of between 3000 and 18000 AUC units (ng/ml x 75 min) and/or AUC values of ACTH of between 1000 and 6500 AUC units (pg/ml x 75 min). Patients having a low HPA axis dysregulation show AUC values of cortisol of between 300 and 2500 AUC units (ng/ml x 75 min) and/or AUC values of ACTH of between 250 and 1000 AUC units (pg/ml x 75 min) Various antidepressants lead to a reduction of these increased cortisol and ACTH levels in a combined dex/CRH test performed after the treatment with the antidepressants. Treatment response to antidepressants can thus be determined by performing a second dex/CRH test after treatment with the antidepressant and comparing the neuroendocrine response to the one shown in a combined dex/CRH test performed prior to treatment with the antidepressant.

In one embodiment of a combined dexamethasone suppression CRH stimulation test, subjects are pre-treated with dexamethasone and blood is drawn in certain intervals. Further, human CRH is administered after the first pretreatment with dexamethasone. Plasma ACTH and/or cortisol concentrations are then determined. The neuroendocrine response to the dex/CRH test may be analyzed using the total area under the curve (AUC) of the ACTH response. Details of an exemplary dex/CRH test are also described in Example 1 below.

Depressive symptoms comprise *inter alia* low mood, low self-esteem, loss of interest or pleasure, psychosis, poor concentration and memory, social isolation, psychomotor agitation/retardation, thoughts of death or suicide, significant weight change (loss/gain), fatigue, and feeling of worthlessness. The depressive disorders can last for weeks to lifelong disorder with periodic reoccurring depressive episodes. For the diagnosis of the depression mode (e.g. moderate or severe depression) the Hamilton Depression Rating Scale (HAM-D) (Hamilton, J Neurol Neurosurg Psychiatry, 1960) may be used. The depression mode may be also, in addition or alternatively, rated by alternative scales as the Beck Depression Inventory (BDI), the Montgomery-Asberg Depression Scale (MADRS), the Geriatric Depression Scale (GDS), and/or the Zung Self-Rating Depression Scale (ZSRDS).

Anxiety symptoms comprise *inter alia* panic disorders, generalized anxiety disorder, phobias and posttraumatic stress disorder. Typical symptoms of anxiety are or include avoidance behavior which may lead to social isolation, physical ailments like tachycardia, dizziness and sweating, mental apprehension, stress and/or tensions. The strength of these symptoms ranges from nervousness and discomfort to panic and terror in humans or animals. Most anxiety disorders may last for weeks or even months, some of them even for years and worsen if not suitably treated. For measuring the severity of anxiety symptoms, the Hamilton Anxiety Rating Scale (HAM-A) and/or the State-Trait Anxiety Rating Scale (STAI) can be used.

A CRHR1 antagonist which is used according to the invention in the treatment of depressive symptoms and/or anxiety symptoms in patients having CRH overactivity is a compound of formula (I) 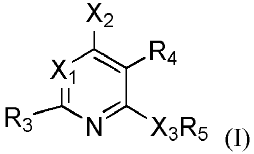 or a pharmaceutically acceptable salt thereof, wherein
X₁ is -CR₆;
X₂ is -NR₁R₂, -CR₁R₂R₉, -C(=CR₂R₁₀)R₁, -NHCHR₁R₂, -OCHR₁R₂, -SCHR₁R₂, -CHR₂OR₁₀, -CHR₂SR₁₀, -C(S)R₂ or -C(O)R₂;
X₃ is NH, O, S, -N(C₁-C₂ alkyl) or -C(R₁₁R₁₂), wherein R₁₁ and R₁₂ are each, independently, hydrogen, trifluoromethyl or methyl, or one of R₁₁ and R₁₂ is cyano and the other is hydrogen or methyl,;
R₁ is C₁-C₆ alkyl which may optionally be substituted with one or two substituents R₇ independently selected from the group consisting of hydroxy, fluoro, chloro, bromo, iodo, CF₃, C₃-C₈ cycloalkyl, C₁-C₄ alkoxy, -O-CO-(C₁-C₄ alkyl), -O-CO-NH(C₁-C₄ alkyl), -O-CO-N(C₁-C₄ alkyl)(C₁-C₂ alkyl), -NH(C₁-C₄ alkyl), -N(C₁-C₂ alkyl)(C₁-C₄ alkyl), -S(C₁-C₄ alkyl), -N(C₁-C₄ alkyl)CO(C₁-C₄ alkyl), - NHCO(C₁-C₄ alkyl), -COO(C₁-C₄ alkyl), -CONH(C₁-C₄ alkyl), -CON(C₁-C₄ alkyl)(C₁-C₂ alkyl), CN, NO₂, -SO(C₁-C₄ alkyl) and -SO₂(C₁-C₄ alkyl), and wherein said C₁-C₆ alkyl and the (C₁-C₄) alkyl moieties in the foregoing R₇ groups may optionally contain one carbon-carbon double or triple bond;
R₂ is C₁-C₁₂ alkyl, C₁-C₁₂ alkoxyalkyl, aryl or -(C₁-C₄ alkylene)aryl wherein said aryl is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiazolyl, benzisoxazolyl, benzimidazolyl, indolyl, oxadiazolyl or benzoxazolyl;
   3- to 8-membered cycloalkyl or -(C₁-C₆ alkylene)cycloalkyl, wherein one or two of the ring carbons of said cycloalkyl having at least 4 ring members and/or the cycloalkyl moiety of said -(C₁-C₆ alkylene)cycloalkyl having at least 4 ring members may optionally be replaced by an oxygen or sulfur atom or by N-Rs wherein R₈ is hydrogen or C₁-C₄ alkyl;
   and wherein each of the foregoing R₂ groups may optionally be substituted with from one to three substituents independently selected from chloro, fluoro and C₁-C₄ alkyl, or with one substituent selected from bromo, iodo, C₁-C₆ alkoxy, -O-CO-(C₁-C₆ alkyl), -O-CO-N(C₁-C₄ alkyl)(C₁-C₂ alkyl), -S(C₁-C₆ alkyl), CN, NO₂, -SO(C₁-C₄ alkyl), and -SO₂(C₁-C₄ alkyl), and wherein said C₁-C₁₂ alkyl and/or the C₁-C₄ alkylene moiety of said -(C₁-C₄ alkylene)aryl may optionally contain one carbon-carbon double or triple bond;
   or -NR₁R₂ or -CR₁R₂R₉ may form a saturated 5- to 8-membered ring which may optionally contain one or two carbon-carbon double bonds and/or in which one or two of the ring carbons may optionally be replaced by an oxygen, nitrogen or sulfur atom and which may be substituted with at least one substituent;
R₃ is methyl, ethyl, fluoro, chloro, bromo, iodo, cyano, methoxy, OCF₃, methylthio, methylsulfonyl, CH₂OH, or CH₂OCH₃;
R₄ is hydrogen, C₁-C₄ alkyl, fluoro, chloro, bromo, iodo, C₁-C₄ alkoxy, trifluoromethoxy, -CH₂OCH₃, -CH₂OCH₂CH₃, -CH₂CH₂OCH₃, -CH₂OCF₃, CF₃, amino, nitro, -NH(C₁-C₄ alkyl), -N(CH₃)₂, -NHCOCH₃ -NHCONHCH₃, -SOₙ(C₁-C₄ alkyl) wherein n is 0, 1 or 2, hydroxy, -CO(C₁-C₄ alkyl), -CHO, cyano or - COO(C₁-C₄ alkyl) wherein said C₁-C₄ alkyl may optionally contain one double or triple bond and/or may optionally be substituted with one substituent selected from hydroxy, amino, -NHCOCH₃, -NH(C₁-C₂ alkyl), -N(C₁-C₂ alkyl)₂, - COO(C₁-C₄ alkyl), -CO(C₁-C₄ alkyl), C₁-C₃ alkoxy, C₁-C₃ thioalkyl, fluoro, chloro, cyano and nitro;
R₅ is phenyl, naphthyl, thienyl, benzothienyl, pyridyl, quinolyl, pyrazinyl, pyrimidyl, furanyl, benzofuranyl, benzothiazolyl, or indolyl,
   wherein each of the above groups R₅ is substituted with from one to three substituents independently selected from fluoro, chloro, C₁-C₆ alkyl and C₁-C₆ alkoxy, or with one substituent selected from hydroxy, iodo, bromo, formyl, cyano, nitro, trifluoromethyl, amino, (C₁-C₆ alkyl)O(C₁-C₆)alkyl, -NHCH₃, - N(CH₃)₂, -COOH, -COO(C₁-C₄ alkyl), -CO(C₁-C₄ alkyl), -SO₂NH(C₁-C₄ alkyl), - SO₂N(C₁-C₄ alkyl)(C₁-C₂ alkyl), -SO₂NH₂, -NHSO₂(C₁-C₄ alkyl), -S(C₁-C₆ alkyl) and SO₂(C₁-C₆ alkyl), and wherein the C₁-C₄ alkyl and the C₁-C₆ alkyl moieties of the foregoing R₅ groups may optionally be substituted with one or two fluoro groups or with one substituent selected from hydroxy, amino, methylamino, dimethylamino and acetyl;
R₆ is hydrogen, methyl, fluoro, chloro, bromo, iodo, cyano, hydroxy, -O(C₁-C₄ alkyl), -C(O)(C₁-C₄ alkyl), -C(O)O(C₁-C₄ alkyl), -OCF₃, CF₃, -CH₂OH, - CH₂OCH₃ or -CH₂OCH₂CH₃;
R₉ is hydrogen, hydroxy, fluoro, or methoxy;
R₁₀ is hydrogen or C₁-C₄ alkyl.

In an embodiment of the invention, the CRHR1 antagonist of the present invention is a compound of formula (I) as defined above, wherein X₁ is -CR₆.

If X₁ is -CR₆, R₆ may be selected from the group consisting of hydrogen, methyl, fluoro, chloro, bromo, iodo, cyano, hydroxy, -O(C₁-C₄ alkyl), -C(O)(C₁-C₄ alkyl), - C(O)O(C₁-C₄ alkyl), -OCF₃, CF₃, -CH₂OH, -CH₂OCH₃ or -CH₂OCH₂CH₃. In a specific embodiment of the CRHR1 antagonists of the invention R₆ is hydrogen.

A further embodiment of the invention relates to a CRHR1 antagonist of formula (I) as defined above, wherein X₂ is selected from the group consisting of -NHCHR₁R₂, - OCHR₁R₂ or -NR₁R₂.

In a specific embodiment, X₂ is -NHCHR₁R₂. In another specific embodiment, X₂ is -OCHR₁R₂. In a further specific embodiment, X₂ is -NR₁R₂.

If X₂ is -NHCHR₁R₂, it is optionally selected from the group consisting of - NHCH(CH₂OCH₃)₂, -NHCH(CH₂OCH₃)(CH₂CH₃), -NHCH(CH₂CH₃)₂, - NHCH(CH₂CH₂OCH₃)₂, -NHCH(CH₃)(CH₂CH₃) or -NHCHR₁R₂, wherein R₁ is ethyl and R₂ is oxadiazolyl substituted with methyl, isopropyl, cyclopropyl, trifluoromethyl, or ethyl. More specifically X₂ is -NHCH(CH₂CH₃)₂. Alternatively, X₂ is -NHCHR₁R₂ wherein R₁ is ethyl and R₂ is oxadiazolyl substituted with methyl. Alternatively, X₂ is -NHCH(CH₂CH₂OCH₃)₂. Alternatively, X₂ is - NHCH(CH₂OCH₃)₂. Alternatively, X₂ is -NHCH(CH₃)(CH₂CH₃). More specifically, X₂ is

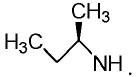

Alternatively, if X₂ is -NR₁R₂, it is optionally selected from the group consisting of - N(CH₂CH₃)(CH₃), -N(CH₂CH₂CH₃)₂, -N(CH₂CH₂CH₃)(CH₂-cyclopropyl), - N(CH₂CH₃)(CH₂CH₂CH₂CH₃), -N(CH₂CH₂OCH₃)₂, or-N(CH₂CH₂OCH₃)(CH₂CH₂CH₃). Alternatively, X₂ is -N(CH₂CH₂OCH₃)₂.

If X₂ is -OCHR₁R₂, it is optionally selected from -OCH(CH₂CH₃)₂, - OCH(CH₂CH₃)CH₃, -OCH(CH₂CH₃)(CH₂CH₂CH₃), -OCH(CH₂CH₃)(CH₂OCH₃). More specifically, X₂ is -OCH(CH₂CH₃)₂.

Further CRHR1 antagonists for use according to the invention encompass compounds of formula (I) as defined above wherein R₁ is C₁-C₆ alkyl.

In another embodiment of the invention, R₁ of formula (I) is C₁ alkyl. In a further embodiment of the invention R₁ of formula (I) is C₂ alkyl. In a further embodiment of the invention, R₁ of formula (I) is C₃ alkyl. In a further embodiment of the invention, R₁ of formula (I) is C₄ alkyl. In a further embodiment, of the invention, R₁ of formula (I) is C₅ alkyl. In a further embodiment of the invention, R₁ of formula (I) is C₆ alkyl.

Any of the aforementioned R₁ groups may optionally be substituted with one or two substituents R₇ independently selected from the group consisting of hydroxy, fluoro, chloro, bromo, iodo, CF₃, C₃-C₈ cycloalkyl, C₁-C₄ alkoxy, -O-CO-(C₁-C₄ alkyl), -O-CO-NH(C₁-C₄ alkyl), -O-CO-N(C₁-C₄ alkyl)(C₁-C₂ alkyl), -NH(C₁-C₄ alkyl), -N(C₁-C₂ alkyl)(C₁-C₄ alkyl), -S(C₁-C₄ alkyl), -N(C₁-C₄ alkyl)CO(C₁-C₄ alkyl), -NHCO(C₁-C₄ alkyl), -COO(C₁-C₄ alkyl), -CONH(C₁-C₄ alkyl), -CON(C₁-C₄ alkyl)(C₁-C₂ alkyl), CN, NO₂, -SO(C₁-C₄ alkyl) and -SO₂(C₁-C₄ alkyl). Said C₁-C₆ alkyl and the (C₁-C₄) alkyl moieties in the foregoing R₇ groups may optionally contain one carbon-carbon double or triple bond.

Specifically, R₁ is C₁-C₆ alkyl substituted with substituent R₇ selected from the group consisting of F, CF₃, C₁-C₄ alkoxy and C₃-C₈ cycloalkyl. More specifically, R₁ is selected from the group consisting of -CH₂OCH₃, -CH₂CH₂OCH₃ and -CH₂-cyclopropyl.

In another embodiment of the invention, R₂ of formula (I) is C₁-C₁₂ alkyl. In another embodiment of the invention, R₂ of formula (I) is C₁-C₁₂ alkoxyalkyl. In a further embodiment, R₂ is aryl. In another embodiment, R₂ is -(C₁-C₄ alkylene)aryl. In a further embodiment of the invention, R₂ is a 3- to 8-membered cycloalkyl. In another embodiment of the invention, R₂ is a -(C₁-C₆ alkylene)cycloalkyl.

Optionally, if the R₂ group is aryl or -(C₁-C₄ alkylene)alkyl said aryl is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiazolyl, benzisoxazolyl, benzimidazolyl, indolyl, oxadiazolyl or benzoxazolyl. More specifically, R₂ is oxadiazolyl.

Optionally, if the R₂ group is a 3- to 8-membered cycloalkyl or a -(C₁-C₆ alkylene)cycloalkyl, one or two of the ring carbons of said cycloalkyl having at least 4 ring members and/or the cycloalkyl moiety of said -(C₁-C₆ alkylene)cycloalkyl having at least 4 ring members may be replaced by an oxygen or sulfur atom or by N-R₈ wherein R₈ is hydrogen or C₁-C₄ alkyl.

Each of the aforementioned R₂ groups may be substituted with from one to three substituents independently selected from chloro, fluoro and C₁-C₄ alkyl. Alternatively, each of the aforementioned R₂ groups may be substituted with one substituent selected from bromo, iodo, C₁-C₆ alkoxy, -O-CO-(C₁-C₆ alkyl), -O-CON(C₁-C₄ alkyl)(C₁-C₂ alkyl), -S(C₁-C₆ alkyl), CN, NO₂, -SO(C₁-C₄ alkyl), and -SO₂(C₁-C₄ alkyl). Optionally, said C₁-C₁₂ alkyl and/or the C₁-C₄ alkylene moiety of said -(C₁-C₄ alkylene)aryl may contain one carbon-carbon double or triple bond.

In a specific embodiment of the invention, R₂ is aryl substituted with from one to three substituents independently selected from chloro, fluoro and C₁-C₄ alkyl. Optionally, R₂ is aryl substituted with one substituent selected from chloro, fluoro and C₁-C₄ alkyl. More specifically, R₂ is oxadiazolyl substituted with one substituent independently selected from chloro, fluoro, C₁-C₄ alkyl, CF₃ and cyclopropyl. Even more specifically, R₂ is oxadiazolyl substituted with methyl.

In another embodiment of the invention, X₂ is -NHCHR₁R₂, wherein R₁ is C₁-C₆ alkyl which may optionally be substituted with one or two substituents R₇ independently selected from the group consisting of hydroxy, fluoro, chloro, bromo, iodo, CF₃, C₃-C₈ cycloalkyl, C₁-C₄ alkoxy, -O-CO-(C₁-C₄ alkyl), -O-CO-NH(C₁-C₄ alkyl), -O-CO-N(C₁-C₄ alkyl)(C₁-C₂ alkyl), -NH(C₁-C₄ alkyl), -N(C₁-C₂ alkyl)(C₁-C₄ alkyl), -S(C₁-C₄ alkyl), -N(C₁-C₄ alkyl)CO(C₁-C₄ alkyl), -NHCO(C₁-C₄ alkyl), - COO(C₁-C₄ alkyl), -CONH(C₁-C₄ alkyl), -CON(C₁-C₄ alkyl)(C₁-C₂ alkyl), CN, NO₂, -SO(C₁-C₄ alkyl) and -SO₂(C₁-C₄ alkyl), and wherein said C₁-C₆ alkyl and the (C₁-C₄) alkyl moieties in the foregoing R₇ groups may optionally contain one carbon-carbon double or triple bond and R₂ is oxadiazolyl substituted with methyl. Optionally, X₂ is -NHCR₁R₂, wherein R₁ is ethyl and R₂ is oxadiazolyl substituted with methyl.

In another embodiment of the invention X₂ is -NR₁R₂ which forms a 5- to 8-membered ring. In a further embodiment of the invention X₂ is -CR₁R₂R₉ which forms a saturated 5- to 8-membered ring. The ring formed by -NR₁R₂ or -CR₁R₂R₉ optionally contains one or two carbon-carbon double bonds. Alternatively or additionally, one or two of the ring carbons may be replaced by an oxygen, nitrogen or sulfur atom.

In a specific embodiment, -NR₁R₂ forms a 5-membered ring containing two double bonds, wherein one of the rings carbons is replaced by an oxygen, nitrogen or sulfur atom. Optionally, -NR₁R₂ forms a 5-membered ring containing two double bonds, wherein one of the rings carbons is replaced by a nitrogen atom. More specifically, the 5-membered ring formed by -NR₁R₂ is a pyrazol-1-yl. Alternatively, -NR₁R₂ forms a 5-membered ring containing two double bonds, wherein one of the rings carbons is replaced by a sulfur atom.

The above described 5- to 8-membered ring formed by -NR₁R₂ or -CR₁R₂R₉ may be substituted with at least one substituent. In one embodiment of the invention, the 5-to 8-membered ring formed by -NR₁R₂ or -CR₁R₂R₉ is substituted with one substituent selected from C₁-C₄ alkyl or a 4-8 membered ring. In another embodiment of the invention, the 5- to 8-membered ring formed by -NR₁R₂ or - CR₁R₂R₉ is substituted with two substituents independently selected from C₁-C₄ alkyl or a 4-8 membered ring. In another embodiment of the invention, the 5- to 8-membered ring formed by -NR₁R₂ or -CR₁R₂R₉ is substituted with three substituents independently selected from C₁-C₄ alkyl or a 4-8 membered ring. In another embodiment of the invention, the 5- to 8-membered ring formed by -NR₁R₂ or - CR₁R₂R₉ is substituted with four substituents independently selected from C₁-C₄ alkyl or a 4-8 membered ring.

Said 4-8 membered ring may be saturated or may contain one to three double bonds. Optionally, the 4-8 membered ring is saturated. Additionally, one carbon atom of said 4-8 membered ring may be replaced by CO. Alternatively, one carbon atom of said 4-8 membered ring may be replaced by SO₂. Alternatively or additionally, one to four carbon atom(s) of the 4-8 membered ring may be replaced by nitrogen. Optionally, two carbon atoms are replaced by nitrogen. More specifically, two carbon atoms of the 4-8 membered ring are replaced by nitrogen and one carbon atom is replaced by CO. Alternatively, two carbon atoms of the 4-8 membered ring are replaced by nitrogen and one carbon atom is replaced by SO₂. In a specific embodiment of the invention, the above described 5- to 8-membered ring formed by -NR₁R₂ is substituted with

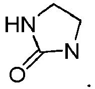

Optionally, X₂ is

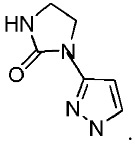

In an alternative embodiment, the 4-8 membered ring contains one to three double bonds. Optionally, the 4-8 membered ring contains two double bonds. Additionally, one carbon atom of the 4-8 membered ring may be replaced by nitrogen and one carbon atom may be replaced by sulfur. Optionally, the above described ring is a 5 membered ring. In a specific embodiment of the invention, the above described 5- to 8-membered ring formed by -NR₁R₂ or -CR₁R₂R₉ is substituted with thiazol. Optionally, X₂ is a 5-membered ring formed by -NR₁R₂ containing two double bonds, wherein one of the rings carbons is replaced by a nitrogen atom and wherein said 5-membered ring is substituted with thiazol.

In a specific embodiment of the invention, the 5-membered ring formed by -NR₁R₂, containing two double bonds and wherein one carbon atom is replaced by a nitrogen atom, is substituted with at least one C₁-C₄ alkyl. In another embodiment, the 5-membered ring formed by -NR₁R₂, containing two double bonds and wherein one carbon atom is replaced by a nitrogen atom, is substituted with two C₁-C₄ alkyl substituents. In another embodiment, the 5-membered ring formed by -NR₁R₂, containing two double bonds and wherein one carbon atom is replaced by a nitrogen atom, is substituted with three C₁-C₄ alkyl substituents. Optionally, X₂ is 3,5-dialkylpyrazol-1-yl, such as 3,5-diethylpyrazol-1-yl.

A further embodiment of the invention encompasses CRHR1 antagonists of formula (I) as defined above, wherein X₃ is NH, O, or NCH₃., Specifically, X₃ is O or NH, more specifically X₃ is O.

The CRHR1 antagonist of the invention further encompasses a compound of formula (I) as defined above, wherein R₃ is methyl. In a further embodiment of the invention, R₄ of formula (I) as defined above is C₁-C₄ alkyl. Optionally R₄ of formula (I) as defined above is methyl. Alternatively, R₄ of formula (I) as defined above is ethyl. The present invention also encompasses CRHR1 antagonists wherein both R₃ and R₄ are methyl.

In another embodiment of the invention, the CRHR1 antagonist is a compound of formula (I) as defined above, wherein R₅ is phenyl substituted with from one to three substituents independently selected from fluoro, chloro, C₁-C₆ alkyl and C₁-C₆ alkoxy, or with one substituent selected from hydroxy, iodo, bromo, formyl, cyano, nitro, trifluoromethyl, amino, (C₁-C₆ alkyl)O(C₁-C₆)alkyl, -NHCH₃, -N(CH₃)₂, - COOH, -COO(C₁-C₄ alkyl), -CO(C₁-C₄ alkyl), -SO₂NH(C₁-C₄ alkyl), -SO₂N(C₁-C₄ alkyl)(C₁-C₂ alkyl), -SO₂NH₂, -NHSO₂(C₁-C₄ alkyl), -S(C₁-C₆ alkyl) and SO₂(C₁-C₆ alkyl). The C₁-C₄ alkyl and the C₁-C₆ alkyl moieties of the foregoing R₅ groups may be substituted with one or two fluoro groups or with one substituent selected from hydroxy, amino, methylamino, dimethylamino and acetyl.

In an embodiment of the invention, the CRHR1 antagonist encompasses a compound of formula (I) as defined above, wherein R₅ is phenyl substituted with from one to three substituent(s) independently selected from the group CH₃, CH₂CH₃, OCH₃, Cl, F, and CF₃. In a specific embodiment of the invention, the CRHR1 antagonist encompasses a compound of formula (I) as defined above, wherein R₅ is phenyl substituted with one substituent independently selected from the group CH₃, CH₂CH₃, OCH₃, Cl, F, and CF₃. In another embodiment of the invention, the CRHR1 antagonist encompasses a compound of formula (I) as defined above, wherein R₅ is phenyl substituted with two substituents independently selected from the group CH₃, CH₂CH₃, OCH₃, Cl, F, CF₃. In another embodiment of the invention, the CRHR1 antagonist encompasses a compound of formula (I) as defined above, wherein R₅ is phenyl substituted with three substituents independently selected from the group CH₃, CH₂CH₃, OCH₃, Cl, F, and CF₃.

In a specific embodiment of the CRHR1 antagonist of the invention, R₅ is phenyl substituted with two substituents, wherein the two substituents are CH₃ and OCH₃.

In another specific embodiment of the CRHR1 antagonist of the invention, R₅ is phenyl substituted with three substituents, wherein the three substituents are CH₃.

In another specific embodiment of the CRHR1 antagonist of the invention, the three substituents of said phenyl are independently selected from CH₃ and Cl.

Specifically, R₅ is 2-chloro-4-methoxy-5-methyl-phenyl, 2,4,6-trimethyl-phenyl, 2,4,6-trichloro-phenyl, 4-methoxy-2-methyl-phenyl, 2,4-dichloro-phenyl, 4-chloro-2,6-dimethyl-phenyl, 2,4-dimethyl-phenyl, or 2,4-dimethoxy-phenyl. More specifically R₅ is 2,4,6-trimethyl-phenyl or 4-chloro-2,6-dimethyl-phenyl. Even more specifically, R₅ is 4-chloro-2,6-dimethyl-phenyl.

In another specific embodiment of the invention the CRHR1 antagonist for use in the treatment of depressive symptoms and/or anxiety symptoms in patients having CRH overactivity is a compound of formula (VI) 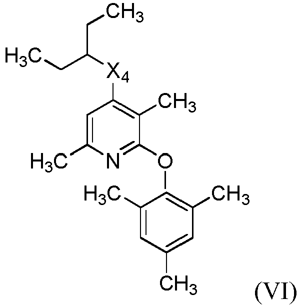 or a pharmaceutically acceptable salt thereof, wherein
X₄ is O or NH.

In another embodiment of the invention, the CRHR1 antagonist is selected from the group consisting of CP-316,311and CP-376,395.

In a particular embodiment of the invention, the CRHR1 antagonist is CP-316,311.

The corresponding structural formulas of the above-mentioned CRHR1 antagonists for use in the present invention are set out in Table 1 below:

**Table 1**

| **Structural formula** | **CRHR1 antagonist (name)** |
|---|---|
| 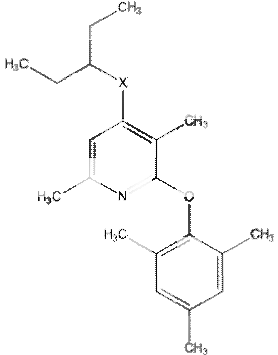 | X = O **CP-316,311** |
| | X = NH **CP-376,395** |

The above-described CRHR1 antagonists are for use in the treatment of depressive symptoms and/or anxiety symptoms in patients having CRH overactivity, wherein CRH overactivity is detected by determining the status of a marker indicative for CRH overactivity.

The marker indicative for CRH overactivity is selected from the group consisting of a biomarker, or a set of biomarkers.

The one or more biomarkers may be obtained by a genome-wide screening for single nucleotide polymorphisms in patients with depressive and/or anxiety symptoms.

For example, the biomarker or set of biomarkers constituting a marker for CRH activity may be selected from one or more biomarkers of the group comprising:
- SNP rs6437726,
- SNP rs1986684,
- SNP rs7380830,
- SNP rs3903768,
- SNP rs7325978,
- SNP rs13585,
- SNP rs9368373,
- SNP rs10935354,
- SNP rs8095703,
- SNP rs10206851,
- SNP rs9542977,
- SNP rs4942879,
- SNP rs9542954,
- SNP rs1593478,
- SNP rs9542951,
- SNP rs2188534,
- SNP rs12524124,
- SNP rs4352629,
- SNP rs7448716,
- SNP rs11873533,
- SNP rs10062658,
- SNP rs12547917,
- SNP rs1038268,
- SNP rs2375811,
- SNP rs1352671,
- SNP rs364331,
- SNP rs1924949,
- SNP rs11025990,
- SNP rs3758562,
- SNP rs10156056.

In particular, the biomarker or set of biomarkers constituting a marker for CRH activity may be selected from one or more biomarkers of the group comprising:
- SNP rs6437726 which is represented by a single polymorphic change at position 201 of SEQ ID NO: 1, wherein in one or two alleles the wild-type nucleotide A is replaced by indicator nucleotide G,
- SNP rs1986684 which is represented by a single polymorphic change at position 201 of SEQ ID NO: 2, wherein in one or two alleles the wild-type nucleotide A is replaced by indicator nucleotide G,
- SNP rs7380830 which is represented by a single polymorphic change at position 201 of SEQ ID NO: 3, wherein in one or two alleles the wild-type nucleotide C is replaced by indicator nucleotide T,
- SNP rs3903768 which is represented by a single polymorphic change at position 201 of SEQ ID NO: 4, wherein in one or two alleles the wild-type nucleotide A is replaced by indicator nucleotide G,
- SNP rs7325978 which is represented by a single polymorphic change at position 201 of SEQ ID NO: 5, wherein in one or two alleles the wild-type nucleotide C is replaced by indicator nucleotide T,
- SNP rs13585 which is represented by a single polymorphic change at position 185 of SEQ ID NO: 6, wherein in one or two alleles the wild-type nucleotide C is replaced by indicator nucleotide T,
- SNP rs9368373 which is represented by a single polymorphic change at position 201 of SEQ ID NO: 7, wherein in one or two alleles the wild-type nucleotide C is replaced by indicator nucleotide T,
- SNP rs10935354 which is represented by a single polymorphic change at position 201 of SEQ ID NO: 8, wherein in one or two alleles the wild-type nucleotide A is replaced by indicator nucleotide G,
- SNP rs8095703 which is represented by a single polymorphic change at position 201 of SEQ ID NO: 9, wherein in one or two alleles the wild-type nucleotide A is replaced by indicator nucleotide G,
- SNP rs10206851 which is represented by a single polymorphic change at position 201 of SEQ ID NO: 10, wherein in one or two alleles the wild-type nucleotide C is replaced by indicator nucleotide T,
- SNP rs9542977 which is represented by a single polymorphic change at position 201 of SEQ ID NO: 11, wherein in one or two alleles the wild-type nucleotide C is replaced by indicator nucleotide T,
- SNP rs4942879 which is represented by a single polymorphic change at position 201 of SEQ ID NO: 12, wherein in one or two alleles the wild-type nucleotide A is replaced by indicator nucleotide G,
- SNP rs9542954 which is represented by a single polymorphic change at position 201 of SEQ ID NO: 13, wherein in one or two alleles the wild-type nucleotide A is replaced by indicator nucleotide C,
- SNP rs1593478 which is represented by a single polymorphic change at position 201 of SEQ ID NO: 14, wherein in one or two alleles the wild-type nucleotide C is replaced by indicator nucleotide T,
- SNP rs9542951which is represented by a single polymorphic change at position 201 of SEQ ID NO: 15, wherein in one or two alleles the wild-type nucleotide A is replaced by indicator nucleotide G,
- SNP rs2188534 which is represented by a single polymorphic change at position 200 of SEQ ID NO: 16, wherein in one or two alleles the wild-type nucleotide G is replaced by indicator nucleotide T,
- SNP rs12524124 which is represented by a single polymorphic change at position 201 of SEQ ID NO: 17, wherein in one or two alleles the wild-type nucleotide A is replaced by indicator nucleotide G,
- SNP rs4352629 which is represented by a single polymorphic change at position 201 of SEQ ID NO: 18, wherein in one or two alleles the wild-type nucleotide C is replaced by indicator nucleotide T,
- SNP rs7448716 which is represented by a single polymorphic change at position 201 of SEQ ID NO: 19, wherein in one or two alleles the wild-type nucleotide A is replaced by indicator nucleotide G,
- SNP rs11873533 which is represented by a single polymorphic change at position 201 of SEQ ID NO: 20, wherein in one or two alleles the wild-type nucleotide A is replaced by indicator nucleotide C,
- SNP rs10062658 which is represented by a single polymorphic change at position 201 of SEQ ID NO: 21, wherein in one or two alleles the wild-type nucleotide A is replaced by indicator nucleotide G,
- SNP rs12547917 which is represented by a single polymorphic change at position 201 of SEQ ID NO: 22, wherein in one or two alleles the wild-type nucleotide C is replaced by indicator nucleotide T,
- SNP rs1038268 which is represented by a single polymorphic change at position 201 of SEQ ID NO: 23, wherein in one or two alleles the wild-type nucleotide C is replaced by indicator nucleotide T,
- SNP rs2375811 which is represented by a single polymorphic change at position 201 of SEQ ID NO: 24, wherein in one or two alleles the wild-type nucleotide A is replaced by indicator nucleotide G,
- SNP rs1352671 which is represented by a single polymorphic change at position 201 of SEQ ID NO: 25, wherein in one or two alleles the wild-type nucleotide A is replaced by indicator nucleotide C,
- SNP rs364331 which is represented by a single polymorphic change at position 201 of SEQ ID NO: 26, wherein in one or two alleles the wild-type nucleotide A is replaced by indicator nucleotide C,
- SNP rs1924949 which is represented by a single polymorphic change at position 201 of SEQ ID NO: 27, wherein in one or two alleles the wild-type nucleotide A is replaced by indicator nucleotide G,
- SNP rs11025990 which is represented by a single polymorphic change at position 201 of SEQ ID NO: 28, wherein in one or two alleles the wild-type nucleotide A is replaced by indicator nucleotide G,
- SNP rs3758562 which is represented by a single polymorphic change at position 201 of SEQ ID NO: 29, wherein in one or two alleles the wild-type nucleotide A is replaced by indicator nucleotide G,
- SNP rs10156056 which is represented by a single polymorphic change at position 201 of SEQ ID NO: 30, wherein in one or two alleles the wild-type nucleotide C is replaced by indicator nucleotide G.

In a specific embodiment, the set of biomarkers comprises at least 15, at least 20, at least 25 or all of the following biomarkers:
- SNP rs6437726,
- SNP rs1986684,
- SNP rs7380830,
- SNP rs3903768,
- SNP rs7325978,
- SNP rs13585,
- SNP rs9368373,
- SNP rs10935354,
- SNP rs8095703,
- SNP rs10206851,
- SNP rs9542977,
- SNP rs4942879,
- SNP rs9542954,
- SNP rs1593478,
- SNP rs9542951,
- SNP rs2188534,
- SNP rs12524124,
- SNP rs4352629,
- SNP rs7448716,
- SNP rs11873533,
- SNP rs10062658,
- SNP rs12547917,
- SNP rs1038268,
- SNP rs2375811,
- SNP rs1352671,
- SNP rs364331,
- SNP rs1924949,
- SNP rs11025990,
- SNP rs3758562,
- SNP rs10156056.

In another specific embodiment, the set of biomarkers comprises at least 15, at least 20, at least 25 or all of the following biomarkers:
- SNP rs6437726 which is represented by a single polymorphic change at position 201 of SEQ ID NO: 1, wherein in one or two alleles the wild-type nucleotide A is replaced by indicator nucleotide G,
- SNP rs1986684 which is represented by a single polymorphic change at position 201 of SEQ ID NO: 2, wherein in one or two alleles the wild-type nucleotide A is replaced by indicator nucleotide G,
- SNP rs7380830 which is represented by a single polymorphic change at position 201 of SEQ ID NO: 3, wherein in one or two alleles the wild-type nucleotide C is replaced by indicator nucleotide T,
- SNP rs3903768 which is represented by a single polymorphic change at position 201 of SEQ ID NO: 4, wherein in one or two alleles the wild-type nucleotide A is replaced by indicator nucleotide G,
- SNP rs7325978 which is represented by a single polymorphic change at position 201 of SEQ ID NO: 5, wherein in one or two alleles the wild-type nucleotide C is replaced by indicator nucleotide T,
- SNP rs13585 which is represented by a single polymorphic change at position 185 of SEQ ID NO: 6, wherein in one or two alleles the wild-type nucleotide C is replaced by indicator nucleotide T,
- SNP rs9368373 which is represented by a single polymorphic change at position 201 of SEQ ID NO: 7, wherein in one or two alleles the wild-type nucleotide C is replaced by indicator nucleotide T,
- SNP rs10935354 which is represented by a single polymorphic change at position 201 of SEQ ID NO: 8, wherein in one or two alleles the wild-type nucleotide A is replaced by indicator nucleotide G,
- SNP rs8095703 which is represented by a single polymorphic change at position 201 of SEQ ID NO: 9, wherein in one or two alleles the wild-type nucleotide A is replaced by indicator nucleotide G,
- SNP rs10206851 which is represented by a single polymorphic change at position 201 of SEQ ID NO: 10, wherein in one or two alleles the wild-type nucleotide C is replaced by indicator nucleotide T,
- SNP rs9542977 which is represented by a single polymorphic change at position 201 of SEQ ID NO: 11, wherein in one or two alleles the wild-type nucleotide C is replaced by indicator nucleotide T,
- SNP rs4942879 which is represented by a single polymorphic change at position 201 of SEQ ID NO: 12, wherein in one or two alleles the wild-type nucleotide A is replaced by indicator nucleotide G,
- SNP rs9542954 which is represented by a single polymorphic change at position 201 of SEQ ID NO: 13, wherein in one or two alleles the wild-type nucleotide A is replaced by indicator nucleotide C,
- SNP rs1593478 which is represented by a single polymorphic change at position 201 of SEQ ID NO: 14, wherein in one or two alleles the wild-type nucleotide C is replaced by indicator nucleotide T,
- SNP rs9542951which is represented by a single polymorphic change at position 201 of SEQ ID NO: 15, wherein in one or two alleles the wild-type nucleotide A is replaced by indicator nucleotide G,
- SNP rs2188534 which is represented by a single polymorphic change at position 200 of SEQ ID NO: 16, wherein in one or two alleles the wild-type nucleotide G is replaced by indicator nucleotide T,
- SNP rs12524124 which is represented by a single polymorphic change at position 201 of SEQ ID NO: 17, wherein in one or two alleles the wild-type nucleotide A is replaced by indicator nucleotide G,
- SNP rs4352629 which is represented by a single polymorphic change at position 201 of SEQ ID NO: 18, wherein in one or two alleles the wild-type nucleotide C is replaced by indicator nucleotide T,
- SNP rs7448716 which is represented by a single polymorphic change at position 201 of SEQ ID NO: 19, wherein in one or two alleles the wild-type nucleotide A is replaced by indicator nucleotide G,
- SNP rs11873533 which is represented by a single polymorphic change at position 201 of SEQ ID NO: 20, wherein in one or two alleles the wild-type nucleotide A is replaced by indicator nucleotide C,
- SNP rs10062658 which is represented by a single polymorphic change at position 201 of SEQ ID NO: 21, wherein in one or two alleles the wild-type nucleotide A is replaced by indicator nucleotide G,
- SNP rs12547917 which is represented by a single polymorphic change at position 201 of SEQ ID NO: 22, wherein in one or two alleles the wild-type nucleotide C is replaced by indicator nucleotide T,
- SNP rs1038268 which is represented by a single polymorphic change at position 201 of SEQ ID NO: 23, wherein in one or two alleles the wild-type nucleotide C is replaced by indicator nucleotide T,
- SNP rs2375811 which is represented by a single polymorphic change at position 201 of SEQ ID NO: 24, wherein in one or two alleles the wild-type nucleotide A is replaced by indicator nucleotide G,
- SNP rs1352671 which is represented by a single polymorphic change at position 201 of SEQ ID NO: 25, wherein in one or two alleles the wild-type nucleotide A is replaced by indicator nucleotide C,
- SNP rs364331 which is represented by a single polymorphic change at position 201 of SEQ ID NO: 26, wherein in one or two alleles the wild-type nucleotide A is replaced by indicator nucleotide C,
- SNP rs1924949 which is represented by a single polymorphic change at position 201 of SEQ ID NO: 27, wherein in one or two alleles the wild-type nucleotide A is replaced by indicator nucleotide G,
- SNP rs11025990 which is represented by a single polymorphic change at position 201 of SEQ ID NO: 28, wherein in one or two alleles the wild-type nucleotide A is replaced by indicator nucleotide G,
- SNP rs3758562 which is represented by a single polymorphic change at position 201 of SEQ ID NO: 29, wherein in one or two alleles the wild-type nucleotide A is replaced by indicator nucleotide G,
- SNP rs10156056 which is represented by a single polymorphic change at position 201 of SEQ ID NO: 30, wherein in one or two alleles the wild-type nucleotide C is replaced by indicator nucleotide G.

For example, the set of biomarkers consists of the following biomarkers:
- SNP rs6437726,
- SNP rs1986684,
- SNP rs7380830,
- SNP rs3903768,
- SNP rs7325978,
- SNP rs13585,
- SNP rs9368373,
- SNP rs10935354,
- SNP rs8095703,
- SNP rs10206851,
- SNP rs9542977,
- SNP rs4942879,
- SNP rs9542954,
- SNP rs1593478,
- SNP rs9542951,
- SNP rs2188534,
- SNP rs12524124,
- SNP rs4352629,
- SNP rs7448716,
- SNP rs11873533,
- SNP rs10062658,
- SNP rs12547917,
- SNP rs1038268,
- SNP rs2375811,
- SNP rs1352671,
- SNP rs364331,
- SNP rs1924949,
- SNP rs11025990,
- SNP rs3758562,
- SNP rs10156056.

In another example, the set of biomarkers consists of the following biomarkers:
- SNP rs6437726 which is represented by a single polymorphic change at position 201 of SEQ ID NO: 1, wherein in one or two alleles the wild-type nucleotide A is replaced by indicator nucleotide G,
- SNP rs1986684 which is represented by a single polymorphic change at position 201 of SEQ ID NO: 2, wherein in one or two alleles the wild-type nucleotide A is replaced by indicator nucleotide G,
- SNP rs7380830 which is represented by a single polymorphic change at position 201 of SEQ ID NO: 3, wherein in one or two alleles the wild-type nucleotide C is replaced by indicator nucleotide T,
- SNP rs3903768 which is represented by a single polymorphic change at position 201 of SEQ ID NO: 4, wherein in one or two alleles the wild-type nucleotide A is replaced by indicator nucleotide G,
- SNP rs7325978 which is represented by a single polymorphic change at position 201 of SEQ ID NO: 5, wherein in one or two alleles the wild-type nucleotide C is replaced by indicator nucleotide T,
- SNP rs13585 which is represented by a single polymorphic change at position 185 of SEQ ID NO: 6, wherein in one or two alleles the wild-type nucleotide C is replaced by indicator nucleotide T,
- SNP rs9368373 which is represented by a single polymorphic change at position 201 of SEQ ID NO: 7, wherein in one or two alleles the wild-type nucleotide C is replaced by indicator nucleotide T,
- SNP rs10935354 which is represented by a single polymorphic change at position 201 of SEQ ID NO: 8, wherein in one or two alleles the wild-type nucleotide A is replaced by indicator nucleotide G,
- SNP rs8095703 which is represented by a single polymorphic change at position 201 of SEQ ID NO: 9, wherein in one or two alleles the wild-type nucleotide A is replaced by indicator nucleotide G,
- SNP rs10206851 which is represented by a single polymorphic change at position 201 of SEQ ID NO: 10, wherein in one or two alleles the wild-type nucleotide C is replaced by indicator nucleotide T,
- SNP rs9542977 which is represented by a single polymorphic change at position 201 of SEQ ID NO: 11, wherein in one or two alleles the wild-type nucleotide C is replaced by indicator nucleotide T,
- SNP rs4942879 which is represented by a single polymorphic change at position 201 of SEQ ID NO: 12, wherein in one or two alleles the wild-type nucleotide A is replaced by indicator nucleotide G,
- SNP rs9542954 which is represented by a single polymorphic change at position 201 of SEQ ID NO: 13, wherein in one or two alleles the wild-type nucleotide A is replaced by indicator nucleotide C,
- SNP rs1593478 which is represented by a single polymorphic change at position 201 of SEQ ID NO: 14, wherein in one or two alleles the wild-type nucleotide C is replaced by indicator nucleotide T,
- SNP rs9542951which is represented by a single polymorphic change at position 201 of SEQ ID NO: 15, wherein in one or two alleles the wild-type nucleotide A is replaced by indicator nucleotide G,
- SNP rs2188534 which is represented by a single polymorphic change at position 200 of SEQ ID NO: 16, wherein in one or two alleles the wild-type nucleotide G is replaced by indicator nucleotide T,
- SNP rs12524124 which is represented by a single polymorphic change at position 201 of SEQ ID NO: 17, wherein in one or two alleles the wild-type nucleotide A is replaced by indicator nucleotide G,
- SNP rs4352629 which is represented by a single polymorphic change at position 201 of SEQ ID NO: 18, wherein in one or two alleles the wild-type nucleotide C is replaced by indicator nucleotide T,
- SNP rs7448716 which is represented by a single polymorphic change at position 201 of SEQ ID NO: 19, wherein in one or two alleles the wild-type nucleotide A is replaced by indicator nucleotide G,
- SNP rs11873533 which is represented by a single polymorphic change at position 201 of SEQ ID NO: 20, wherein in one or two alleles the wild-type nucleotide A is replaced by indicator nucleotide C,
- SNP rs10062658 which is represented by a single polymorphic change at position 201 of SEQ ID NO: 21, wherein in one or two alleles the wild-type nucleotide A is replaced by indicator nucleotide G,
- SNP rs12547917 which is represented by a single polymorphic change at position 201 of SEQ ID NO: 22, wherein in one or two alleles the wild-type nucleotide C is replaced by indicator nucleotide T,
- SNP rs1038268 which is represented by a single polymorphic change at position 201 of SEQ ID NO: 23, wherein in one or two alleles the wild-type nucleotide C is replaced by indicator nucleotide T,
- SNP rs2375811 which is represented by a single polymorphic change at position 201 of SEQ ID NO: 24, wherein in one or two alleles the wild-type nucleotide A is replaced by indicator nucleotide G,
- SNP rs1352671 which is represented by a single polymorphic change at position 201 of SEQ ID NO: 25, wherein in one or two alleles the wild-type nucleotide A is replaced by indicator nucleotide C,
- SNP rs364331 which is represented by a single polymorphic change at position 201 of SEQ ID NO: 26, wherein in one or two alleles the wild-type nucleotide A is replaced by indicator nucleotide C,
- SNP rs1924949 which is represented by a single polymorphic change at position 201 of SEQ ID NO: 27, wherein in one or two alleles the wild-type nucleotide A is replaced by indicator nucleotide G,
- SNP rs11025990 which is represented by a single polymorphic change at position 201 of SEQ ID NO: 28, wherein in one or two alleles the wild-type nucleotide A is replaced by indicator nucleotide G,
- SNP rs3758562 which is represented by a single polymorphic change at position 201 of SEQ ID NO: 29, wherein in one or two alleles the wild-type nucleotide A is replaced by indicator nucleotide G,
- SNP rs10156056 which is represented by a single polymorphic change at position 201 of SEQ ID NO: 30, wherein in one or two alleles the wild-type nucleotide C is replaced by indicator nucleotide G.

The status of a marker indicative for CRH overactivity may be determined by a method comprising determining the status of a marker as defined herein in a nucleic acid isolated from a patient's sample, wherein the presence of indicator nucleotides as defined herein is indicative for CRH overactivity.The term "biomarker", as used herein, relates to any nucleic acid sequence of any length, or a derivative thereof, which comprises a polymorphic variant as defined in:
- SNP rs6437726,
- SNP rs1986684,
- SNP rs7380830,
- SNP rs3903768,
- SNP rs7325978,
- SNP rs13585,
- SNP rs9368373,
- SNP rs10935354,
- SNP rs8095703,
- SNP rs10206851,
- SNP rs9542977,
- SNP rs4942879,
- SNP rs9542954,
- SNP rs1593478,
- SNP rs9542951,
- SNP rs2188534,
- SNP rs12524124,
- SNP rs4352629,
- SNP rs7448716,
- SNP rs11873533,
- SNP rs10062658,
- SNP rs12547917,
- SNP rs1038268,
- SNP rs2375811,
- SNP rs1352671,
- SNP rs364331,
- SNP rs1924949,
- SNP rs11025990,
- SNP rs3758562, and/or
- SNP rs10156056.

A biomarker may, for instance, be represented by a nucleic acid molecule of a length of e.g. 1 nt, 2 nt, 3 nt, 4 nt, 5 nt, 10 nt, 15 nt, 20 nt, 25 nt, 30 nt, 35 nt, 40 nt, 45 nt, 50 nt, 60 nt, 70 nt, 80 nt, 90 nt, 100 nt, 200 nt, 300 nt, 400 nt, 500 nt, 1000 nt, 2000 nt, or more or any length in between these lengths. The representing nucleic acid may be any suitable nucleic acid molecule, e.g. a DNA molecule, e.g. a genomic DNA molecule or a cDNA molecule, or a RNA molecule, or a derivative thereof. The biomarker may further be represented by translated forms of the nucleic acid, e.g. a peptide or protein as long as the polymorphic modification leads to a corresponding modification of the peptide or protein. Corresponding information may be readily available to the skilled person from databases such as the NCBI SNP repository and NCBI Genbank.

The SNPs as described herein may be present on the Watson or the Crick strand, with presence of the corresponding base. I.e. if, for example, a polymorphism is present on the Watson strand as A, it is present on the Crick strand as T, if the polymorphism is present on the Watson strand as T, it is present on the Crick strand as A, if the polymorphism is present on the Watson strand as G, it is present on the Crick strand as C, and if the polymorphism is present on the Watson strand as C, it is present on the Crick strand as G, and vice versa. Also the insertion or deletion of bases may be detected on the Watson and/or the Crick strand, with correspondence as defined above. For analytic purposes the strand identity may be defined, or fixed, or may be choose at will, e.g. in dependence on factors such the availability of binding elements, GC- content etc. Furthermore, for the sake of accuracy, the SNP may be defined on both strands (Crick and Watson) at the same time, and accordingly be analyzed.

A "polymorphic site" or "polymorphic variant" as used herein relates to the position of a polymorphism or SNP as described herein within the genome or portion of a genome of a subject, or within a genetic element derived from the genome or portion of a genome of a subject.

The term "wildtype sequence" as used herein refers to the sequence of an allele, which does not show the CRH overactivity phenotype according to the present invention. The term may further refer to the sequence of the non phenotype-associated allele with the highest prevalence within a population, e.g. within a Caucasian population.

The term "indicator sequence" as used herein refers to the sequence of an allele, which shows an association with a CRH overactivity phenotype according to the present invention. The term "indicator nucleotide" as used herein refers to the identity of the nucleotide at the position of a SNP or polymorphic site as defined herein, associated with a CRH overactivity phenotype according to the present invention. The term may refer to a non-wildtype nucleotide at positions of SEQ ID NO: 1 to 30 as described below:
- SNP rs6437726 which is represented by a single polymorphic change at position 201 of SEQ ID NO: 1, wherein in one or two alleles the wild-type nucleotide A is replaced by indicator nucleotide G,
- SNP rs1986684 which is represented by a single polymorphic change at position 201 of SEQ ID NO: 2, wherein in one or two alleles the wild-type nucleotide A is replaced by indicator nucleotide G,
- SNP rs7380830 which is represented by a single polymorphic change at position 201 of SEQ ID NO: 3, wherein in one or two alleles the wild-type nucleotide C is replaced by indicator nucleotide T,
- SNP rs3903768 which is represented by a single polymorphic change at position 201 of SEQ ID NO: 4, wherein in one or two alleles the wild-type nucleotide A is replaced by indicator nucleotide G,
- SNP rs7325978 which is represented by a single polymorphic change at position 201 of SEQ ID NO: 5, wherein in one or two alleles the wild-type nucleotide C is replaced by indicator nucleotide T,
- SNP rs13585 which is represented by a single polymorphic change at position 185 of SEQ ID NO: 6, wherein in one or two alleles the wild-type nucleotide C is replaced by indicator nucleotide T,
- SNP rs9368373 which is represented by a single polymorphic change at position 201 of SEQ ID NO: 7, wherein in one or two alleles the wild-type nucleotide C is replaced by indicator nucleotide T,
- SNP rs10935354 which is represented by a single polymorphic change at position 201 of SEQ ID NO: 8, wherein in one or two alleles the wild-type nucleotide A is replaced by indicator nucleotide G,
- SNP rs8095703 which is represented by a single polymorphic change at position 201 of SEQ ID NO: 9, wherein in one or two alleles the wild-type nucleotide A is replaced by indicator nucleotide G,
- SNP rs10206851 which is represented by a single polymorphic change at position 201 of SEQ ID NO: 10, wherein in one or two alleles the wild-type nucleotide C is replaced by indicator nucleotide T,
- SNP rs9542977 which is represented by a single polymorphic change at position 201 of SEQ ID NO: 11, wherein in one or two alleles the wild-type nucleotide C is replaced by indicator nucleotide T,
- SNP rs4942879 which is represented by a single polymorphic change at position 201 of SEQ ID NO: 12, wherein in one or two alleles the wild-type nucleotide A is replaced by indicator nucleotide G,
- SNP rs9542954 which is represented by a single polymorphic change at position 201 of SEQ ID NO: 13, wherein in one or two alleles the wild-type nucleotide A is replaced by indicator nucleotide C,
- SNP rs1593478 which is represented by a single polymorphic change at position 201 of SEQ ID NO: 14, wherein in one or two alleles the wild-type nucleotide C is replaced by indicator nucleotide T,
- SNP rs9542951 which is represented by a single polymorphic change at position 201 of SEQ ID NO: 15, wherein in one or two alleles the wild-type nucleotide A is replaced by indicator nucleotide G,
- SNP rs2188534 which is represented by a single polymorphic change at position 200 of SEQ ID NO: 16, wherein in one or two alleles the wild-type nucleotide G is replaced by indicator nucleotide T,
- SNP rs12524124 which is represented by a single polymorphic change at position 201 of SEQ ID NO: 17, wherein in one or two alleles the wild-type nucleotide A is replaced by indicator nucleotide G,
- SNP rs4352629 which is represented by a single polymorphic change at position 201 of SEQ ID NO: 18, wherein in one or two alleles the wild-type nucleotide C is replaced by indicator nucleotide T,
- SNP rs7448716 which is represented by a single polymorphic change at position 201 of SEQ ID NO: 19, wherein in one or two alleles the wild-type nucleotide A is replaced by indicator nucleotide G,
- SNP rs11873533 which is represented by a single polymorphic change at position 201 of SEQ ID NO: 20, wherein in one or two alleles the wild-type nucleotide A is replaced by indicator nucleotide C,
- SNP rs10062658 which is represented by a single polymorphic change at position 201 of SEQ ID NO: 21, wherein in one or two alleles the wild-type nucleotide A is replaced by indicator nucleotide G,
- SNP rs12547917 which is represented by a single polymorphic change at position 201 of SEQ ID NO: 22, wherein in one or two alleles the wild-type nucleotide C is replaced by indicator nucleotide T,
- SNP rs1038268 which is represented by a single polymorphic change at position 201 of SEQ ID NO: 23, wherein in one or two alleles the wild-type nucleotide C is replaced by indicator nucleotide T,
- SNP rs2375811 which is represented by a single polymorphic change at position 201 of SEQ ID NO: 24, wherein in one or two alleles the wild-type nucleotide A is replaced by indicator nucleotide G,
- SNP rs1352671 which is represented by a single polymorphic change at position 201 of SEQ ID NO: 25, wherein in one or two alleles the wild-type nucleotide A is replaced by indicator nucleotide C,
- SNP rs364331 which is represented by a single polymorphic change at position 201 of SEQ ID NO: 26, wherein in one or two alleles the wild-type nucleotide A is replaced by indicator nucleotide C,
- SNP rs1924949 which is represented by a single polymorphic change at position 201 of SEQ ID NO: 27, wherein in one or two alleles the wild-type nucleotide A is replaced by indicator nucleotide G,
- SNP rs11025990 which is represented by a single polymorphic change at position 201 of SEQ ID NO: 28, wherein in one or two alleles the wild-type nucleotide A is replaced by indicator nucleotide G,
- SNP rs3758562 which is represented by a single polymorphic change at position 201 of SEQ ID NO: 29, wherein in one or two alleles the wild-type nucleotide A is replaced by indicator nucleotide G,
- SNP rs10156056 which is represented by a single polymorphic change at position 201 of SEQ ID NO: 30, wherein in one or two alleles the wild-type nucleotide C is replaced by indicator nucleotide G.

The term "determining the status of a biomarker" or determining the status of a marker" as used herein refers to any suitable method or technique of detecting the identity of an SNP, e.g. at the positions of the biomarkers described herein. The determination method may be a sequencing technique or a technique based on complementary nucleic acid binding. The context of the indicated positions, as well as the strand may differ, e.g. from patient to patient, or from sample to sample etc.

The term "allele" or "allelic sequence" as used herein refers to a particular form of a gene or a particular nucleotide, e.g. a DNA sequence at a specific chromosomal location or locus. In certain embodiments of the present invention a SNP as defined herein may be found at or on one of two alleles in the human genome of a single subject. In further, specific embodiments, a SNP as defined herein may also be found at or on both alleles in the human genome of a single subject. The presence of an indicator nucleotide or an indicator triplet as defined herein on both alleles may have a higher predictive value than the presence of an indicator nucleotide or an indicator triplet on one allele only, the other allele comprising a wildtype genotype. The presence or absence of indicator nucleotides or indicator triplets on one or two alleles may be connected or linked with an algorithm for predicting the a treatment response to CRHR1 antagonists in patients with depressive symptoms and/or anxiety symptoms as described herein.

A person skilled in the art would be able to derive the exact position, nucleotide sequence, and indicator sequence from the above identified rs-nomenclature, e.g. from suitable database entries and associated information systems, e.g. the Single Nucleotide Polymorphism database (dbSNP) which is incorporated herein by reference. The information may also be retrievable in case of changes to the nomenclature, or to the surrounding sequence elements, e.g. based on history functions of a suitable database.

The term "isolated nucleic acid molecule" a used herein refers to a nucleic acid entity, e.g. DNA, RNA etc, wherein the entity is substantially free of other biological molecules, such as, proteins, lipids, carbohydrates, other nucleic acids or other material, such as cellular debris and growth media. Generally, the term "isolated" is not intended to refer to the complete absence of such material, or to the absence of water, buffers, or salts, unless they are present in amounts which substantially interfere with the methods of the present invention.

In another embodiment, the biomarker constituting a marker for CRH overactivity is REM density.

Such a marker or set of markers or combination of markers as described above may be used for predicting a treatment response to CRH antagonists in patients with depressive and/anxiety symptoms.

Exemplary embodiments for determining the status of a marker indicative for CRH overactivity are described below.

In particular, it has been found that a treatment response to CRH antagonists in patients with depressive symptoms and/or anxiety symptoms can be reliably predicted by using a machine-learning based prediction algorithm derived from the association of SNPs with values indicative for CRH overactivity.

The term "treatment response to CRHR1 antagonists in patients with depressive symptoms and/or anxiety symptoms" in the sense of the invention refers to a response in a patient with depressive symptoms and/or anxiety symptoms during and/or after the treatment with one or more CRHR1 antagonists compared to before the treatment. The response may range from a partial alleviation of the symptoms to a complete remission of the symptoms, indicated by the change of symptoms strength and/or frequency of relapse of individual symptoms and/or the mean change on a depression scale, e.g. as described herein. The response can occur shortly after treatment or after a certain time period. A decrease in symptom severity from pretreatment of 25% or more is usually considered a partial alleviation of symptoms. Remission may be defined as achieving a value of 8 or less on the Hamilton Depression Rating Scale (HAM-D) or equivalent values on other rating scales named below.

A further aspect of the disclosure concerns the provision of an algorithm for predicting a treatment response to CRHR1 antagonists in patients with depressive symptoms and/or anxiety symptoms. The method may comprise the following steps:
(a) performing a single nucleotide polymorphism (SNP) genotyping analysis in a group of patients with depressive symptoms and/or anxiety symptoms;
(b) determining a value indicative for CRH activity in each patient of the group, wherein a value indicative for CRH overactivity is indicative or predictive for a patient responding to a treatment with a CRH1 antagonist;
(c) identifying at least one SNP associated with a value indicative for CRH overactivity as determined in step (b);
(d) determining the algorithm by machine-learning from the association of the at least one SNP identified in step (c) with the value indicative for CRH overactivity.

In a step (a), a single nucleotide polymorphism (SNP) genotyping analysis in a group of patients with depressive symptoms and/or anxiety symptoms is performed.

A "group of patients" as used herein comprises at least two patients, such as at least 10 patients, or at least 100 patients, or at least 150 patients. Patients included in the analysis of step (a) may exhibit at least a moderate to severe depressive mode. The group of patients may comprise patients with CRH overactivity and/or patients with normal CRH activity.

The term "polymorphism" as used herein refers to a variation in the genome of individuals, including, insertions, deletions, point mutations and translocations.

The term "single nucleotide polymorphism" is well understood by the skilled person and refers to a point mutation at a certain position in the nucleotide sequence. In other words, only one nucleotide differs in a certain region.

The nucleotide, that is present in the majority of the population, is also referred to as wild-type allele or major allele. As used herein, this state is defined as "absence of a SNP".

The specific nucleotide that is present in the minority of the population is also referred as the point mutation, mutated nucleotide or minor allele. As used herein this state is defined as "presence of a SNP".

In theory, the wild-type allele could be mutated to three different nucleotides. However, the event of a mutation to a first nucleotide in the reproductive cells of an individual that gets established in a population occurs very rarely. The event that the same position is mutated to a second nucleotide and established in the population virtually never occurs and can be therefore neglected. Therefore, as used herein, a certain nucleotide position in the genome of an individual can have two states, the wild-type state (absence of a SNP) and the mutated state (presence of a SNP).

The term "single nucleotide polymorphism (SNP) genotyping analysis" as used herein refers to a test of determining in one or several patients the presence or absence of at least one SNP, typically several SNPs, and in some embodiments all (known) SNPs the human genome, including endogenous and exogenous regions. In particular, a SNP genotyping analysis as used herein may not be limited to the CRHR1 gene or to genes of the CRH pathway. In other words, an SNP genotyping analysis as used herein can be a genome-wide screening for SNPs.

SNP genotyping analysis can be performed by methods known in the art such as microarray analysis or sequencing analysis or PCR related methods or mass spectrometry or 5'-nuclease assays or allele specific hybridization or high-throughput variants of these techniques or combinations thereof. These and other methods are known in the art. See for example Rampal, DNA Arrays: Methods and Protocols (Methods in Molecular Biology) 2010; Graham & Hill, DNA Sequencing Protocols (Methods in Molecular Biology) 2001; Schuster, Nat. Methods, 2008 and Brenner, Nat. Biotech., 2000; Mardis, Annu Rev Genomics Hum Genet., 2008. Genomewide arrays can be purchased from different suppliers such as Illumia and Affymetix.

For example, the determination of the nucleotide sequence and/or molecular structure may be carried out through allele-specific oligonucleotide (ASO)-dot blot analysis, primer extension assays, iPLEX SNP genotyping, Dynamic allele-specific hybridization (DASH) genotyping, the use of molecular beacons, tetra primer ARMS PCR, a flap endonuclease invader assay, an oligonucleotide ligase assay, PCR-single strand conformation polymorphism (SSCP) analysis, quantitative real-time PCR assay, SNP microarray based analysis, restriction enzyme fragment length polymorphism (RFLP) analysis, targeted resequencing analysis and/or whole genome sequencing analysis.

In some embodiments of the disclosure, any of the methods described herein comprises the determination of the haplotype for two copies of the chromosome comprising the SNPs identified herein.

A "subject's sample" as used herein may be any sample derived from any suitable part or portion of a subject's body. In some embodiments, blood or saliva samples are used. The sample used in the context of the present disclosure should be collected in a clinically acceptable manner, in particular in a way that nucleic acids and/or proteins are preserved.

The term "primer" may denote an oligonucleotide that acts as an initiation point of nucleotide synthesis under conditions in which synthesis of a primer extension product complementary to a nucleic acid strand is induced.

The term "probe" may denote an oligonucleotide that selectively hybridizes to a target nucleic acid under suitable conditions.

The primers and probes may be generated such that they are able to discriminate between wild-type allele or mutated allele of the position of a SNP to be analyzed. Methods for the design of sequence specific primers and probes are known in the art (see e.g. William B. Coleman, Gregory J. Tsongalis, Molecular Diagnostics: For the Clinical Laboratorian, 2007; Weiner et al. Genetic Variation: A Laboratory Manual, 2010).

Typically, a SNP is considered in the genotyping analysis if it occurs in a certain percentage in the population, for example in at least 5 % or at least 10% of the population. In other words, the minor allele frequency (MAF) is larger than 0.05 or 0.10 (MAF > 0.05 or MAF > 0.10).

For the SNP genotyping analysis a nucleic acid or DNA sample from a subject or patient may be used. The nucleic acid or DNA sample can be a blood sample, a hair sample, a skin sample or a salvia sample of the patient. Any other sample obtainable from the patient and containing patient nucleic acid or DNA can also be used. The sample can be collected from the patient by any method known in the art. For example, a blood sample can be taken from a patient by use of a sterile needle. The collection of salvia out of the mouth and throat of the patient can be performed by use of a sterile cotton bud or by flushing said area and collecting the flushing solution.

Usually, the nucleic acid or DNA is extracted or isolated or purified from the sample prior to SNP genotyping analysis. Any method known in the art may be used for DNA extraction or purification. Suitable methods comprise *inter alia* steps such as centrifugation steps, precipitation steps, chromatography steps, dialyzing steps, heating steps, cooling steps and/or denaturation steps. For some embodiments, a certain nucleic acid or DNA content in the sample may be reached. Nucleic acid or DNA content can be measured for example via UV spectrometry as described in the literature. However, in alternative embodiments SNP genotyping analysis may also be performed by using a non-extracted or non-purified sample.

DNA amplification may also be useful prior to the SNP analysis step. Any method known in the art can be used for DNA amplification. The sample can thus be provided in a concentration and solution appropriate for the SNP analysis.

The analyzed SNPs may be represented by values 0, 1 or 2. The value "0" may indicate that the SNP is present on none of the two homologous chromosomes. The value "1" may indicate that the SNP is present on one of the two homologous chromosomes. The value "2" may indicate that the SNP is present on both homologous chromosomes. Homologous chromosomes correspond to each other in terms of chromosome length, gene loci and staining pattern. One is inherited from the mother, the other is inherited from the father.

In a step (b) of the method for providing a prediction algorithm, a value indicative for CRH activity in each patient is determined.

Steps (c) and (d) of the method for providing a prediction algorithm may analyze the association of the analyzed SNPs with the value indicative for CRH overactivity and/or normal CRH activity and generate an algorithm for predicting the treatment response to CRHR1 antagonists.

In an exemplary embodiment of the disclosure, the group of patients may be split into two sets of similar size and similar values for descriptors such as demographic descriptors or clinical descriptors. These two sets are hereinafter also referred to as "training set" and "test set".

In step (c) of the method of this exemplary embodiment of the disclosure, at least one SNP associated with the value indicative for CRH overactivity as determined in step (b) is identified in the training set.

Further, there can be at least two alternatives for the result provided by the prediction algorithm. First, the result may be a categorical answer whether the patient responds to CRHR1 antagonist treatment or not. Second, the prediction algorithm may provide the answer to which degree the patient responds or does not respond to the treatment. Depending on the desired result provided by the prediction algorithm the way of determining the algorithm may differ.

In the alternative that the prediction algorithm will analyze whether a patient responds or does not respond to CRHR1 antagonist treatment, the values indicative for CRH activity may be provided as logic data variable (Boolean type; 0 vs. 1; true vs. false, high vs. low responder). Therefore, if the test performed to determine values indicative for CRH overactivity provides a data range, the patients may be dichotomized by a threshold into high vs. low responders.

In the alternative that the test will analyze to which degree the patient responds or does not respond to the CRHR1 antagonist treatment, the values indicative for CRH activity may be provided as numerical values.

Typically, SNPs that are modified in a significant percentage of the population are used in the method for providing a prediction algorithm. For example, only SNPs with a minor allele frequency (MAF) greater than 0.05 or 0.10 may be selected for further analysis. This means that only SNPs that are modified in at least 5 % or 10% of the population are selected for further analysis.

Association for all SNPs with the value indicative for CRH activity is tested by an association analysis testing the likelihood for a patient to be CRH overactive vs. CRH non-overactive in dependence of the genotype of said patient. Said association analysis may be conducted for example by an additive genetic model and/or by a logistic regression. A SNP is e.g. identified to be associated with a value indicative for CRH overactivity if the corresponding p-value is at least 1 x 10⁻³ or at least 1 x 10⁻⁴ or at least 1 x 10⁻⁵. The lower the p-value the more the SNP is associated with a value indicative for CRH overactivity. Accordingly, a SNP is e.g. identified to be associated with a value indicative for normal CRH activity if the corresponding p-value is at least 1 x 10⁻³ or at least 1 x 10⁻⁴ or at least 1 x 10⁻⁵.

In the step (d) of this exemplary embodiment of the disclosure, the algorithm for predicting a treatment response to CRHR1 antagonists may be determined by the use of SNPs in the test set by a machine learning method.

The term "algorithm for predicting" as used herein may refer to a classification function (also known as binary classification test).

The term "machine-learning" as used herein may refer to a method known to the person skilled in the art of machine learning. In particular, machine learning is concerned with the design and development of algorithms that allow computers to evolve behaviors based on empirical data, such as from sensor data or databases. It may be selected from the group consisting of artificial neural network learning, decision tree learning, support vector machine learning, Bayesian network learning, clustering, and regression analysis.

The term "reliable prediction of the treatment response to CRHR1 antagonists" as used herein may refer to a high performance of the prediction algorithm. The evaluation of the performance of the prediction algorithm may depend on the problem the algorithm is applied for. If the algorithm is used to identify patients that are likely to response to the treatment with CRHR1 antagonists the performance is usually expressed by a high accuracy and/or sensitivity and/or precision. If patients should be identified which are likely not to respond to the treatment with CRHR1 antagonists, specificity and/or negative predictive value are typical statistical measures to describe the performance of the prediction algorithm.

For optimizing the prediction performance of the algorithm, the step of determining the algorithm by a machine-learning method in a first subset of the test set and testing the prediction performance in an second independent subset of the test set may be repeated based on different numbers and groups of SNPs, until the desired prediction performance is reached.

Accuracy, sensitivity, precision, specificity and negative predictive value are exemplary statistical measure of the performance of the prediction algorithm. In the following, examples are given for determining the performance of the prediction algorithm.

As used herein, accuracy may be calculated as (number of true positives + number of true negatives) / (number of true positives + number of false positives + number of true negatives + number of false negatives), e.g. (number of patients correctly diagnosed as responding to CRHR1 antagonist + number of patients correctly diagnosed as not responding to CRHR1 antagonist) / (number of patients correctly diagnosed as responding to CRHR1 antagonist + number of patients wrongly diagnosed as responding to CRHR1 antagonist + number of patients correctly diagnosed as not responding to CRHR1 antagonist + number of patients wrongly diagnosed as not responding to CRHR1 antagonist). The accuracy of prediction may e.g. be at least 60%, at least 70%, at least 80% or at least 90%.

A used herein, sensitivity may be calculated as (true positives) / (true positives + false negatives), e.g.: (number of patients correctly diagnosed as responding to CRHR1 antagonist) / (number of patients correctly diagnosed as responding to CRHR1 antagonist + number of patients wrongly diagnosed as not responding to CRHR1 antagonist). The sensitivity of prediction may be at least 60%, at least 70%, at least 80% or at least 90%.

As used herein, precision (also referred to as positive predictive value) may be calculated as (true positives) / (true positives + false positives), e.g.: (number of patients correctly diagnosed as responding to CRHR1 antagonist) / (number of patients correctly diagnosed as responding to CRHR1 antagonist + number of patients wrongly diagnosed as responding to CRHR1 antagonist). The precision of prediction may be at least 60%, at least 70%, at least 80% or at least 90%.

As used herein, specificity is calculated as (true negatives) / (true negatives + false positives), e.g.: (number of patients correctly diagnosed as not responding to CRHR1 antagonist) / (number of patients correctly diagnosed as not responding to CRHR1 antagonist + number of patients wrongly diagnosed as responding to CRHR1 antagonist). The specificity of prediction may be at least 60%, at least 70%, at least 80% or at least 90%.

As used herein, negative predictive value is calculated as (true negatives) / (true negatives + false negatives), e.g.: (number of patients correctly diagnosed as not responding to CRHR1 antagonist) / (number of patients correctly diagnosed as not responding to CRHR1 antagonist + number of patients wrongly diagnosed as not responding to CRHR1 antagonist). The negative predictive value may be at least 60%, at least 70%, at least 80% or at least 90%.

Other statistical measures useful for describing the performance of the prediction algorithm are geometric mean of sensitivity and specificity, geometric mean of positive predictive value and negative predictive value, F-measure and area under ROC curve, and the positive and negative likelihood ratios, the false discovery rate and Matthews correlation coefficient. These measures and method for their determination are well known in the art.

In general, a prediction algorithm with high sensitivity may have low specificity and vice versa. The decision to select an algorithm having certain statistical characteristics such as accuracy, sensitivity or specificity may also depend on the costs associated with a treatment with a CRHR1 antagonist should the prediction be positive and/or whether such a treatment is detrimental in cases where the result is a false positive.

For a prediction whether a patient likely responds to the treatment with CRHR1 antagonists the prediction algorithm may be based on a number of SNPs sufficient to achieve a prediction sensitivity and/or precision of at least 55%, optionally at least 80%.

For the prediction whether it is unlikely that a patient responds to the treatment with CRHR1 antagonists the prediction algorithm may be based on a number of SNPs sufficient to achieve a prediction specificity and/or negative predictive value of at least 55%, optionally at least 80%.

For a prediction whether a patient responds to a treatment with CRHR1 antagonists or not the prediction algorithm may be based on a number of SNPs sufficient to achieve sensitivity and/or precision and/or specificity and/or negative predictive value of at least 55%, optionally at least 80%.

In one embodiment of the disclosure, a number N of SNPs is associated with a value indicative for CRH overactivity or normal CRH activity in step (d) of the method for providing an algorithm and/or the presence or absence of a number N of SNPs is determined in step (a) of the method for predicting a treatment response, wherein N is sufficient to provide an accuracy of at least 80% and a sensitivity of at least 70% and a specificity of at least 70%. In another embodiment, a number N of SNPs is associated with a value indicative for CRH overactivity in step (d) of the method for providing an algorithm and/or the presence or absence of a number N of SNPs is determined in step (a) of the method for predicting a treatment response, wherein N is sufficient to provide an accuracy of at least 85% and a sensitivity of at least 80% and a specificity of at least 80%.

Typically, at least 10, at least 20, at least 25 or at least 30 SNPs are used for determination of the algorithm in step (d) of the method for providing a prediction algorithm.

The skilled person in the art knows that the use of different machine-learning methods and adapting parameters used therein can be also used for improvement of the prediction reliability. The whole statistical work-flow can be automated by a computer.

Another aspect of the disclosure is a method for predicting a treatment response to CRHR1 antagonists in patients with depressive symptoms and/or anxiety symptoms, wherein the method may comprise the following steps:
(a) determining in a nucleic acid sample obtained from a patient the presence or absence of at least one single nucleotide polymorphism (SNP) associated with a value indicative for CRH overactivity;
(b) predicting the treatment response to CRHR1 antagonists by linking the prediction algorithm provided by the method described above with the presence or absence of the at least one SNP determined in step (a).

"Linking an algorithm for predicting a treatment response to CRHR1 antagonists in patients having depressive symptoms and/or anxiety symptoms with the presence or absence of the at least one SNP" as used herein may refer to using such an algorithm to predict the treatment response based on the determined presence or absence of the at least one SNP, e.g. by integrating the at least one SNP determined in step (a) of the above method by the algorithm.

In one embodiment of the disclosure the method comprises a further step of obtaining a nucleic acid sample from a patient preceding the step of SNP determination.

In one embodiment of the disclosure for the method for predicting a treatment response to CRHR1 antagonists in patients with depressive symptoms and/or anxiety symptoms, step (a) comprises determining at least one of the SNPs, optionally all of the SNPs which were associated with a value indicative for CRH overactivity when determining the algorithm by machine-learning from this association.

Typically, a SNP is considered in the genotyping analysis of step (a) of the method of prediction if it occurs in a certain percentage in the population, for example in at least 5 % or at least 10% of the population. In other words, the minor allele frequency (MAF) is larger than 0.05 (MAF > 0.05) or 0.10 (MAF > 0.10).

For the SNP genotyping analysis of step (a) of the method of prediction, a nucleic acid or a DNA sample from a patient may be used. The nucleic acid or DNA sample can be a blood sample, a hair sample, a skin sample or a salvia sample of the patient. Any other sample of the patient containing nucleic acid or DNA can also be used. The sample can be collected from the patient by any method known in the art. For example, a blood sample can be taken from a patient by use of a sterile needle. The collection of salvia out of the mouth and throat of the patient can be performed by use of a sterile cotton bud or by flushing said area and collecting the flushing solution.

Usually, the nucleic acid or DNA is extracted or purified from the sample prior to SNP genotyping analysis. Any method known in the art may be used for DNA extraction or purification. Suitable methods comprise *inter alia* steps such as centrifugation steps, precipitation steps, chromatography steps, dialyzing steps, heating steps, cooling steps and/or denaturation steps. For some embodiments, a certain DNA content in the sample may be reached. Nucleic acid or DNA content can be measured for example via UV spectrometry as known in art. However, the SNP genotyping analysis may also be performed by using a non-extracted or non-purified sample.

Nucleic acid or DNA amplification may also be useful prior to the SNP analysis step. Any method known in the art can be used for DNA amplification. The sampled can thus be provided in a concentration and solution appropriate for the SNP analysis.

The analyzed SNPs may be represented by values 0, 1 or 2. The value "0" may indicate that the SNP is present on none of the two homologous chromosomes. The value "1" may indicate that the SNP is present on one of the two homologous chromosomes. The value "2" may indicate that the SNP is present on both homologous chromosomes. Homologous chromosomes correspond to each other in terms of chromosome length, gene loci and staining pattern. One is inherited from the mother, the other is inherited from the father.

In order to determine SNPs, SNP-specific primers and/or probes, a primer extension reaction, SNP microarrays, DNA-sequencing may be used. These reagents and methods are routinely used in the art (see for example Chen, PCR Cloning Protocols (Methods in Molecular Biology) 2002; Schuster, Nat. Methods, 2008, Rampal, DNA Arrays: Methods and Protocols (Methods in Molecular Biology) 2010; Brenner, Nat. Biotech., 2000; Mardis, Annu Rev Genomics Hum Genet., 2008). For example, MALDI-TOF (matrix-assisted laser desorption ionization time of flight) mass spectrometry on the Sequenom platform (San Diego, USA) may be used to genotype the selected variants. For primer selection, multiplexing and assay design, and the mass-extension for producing primer extension products the MassARRAY Assay Designer software may be used using the sequences presented in table 2 as input. The MassARRAY Typer 3.4 software may be used for genotype calling. Any other reagent or method known to the person skilled in the art may be used in order to detect the SNPs in an individual.

In one embodiment of the disclosure, at least one SNP determined in step (a) and used for the prediction algorithm of step (b) is a SNP selected from the group consisting of SNPs as shown in table 2 and an SNP in strong linkage disequilibrium with any of the SNPs shown in table 2.

**Table 2: SNPs (together with flanking sequences) which may be used to predict the response to CRHR1 antagonists in patients with depressive symptoms and/or anxiety symptoms. The position of the SNP is indicated in bold as [wild-type allele/mutated allele].**

| **SNP_ID** | **SEQUENCE** |
|---|---|
| RS6437726 SEQ ID NO. 1 | |
| RS1986684 SEQ ID NO. 2 | |
| RS7380830 SEQ ID NO. 3 | |
| RS3903768 SEQ ID NO. 4 | |
| RS7325978 SEQ ID NO. 5 | |
| RS13585 SEQ ID NO. 6 | |
| RS9368373 SEQ ID NO. 7 | |
| RS10935354 SEQ ID NO. 8 | |
| RS8095703 SEQ ID NO. 9 | |
| RS10206851 SEQ ID NO. 10 | |
| RS9542977 SEQ ID NO. 11 | |
| RS4942879 SEQ ID NO. 12 | |
| RS9542954 SEQ ID NO. 13 | |
| RS1593478 SEQ ID NO. 14 | |
| RS9542951 SEQ ID NO. 15 | |
| RS2188534 SEQ ID NO. 16 | |
| RS12524124 SEQ ID NO. 17 | |
| RS4352629 SEQ ID NO. 18 | |
| RS7448716 SEQ ID NO. 19 | |
| RS11873533 SEQ ID NO. 20 | |
| RS10062658 SEQ ID NO. 21 | |
| RS12547917 SEQ ID NO. 22 | |
| RS1038268 SEQ ID NO. 23 | |
| RS2375811 SEQ ID NO. 24 | |
| RS1352671 SEQ ID NO. 25 | |
| RS364331 SEQ ID NO. 26 | |
| RS1924949 SEQ ID NO. 27 | |
| RS11025990 SEQ ID NO. 28 | |
| RS3758562 SEQ ID NO. 29 | |
| RS10156056 SEQ ID NO. 30 | |

Polymorphisms in linkage disequilibrium with a SNP of table 2 can be identified by methods known in the art. For example, Develin and Risch (Genomics, 1995) provide guidance for determining the parameter delta (also referred to as the "r") as a standard measure of the disequilibrium. Gabriel et al. (Science, 2002) provides instructions for finding the maximal r² value in populations for disease gene mapping. Further, Carlson et al. (Am. J. Hum. Genet. (2003) disclose methods for selecting and analyzing polymorphisms based on linkage disequilibrium for disease gene association mapping. Stoyanovich and Pe'er (Bioinformatics, 2008) show that polymorphisms in linkage disequilibrium with identified SNPs have virtually identical response profiles. Currently, several databases provide datasets that can be searched for polymorphisms in strong linkage disequilibrium, which can be accessed by the following addresses: http://1000.genomes.org, http://www.hapmap.org, http://www.broadinsitute.org/mpg/snap. An example workflow for determining SNPs linkage disequilibrium to a specific SNP is outlined in Uhr et al. (Neuron, 2008).

SNP in strong linkage disequilibrium as used herein means that the SNP is in linkage disequilibrium with an r² higher than 0.7 or higher than 0.8 in the tested population or an ethnically close reference population with the identified SNP.

In another embodiment of the disclosure, at least 20, optionally at least 25 or at least 30 SNPs selected from the group of table 2 are determined in step (a) and integrated by the prediction algorithm in step (b). For example, all intergenic SNPs of table 2 are determined in step (a) and integrated by the prediction algorithm in step (b). In another exemplary embodiment, all SNPs selected from the group of table 2 are determined in step (a) and integrated by the prediction algorithm of step (b).

Typically, in step (a) of the method of predicting a treatment response to CRHR1 antagonists, the presence or absence of those SNPs is determined which were identified to be associated with values indicative for normal CRH activity or CRH overactivity and were thus considered when determining the prediction algorithm by machine-learning as described above.

In one embodiment of the disclosure for the method for predicting a treatment response to CRHR1 antagonists in patients with depressive symptoms and/or anxiety symptoms, the method as described above may be accompanied by analyzing the rapid-eye-movement (REM) sleep, e.g. during night sleep of a patient in a sleep EEG.

In other embodiments, an increase in REM sleep may serve as a biomarker to identify patients who would benefit from treatment with a CRHR1 antagonist.

REM sleep typically comprises a characteristic coincidence of nearly complete muscle atonia, a waking-like pattern of brain oscillations and rapid eye movements (REMs). The amount of REMs during consecutive REM sleep episodes is usually increasing throughout the night. Single and short REMs with low amplitude can be characteristic for initial parts of REM sleep. The amount of REMs, in particular within the first REM sleep episode, can be of clinical relevance. Recent clinical and animal data supports the correlation of REM density with an increased CRH activity. For example, Kimura et al. (Mol. Psychiatry, 2010) showed that mice overexpressing CRH in the forebrain exhibit constantly increased rapid eye movement (REM) sleep compared to wildtype mice. In addition, it could be shown that treatment with the CRHR1 antagonist DMP696 could reverse the REM enhancement.

Further, the SNP analysis and REM density analysis as described herein may be combined for predicting the response of patients with depressive symptoms and/or anxiety symptoms to treatment with a CRHR1 antagonist. The REM analysis may be carried out before, concomitant or after the SNP analysis. For example, the REM density analysis may be carried out on persons that where identified by the SNP analysis as CRH hyperdrive patients.

The recording of a "sleep-EEG" (also referred to "polysomatic recordings") may comprise electroencephalography (EEG), vertical and horizontal elecrooculography (EOG), electromyography (EMG) and/or electrocardiography (ECG). In EOG, muscle activities of right and left eye may be recorded by electrooculograms (one or typically two channels) in order to visualize the phasic components of REM sleep.

"REM analysis" or "analyzing the rapid-eye-movement (REM)" may refer to a method comprising recoding of muscle activities of right and left eye by EOG and then analyzing the electrooculogram. The recognition of REM in the electrooculogram may be done manually (for example by standard guidelines Rechtschaffen and Kales, 1968, Bethesda, MD: National Institute of Neurological Diseases and Blindness).

The invention is further described in the following example which is solely for the purpose of illustrating specific embodiments of the invention.

### Example 1

### Methods:

### Patients:

Patients with unipolar or bipolar depression admitted as in-patients to the Max Planck Institute of Psychiatry (MPI), Munich, Germany, for treatment of a depressive episode were included in the study. Patients were diagnosed by psychiatrists according to the Diagnostic and Statistical Manual of Mental Disorders (DSM) IV criteria. Patients with bipolar disorder or depressive disorder due to a general medical or neurological condition were excluded, as were patients with a lifetime diagnosis of drug abuse and depressive symptoms secondary to alcohol or substance abuse or dependency. Ethnicity was recorded using a self-report sheet for nationality, first language and ethnicity of the patient and of all four grandparents. All patients were Caucasian and part of the Munich-Antidepressant-Response-Signature (MARS) project (Hennings et al., J Psychiatr Res., 2009) (www.mars-depression.de). They were treated with antidepressant medications according to doctor's choice. Severity of depressive symptoms was assessed at admission and at the time of the dex/CRH test by trained raters using the 17-item Hamilton Depression Rating Scale (HAM-D, Hamilton, J Neurol Neurosurg Psychiatry, 1960). 192 patients fulfilling the criteria for at least a moderate to severe depressive episode (HAM-D≥18) at both time points and who had been administered a dex/CRH test within 10 days of in-patients admission and had genome-wide SNP data were included in this analysis. The study was approved by the Ethics Committee of the Ludwig Maximilians University in Munich, Germany, and written informed consent was obtained from all subjects.

### Dex/CRH test:

The dex/CRH test was administered as described in detail in Heuser et al. (J Psychiatr Res., 1994). In brief, subjects were pre-treated with 1.5 mg of dexamethasone per os at 11 pm. The following day, at 3 pm, 3.30 pm, 3.45 pm, 4 pm and 4.15 pm blood was drawn. An intravenous bolus of 100 µg of human CRH (Ferring, Kiel, Germany) was given at 3.02 pm. Plasma ACTH concentrations were assessed by an immunometric assay without extraction (Nichols Institute, San Juan Capistrano, California; USA). The neuroendocrine response to the dex/CRH test was analyzed using the total area under the curve (AUC) of the ACTH response.

### SNP Genotyping:

After enrollment in the study 40 ml of EDTA blood was drawn from each patient. DNA was extracted from fresh blood using the Puregene® whole blood DNA-extraction kit (Gentra Systems Inc; MN). Genotyping was performed on Illumina Human 610k quad genotyping arrays (Illumina Inc., San Diego, USA) according to the manufacturer's standard protocols. The average call rate exceeded 99 %, with samples below 98 % being either retyped or excluded from the study. The reproducibility for samples genotyped twice was 99.99 % or better.

### Data analysis:

To identify genetic predictors for the ACTH response to the dex/CRH test in patients with moderate to severe depression, the 192 patients were randomly split into a training (N = 96) and test set (N = 96). The demographic and clinical descriptors of these two samples are given in table 3.

**Table 3:**

| Demographic and clinical description of the 192 patients in the trainings and test set | | | |
|---|---|---|---|
| | **training set** | **test set** | **p-value** |
| | | | |
| **N** | **96** | **96** | |
| | | | |
| **% female** | **52,1** | **57,2** | **n.s.** |
| **% bipolar (1 or 2)** | **12,5** | **12,5** | **n.s.** |
| **% with psychotic symptoms** | **15,2** | **11,4** | **n.s.** |
| | | | |
| **age (mean years (SD))** | **47,2 (14,2)** | **51,3 (13,2)** | **0,038** |
| **BMI at admission (mean SD)** | **24,9 (4,1)** | **24,9 (4,3)** | **n.s.** |
| **age-on (mean years (SD))** | **34,72 (14,5)** | **36,6 (15,9)** | **n.s.** |
| **number of previous episodes (mean N (SD))** | **3,2 (4,0)** | **3,3 (4,7)** | **n.s.** |
| **depression severity at admission (mean HAMD-17 score (SD))** | **26,4 (4,4)** | **27,7 (4,8)** | **0,058** |
| **depression severity at dex-crh test (mean HAMD-17 score (SD))** | **26,9 (5,0)** | **28,5 (5,0)** | **0,029** |
| | | | |
| | | | |
| **Cortisol AUC (mean (SD))** | **3611,4 (3414)** | **3688,6 (3491)** | **n.s.** |
| **ACTH AUC (mean (SD))** | **1246 (910)** | **1482,8 (1319)** | **n.s.** |
| **days after admission when dex-CRH test was performed** | **6,1 (2,2)** | **5,9 (2,2)** | **n.s.** |

After natural log transformation of the AUC of the ACTH response in the dex/CRH test, patients were dichotomized into high vs. low responders. For the ACTH AUC the cut-off was In ACTH AUC > 7.0. This placed 46.4% (89 out of a total of 192 individuals) in the high responder class. See the corresponding histogram in Figure 1.

Using an additive genetic model and a logistic regression with sex and age as covariates in PLINK (http://pngu.mgh.harvard.edu/purcell/plink/), the association of all SNPs with a MAF > 0.05 were tested on the 610k arrays with the dichotomized response for cortisol and ACTH in the dex/CRH test in the training set. The top 30 associated SNPs were then used to predict either ACTH or cortisol response status in the test set using a "Probabilistic Neural Network und General Regression Neural Network" approach (implementation DTREG 10.3.3 www.dtreg.com). All values were derived from a 20 fold cross validation.

### Results:

The top 30 association with ACTH response status are given in table 4. Association p-values ranged from 5.0 e-5 to 2.3 e-4 for the ACTH response.

The genotypes for the 30 SNPs each were then used to predict ACTH response status in the second, independent test cohort (subgroup of the test set).

**Table 4: List of 30 SNPs used to predict ACTH AUC in the second test cohort.**

| SNP | Chromosome | Coordinate_HG18 | P-value training | GeneVariant | GeneName |
|---|---|---|---|---|---|
| | | | | | |
| rs6437726 | chr3 | 108321446 | 5.037e-005 | INTERGENIC | N/A |
| rs1986684 | chr11 | 110629697 | 5.287e-005 | UPSTREAM | N/A |
| rs7380830 | chr5 | 66022641 | 5.459e-005 | INTRONIC | ENSG00000196567 |
| rs3903768 | chr13 | 49294756 | 6.811e-005 | INTERGENIC | N/A |
| rs7325978 | chr13 | 71960761 | 8.206e-005 | INTERGENIC | N/A |
| rs13585 | chr22 | 45131843 | 0.0001016 | REGULATORY_REGION,3PRIME_UTR | TRMU |
| rs9368373 | chr6 | 22024631 | 0.0001063 | INTERGENIC | N/A |
| rs10935354 | chr3 | 141026326 | 0.0001073 | INTERGENIC | N/A |
| rs8095703 | chr18 | 11041081 | 0.0001183 | INTERGENIC | N/A |
| rs10206851 | chr2 | 5381477 | 0.0001267 | INTERGENIC | N/A |
| rs9542977 | chr13 | 71958236 | 0.0001499 | INTERGENIC | N/A |
| rs4942879 | chr13 | 49314940 | 0.0001537 | INTERGENIC | N/A |
| rs9542954 | chr13 | 71918308 | 0.0001643 | INTERGENIC | N/A |
| rs1593478 | chr18 | 2292023 | 0.0001663 | INTERGENIC | N/A |
| rs9542951 | chr13 | 71913959 | 0.0001664 | INTERGENIC | N/A |
| rs2188534 | chr7 | 111837550 | 0.0001823 | INTERGENIC | N/A |
| rs12524124 | chr6 | 22051921 | 0.000185 | INTERGENIC | N/A |
| rs4352629 | chr5 | 87792577 | 0.0001985 | INTERGENIC | N/A |
| rs7448716 | chr5 | 87788451 | 0.0001985 | INTERGENIC | N/A |
| rs11873533 | chr18 | 3732713 | 0.0002107 | INTRONIC | DLGAP1 |
| rs10062658 | chr5 | 102881920 | 0.0002216 | INTERGENIC | N/A |
| rs12547917 | chr8 | 142306580 | 0.0002282 | INTRONIC | SLC45A4 |
| rs1038268 | chr9 | 31967623 | 0.000229 | INTERGENIC | N/A |
| rs2375811 | chr9 | 31979998 | 0.000229 | INTERGENIC | N/A |
| rs1352671 | chr9 | 32033985 | 0.000229 | INTERGENIC | N/A |
| rs364331 | chr9 | 32039631 | 0.000229 | INTERGENIC | N/A |
| rs1924949 | chr13 | 71975473 | 0.0002323 | INTERGENIC | N/A |
| rs11025990 | chr11 | 21218608 | 0.0002323 | INTRONIC | NELL1 |
| rs3758562 | chr10 | 72032086 | 0.0002369 | INTRONIC | PRF1 |
| rs10156056 | chr7 | 22720613 | 0.0002394 | INTERGENIC | N/A |

### The results of the prediction in the test set are summarized below:

For the prediction of the dichotomized ACTH response status in the dex/CRH the following prediction values were achieved:

### ACTH:

Accuracy = 85.33%
Sensitivity = 87.18%
Specificity = 83.33%
Geometric mean of sensitivity and specificity = 85.23%
Positive Predictive Value (PPV) = 85.00%
Negative Predictive Value (NPV) = 85.71%
Geometric mean of PPV and NPV = 85.36%
Precision = 85.00%
Recall = 87.18%
F-Measure = 0.8608
Area under ROC curve (AUC) = 0.851852

Using genome-wide SNP association data for the ACTH response in the dex/CRH test, a subset of 30 SNPs could be identified that allowed an accurate, sensitive and specific prediction of these phenotypes in independent sets of patients. Increased ACTH secretion in this test has been linked to an increase in central CRH/CRHR1 function. Although environmental, pharmacological and disease state-dependent factors have previously been described to influence the endocrine response to this test in depressed patients (Ising et al., Neuropsychopharmacol Biol Psychiatry, 2005; Heim et al. Biol Psychiatry, 2008; Kunzel et al. Neuropsychopharmacology, 2003), it has now been shown that genetic polymorphisms alone are strong predictors.

Patients with depression or anxiety disorders, classified into the high ACTH response group according to the genotypes of the presented 30 SNPs described in this example will be more likely to respond to CRHR1 antagonist treatment. This allows an enrichment of such patients for CRHR1 antagonist treatment studies who will most likely respond to this specific treatment.

### Example 2:

Sleep disturbances, such as decreased slow-wave sleep, increased sleep fragmentation and rapid-eye-movement sleep (REMS) disinhibition, are cardinal symptoms of major depression in humans. This study aims to identify those patients where a central CRH hyperdrive plays a causal role and which would therefore respond favourably to a CRHR1 antagonist. To test the relationship between a central CRH-overexpression and REM-disinhibition in particular, transgenic mouse models where CRH is overexpressed as a result of genetic engineering were employed.

Many animal models of depression share increases in REM-sleeps (REMS) as a common feature. Therefore, increased REMS in animals should reflect REMS-disinhibitions in humans. Mice with CNS-specific CRH-overexpression strikingly share the characteristic increases in REMS. As such, an increase in REMS indicates a central hypersecretion of CRH and may serve as a biomarker to identify those patients who would benefit from treatment with a CRHR1 antagonist.

Experiments were conducted with two different mouse lines of excessive central CRH secretion and their respective control littermates (CL). Mice of the CRH-COE^{CNS} line are characterised by CRH-overexpression within the whole CNS, whereas mice of the Cor26 CRH line display a CRH-overexpression specific to CRH-ergic neurons of the CNS. Three different CRHR1 antagonists were tested. While DMP-696 (bicyclic) and CP-316,311 (monocyclic) are class I CRH-R1 antagonists, SSR125543A (long off-rate, typical slow-tight binding inhibitor) belongs to class II CRH-R1 antagonists. DMP-696 and SSR125543A were applied to CRH-COE^{CNS} mice (n_{DMP696}= 6/6 COE/CL; n_{SSR125543}= 6/5 COE/CL), while CP-316,311 was tested in Cor26 CRH mice (n_{CP316.311}= 5/3 Cor26/CL). In all cases, animals were left to recover from EEG/EMG-electrode implantation for two weeks, after which two days of baseline recording were initiated. Treatment with CRH-R1 antagonist or respective vehicle control commenced thereafter for five consecutive days. Antagonists were applied through the drinking water at a daily dose of 50mg/kg body weight. EEG and EMG recordings were manually scored as wake, non-REMS (NREMS), and REMS in four second epochs by an experienced evaluator.

As previously shown, CRH-COE^{CNS} mice display significantly higher REMS activity under baseline condition as compared to controls. Chronic DMP-696 (50mg/kg/d DMP-696) treatment entails only a mild suppression of REMS in CL mice. However, DMP-696-treated CRH-COE^{CNS} mice show a significant decrease in REMS activity beginning with treatment day two (P<0.05). The strongest suppression of REMS activity in CRH-COE^{CNS} animals could be observed on treatment day three (Figure 2).

Comparable to DMP696 treatment, oral application of SSR125543A (50mg/kg/d) affected REMS levels in CRH-overexpressing mice. No effects of SSR125543 on REMS activity in control animals could be detected. In contrast, a significant suppression of REMS could be observed beginning with day two in CRH-overexpressing animals (P≤0.035). Similar to DMP696 treatment, REMS suppression in CRH-COE^{CNS} mice never exceeded baseline REMS-levels of CL (Figure 3).

Application of CP-316,311 (Pfizer) in the Cor26 CRH mouse line showed no significant effect on REMS levels in CL animals (data not shown). Similarly, in CRH-overexpressing Cor26 CRH mice suppression in REMS apparently seemed weak. However, comparison of the area under the curve (AUC) within the light period of baseline and treatment day three showed a significant decrease (P=0.006) of REMS levels after CP-316,311 application (Figure 4).

CRH is one of the major drivers of the stress response in the brain. Hyperactivity of the CRH system seems to be responsible for cognitive impairments, emotional responses, and behavioural changes which are typical for depression. One of those behavioural changes are sleep disturbances exemplified by REMS disinhibition. The link between CRH-overexpression and REMS level increases is evidenced by the mouse lines used in these experiments. Since CRH-overexpression in the Cor26 CRH mouse line is limited to CRH-ergic neurons, the net increase of CRH is lower when compared to the whole brain overexpression in CRH-COE^{CNS} mice. As a result, the phenotype of increased REMS levels was less profound in Cor26 CRH mice as compared to CRH-COE^{CNS} animals.

The main finding of this study is that the normalization of CRH-elicited sleep-EEG disturbances is striking when (1) different chemical classes of CRHR1 antagonists are used and (2) different animal models for CRH-induced sleep-EEG changes that are typical for human depression are employed. REMS disinhibition is indicative of a central CRH dysfuntion (i.e. hyperactivity) and as such may serve as a biomarker for the identification of depressed patients where depression is caused by central CRH-hyperdrive. Normalization of the sleep pattern by application of different CRHR1 antagonists could be shown in all of our experiments, employing different classes of CRHR1 receptors and different animal models overexpressing centrally CRH.

## Claims

1. Corticotropin releasing hormone receptor type 1 (CRHR1) antagonist for use in the treatment of depressive symptoms and/or anxiety symptoms in a patient, wherein the patient has corticotropin releasing hormone (CRH) overactivity and the treatment comprises detecting whether or not the patient has CRH overactivity by determining the status of one or more biomarkers indicative for CRH overactivity, wherein the biomarker or a set of biomarkers is obtained by a genome-wide screening for single nucleotide polymorphisms in patients with depressive and/or anxiety symptoms and/or the biomarker is REM density
wherein the CRHR1 antagonist is a compound of formula (I) 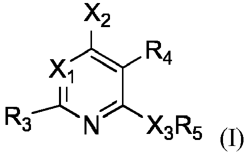 or a pharmaceutically acceptable salt thereof, wherein
X₁ is -CR₆;
X₂ is -NR₁R₂, -CR₁R₂R₉, -C(=CR₂R₁₀)R₁, -NHCHR₁R₂, -OCHR₁R₂, -SCHR₁R₂, -CHR₂OR₁₀, -CHR₂SR₁₀, -C(S)R₂ or -C(O)R₂;
X₃ is NH, O, S, -N(C₁-C₂ alkyl) or -C(R₁₁R₁₂), wherein R₁₁ and R₁₂ are each, independently, hydrogen, trifluoromethyl or methyl, or one of R₁₁ and R₁₂ is cyano and the other is hydrogen or methyl;
R₁ is C₁-C₆ alkyl which may optionally be substituted with one or two substituents R₇ independently selected from the group consisting of hydroxy, fluoro, chloro, bromo, iodo, CF₃, C₃-C₈ cycloalkyl, C₁-C₄ alkoxy, -O-CO-(C₁-C₄ alkyl), -O-CO-NH(C₁-C₄ alkyl), -O-CO-N(C₁-C₄ alkyl)(C₁-C₂ alkyl), -NH(C₁-C₄ alkyl), -N(C₁-C₂ alkyl)(C₁-C₄ alkyl), -S(C₁-C₄ alkyl), -N(C₁-C₄ alkyl)CO(C₁-C₄ alkyl),-NHCO(C₁-C₄ alkyl), -COO(C₁-C₄ alkyl), -CONH(C₁-C₄ alkyl), -CON(C₁-C₄ alkyl)(C₁-C₂ alkyl), CN, NO₂, -SO(C₁-C₄ alkyl) and -SO₂(C₁-C₄ alkyl), and wherein said C₁-C₆ alkyl and the (C₁-C₄) alkyl moieties in the foregoing R₇ groups may optionally contain one carbon-carbon double or triple bond;
R₂ is C₁-C₁₂ alkyl, C₁-C₁₂ alkoxyalkyl,
aryl or -(C₁-C₄ alkylene)aryl wherein said aryl is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiazolyl, benzisoxazolyl, benzimidazolyl, indolyl, oxadiazolyl or benzoxazolyl;
3- to 8-membered cycloalkyl or -(C₁-C₆ alkylene)cycloalkyl, wherein one or two of the ring carbons of said cycloalkyl having at least 4 ring members and the cycloalkyl moiety of said -(C₁-C₆ alkylene)cycloalkyl having at least 4 ring members may optionally be replaced by an oxygen or sulfur atom or by N-R₈ wherein R₈ is hydrogen or C₁-C₄ alkyl;
and wherein each of the foregoing R₂ groups may optionally be substituted with from one to three substituents independently selected from chloro, fluoro and C₁-C₄ alkyl, or with one substituent selected from bromo, iodo, C₁-C₆ alkoxy, -O-CO-(C₁-C₆ alkyl), -O-CO-N(C₁-C₄ alkyl)(C₁-C₂ alkyl), -S(C₁-C₆ alkyl), CN, NO₂, -SO(C₁-C₄ alkyl), and -SO₂(C₁-C₄ alkyl), and wherein said C₁-C₁₂ alkyl and/or the C₁-C₄ alkylene moiety of said -(C₁-C₄ alkylene)aryl may optionally contain one carbon-carbon double or triple bond;
or -NR₁R₂ or -CR₁R₂R₉ may form a saturated 5- to 8-membered ring which may optionally contain one or two carbon-carbon double bonds and/or in which one or two of the ring carbons may optionally be replaced by an oxygen, nitrogen or sulfur atom and which may be substituted with at least one substituent;
R₃ is methyl, ethyl, fluoro, chloro, bromo, iodo, cyano, methoxy, OCF₃, methylthio, methylsulfonyl, CH₂OH, or CH₂OCH₃;
R₄ is hydrogen, C₁-C₄ alkyl, fluoro, chloro, bromo, iodo, C₁-C₄ alkoxy, trifluoromethoxy,
-CH₂OCH₃, -CH₂OCH₂CH₃, -CH₂CH₂OCH₃, -CH₂OCF3, CF₃, amino, nitro, -NH(C₁-C₄ alkyl), -N(CH₃)₂, -NHCOCH₃ -NHCONHCH₃, -SOₙ(C₁-C₄ alkyl) wherein n is 0, 1 or 2, hydroxy, -CO(C₁-C₄ alkyl), -CHO, cyano or -COO(C₁-C₄ alkyl) wherein said C₁-C₄ alkyl may optionally contain one double or triple bond and/or may optionally be substituted with one substituent selected from hydroxy, amino, -NHCOCH₃, -NH(C₁-C₂ alkyl), -N(C₁-C₂ alkyl)₂, -COO(C₁-C₄ alkyl),-CO(C₁-C₄ alkyl), C₁-C₃ alkoxy, C₁-C₃ thioalkyl, fluoro, chloro, cyano and nitro;
R₅ is phenyl, naphthyl, thienyl, benzothienyl, pyridyl, quinolyl, pyrazinyl, pyrimidyl, furanyl, benzofuranyl, benzothiazolyl, or indolyl,
wherein each of the above groups R₅ is substituted with from one to three substituents independently selected from fluoro, chloro, C₁-C₆ alkyl and C₁-C₆ alkoxy, or with one substituent selected from hydroxy, iodo, bromo, formyl, cyano, nitro, trifluoromethyl, amino, (C₁-C₆ alkyl)O(C₁-C₆)alkyl, -NHCH₃,-N(CH₃)₂, -COOH, -COO(C₁-C₄ alkyl), -CO(C₁-C₄ alkyl), -SO₂NH(C₁-C₄ alkyl),-SO₂N(C₁-C₄ alkyl)(C₁-C₂ alkyl), -SO₂NH₂, -NHSO₂(C₁-C₄ alkyl), -S(C₁-C₆ alkyl) and SO₂(C₁-C₆ alkyl), and wherein the C₁-C₄ alkyl and the C₁-C₆ alkyl moieties of the foregoing R₅ groups may optionally be substituted with one or two fluoro groups or with one substituent selected from hydroxy, amino, methylamino, dimethylamino and acetyl;
R₆ is hydrogen, methyl, fluoro, chloro, bromo, iodo, cyano, hydroxy, -O(C₁-C₄ alkyl), -C(O)(C₁-C₄ alkyl), -C(O)O(C₁-C₄ alkyl), -OCF₃, CF₃, -CH₂OH,-CH₂OCH₃ or -CH₂OCH₂CH₃;
R₉ is hydrogen, hydroxy, fluoro, or methoxy;
R₁₀ is hydrogen or C₁-C₄ alkyl.

2. CRHR1 antagonist for use according to claim 1, wherein X₁ is CH.

3. CRHR1 antagonist for use according to claim 1 or 2, wherein the 5- to 8-membered ring formed by -NR₁R₂ or -CR₁R₂R₉ is substituted with at least one substituent selected from C₁-C₄ alkyl or
with a 4-8 membered ring, which may be saturated or may contain one to three double bonds and in which one carbon atom may be replaced by CO or SO₂ and one to four carbon atoms may optionally be replaced by nitrogen.

4. CRHR1 antagonist for use according to any of claims 1 to 3, wherein
X₂ is -NHCHR₁R₂, -OCHR₁R₂ or -NR₁R₂.

5. CRHR1 antagonist for use according to claim 4, wherein
-NHCHR₁R₂ is -NHCH(CH₂OCH₃)₂, -NHCH(CH₂OCH₃)(CH₂CH₃),-NHCH(CH₂CH₃)₂, -NHCH(CH₂CH₂OCH₃)₂, -NHCH(CH₃)(CH₂CH₃) or-NHCHR₁R₂, wherein R₁ is ethyl and R₂ is oxadiazolyl substituted with methyl, isopropyl, cyclopropyl, trifluoromethyl, or ethyl,
or
-NR₁R₂ is -N(CH₂CH₃)(CH₃), -N(CH₂CH₂CH₃)₂, -N(CH₂CH₂CH₃)(CH₂-cyclopropyl), -N(CH₂CH₃)(CH₂CH₂CH₂CH₃), -N(CH₂CH₂OCH₃)₂, or-N(CH₂CH₂OCH₃)(CH₂CH₂CH₃)
or
-OCHR₁R₂ is -OCH(CH₂CH₃)₂, -OCH(CH₂CH₃)CH₃, - OCH(CH₂CH₃)(CH₂CH₂CH₃), -OCH(CH₂CH₃)(CH₂OCH₃).

6. CRHR1 antagonist for use according to any of claims 1 to 5, wherein
R₃ and R₄ are methyl.

7. CRHR1 antagonist for use according to any of claims 1 to 6, wherein
X₃ is O.

8. CRHR1 antagonist for use according to any of claims 1 to 7, wherein
R₅ is phenyl substituted with from one to three substituent(s) independently selected from the group CH₃, CH₂CH₃, OCH₃, Cl, F, CF₃.

9. CRHR1 antagonist for use according to any of claims 1 to 8, wherein the CRHR1 antagonist is a compound of the formula (VI) 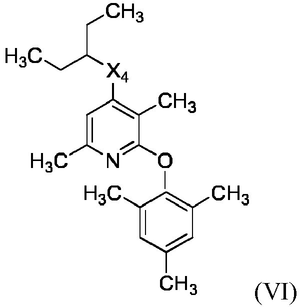 or a pharmaceutically acceptable salt thereof, wherein
X₄ is O or NH.

10. CRHR1 antagonist for use according to claim 1, wherein the CRHR1 antagonist is selected from the group consisting of CP-316,311 and CP-376,395

11. CRHR1 antagonist for use according to claims 1 or 10, wherein the biomarker or the set of biomarkers is selected from the group comprising:
• SNP rs6437726,
• SNP rs1986684,
• SNP rs7380830,
• SNP rs3903768,
• SNP rs7325978,
• SNP rs13585,
• SNP rs9368373,
• SNP rs10935354,
• SNP rs8095703,
• SNP rs10206851,
• SNP rs9542977,
• SNP rs4942879,
• SNP rs9542954,
• SNP rs1593478,
• SNP rs9542951,
• SNP rs2188534,
• SNP rs12524124,
• SNP rs4352629,
• SNP rs7448716,
• SNP rs11873533,
• SNP rs10062658,
• SNP rs12547917,
• SNP rs1038268,
• SNP rs2375811,
• SNP rs1352671,
• SNP rs364331,
• SNP rs1924949,
• SNP rs11025990,
• SNP rs3758562, and/or
• SNP rs10156056.

12. CRHR1 antagonist for use according to any of claims 1 to 11, wherein the biomarker or the set of biomarkers constituting a marker for CRH activity is selected from one or more biomarkers from the group comprising:
• SNP rs6437726 which is represented by a single polymorphic change at position 201 of SEQ ID NO: 1, wherein in one or two alleles the wild-type nucleotide A is replaced by indicator nucleotide G,
• SNP rs1986684 which is represented by a single polymorphic change at position 201 of SEQ ID NO: 2, wherein in one or two alleles the wild-type nucleotide A is replaced by indicator nucleotide G,
• SNP rs7380830 which is represented by a single polymorphic change at position 201 of SEQ ID NO: 3, wherein in one or two alleles the wild-type nucleotide C is replaced by indicator nucleotide T,
• SNP rs3903768 which is represented by a single polymorphic change at position 201 of SEQ ID NO: 4, wherein in one or two alleles the wild-type nucleotide A is replaced by indicator nucleotide G,
• SNP rs7325978 which is represented by a single polymorphic change at position 201 of SEQ ID NO: 5, wherein in one or two alleles the wild-type nucleotide C is replaced by indicator nucleotide T,
• SNP rs13585 which is represented by a single polymorphic change at position 185 of SEQ ID NO: 6, wherein in one or two alleles the wild-type nucleotide C is replaced by indicator nucleotide T,
• SNP rs9368373 which is represented by a single polymorphic change at position 201 of SEQ ID NO: 7, wherein in one or two alleles the wild-type nucleotide C is replaced by indicator nucleotide T,
• SNP rs10935354 which is represented by a single polymorphic change at position 201 of SEQ ID NO: 8, wherein in one or two alleles the wild-type nucleotide A is replaced by indicator nucleotide G,
• SNP rs8095703 which is represented by a single polymorphic change at position 201 of SEQ ID NO: 9, wherein in one or two alleles the wild-type nucleotide A is replaced by indicator nucleotide G,
• SNP rs10206851 which is represented by a single polymorphic change at position 201 of SEQ ID NO: 10, wherein in one or two alleles the wild-type nucleotide C is replaced by indicator nucleotide T,
• SNP rs9542977 which is represented by a single polymorphic change at position 201 of SEQ ID NO: 11, wherein in one or two alleles the wild-type nucleotide C is replaced by indicator nucleotide T,
• SNP rs4942879 which is represented by a single polymorphic change at position 201 of SEQ ID NO: 12, wherein in one or two alleles the wild-type nucleotide A is replaced by indicator nucleotide G,
• SNP rs9542954 which is represented by a single polymorphic change at position 201 of SEQ ID NO: 13, wherein in one or two alleles the wild-type nucleotide A is replaced by indicator nucleotide C,
• SNP rs1593478 which is represented by a single polymorphic change at position 201 of SEQ ID NO: 14, wherein in one or two alleles the wild-type nucleotide C is replaced by indicator nucleotide T,
• SNP rs9542951 which is represented by a single polymorphic change at position 201 of SEQ ID NO: 15, wherein in one or two alleles the wild-type nucleotide A is replaced by indicator nucleotide G,
• SNP rs2188534 which is represented by a single polymorphic change at position 200 of SEQ ID NO: 16, wherein in one or two alleles the wild-type nucleotide G is replaced by indicator nucleotide T,
• SNP rs12524124 which is represented by a single polymorphic change at position 201 of SEQ ID NO: 17, wherein in one or two alleles the wild-type nucleotide A is replaced by indicator nucleotide G,
• SNP rs4352629 which is represented by a single polymorphic change at position 201 of SEQ ID NO: 18, wherein in one or two alleles the wild-type nucleotide C is replaced by indicator nucleotide T,
• SNP rs7448716 which is represented by a single polymorphic change at position 201 of SEQ ID NO: 19, wherein in one or two alleles the wild-type nucleotide A is replaced by indicator nucleotide G,
• SNP rs11873533 which is represented by a single polymorphic change at position 201 of SEQ ID NO: 20, wherein in one or two alleles the wild-type nucleotide A is replaced by indicator nucleotide C,
• SNP rs10062658 which is represented by a single polymorphic change at position 201 of SEQ ID NO: 21, wherein in one or two alleles the wild-type nucleotide A is replaced by indicator nucleotide G,
• SNP rs12547917 which is represented by a single polymorphic change at position 201 of SEQ ID NO: 22, wherein in one or two alleles the wild-type nucleotide C is replaced by indicator nucleotide T,
• SNP rs1038268 which is represented by a single polymorphic change at position 201 of SEQ ID NO: 23, wherein in one or two alleles the wild-type nucleotide C is replaced by indicator nucleotide T,
• SNP rs2375811 which is represented by a single polymorphic change at position 201 of SEQ ID NO: 24, wherein in one or two alleles the wild-type nucleotide A is replaced by indicator nucleotide G,
• SNP rs1352671 which is represented by a single polymorphic change at position 201 of SEQ ID NO: 25, wherein in one or two alleles the wild-type nucleotide A is replaced by indicator nucleotide C,
• SNP rs364331 which is represented by a single polymorphic change at position 201 of SEQ ID NO: 26, wherein in one or two alleles the wild-type nucleotide A is replaced by indicator nucleotide C,
• SNP rs1924949 which is represented by a single polymorphic change at position 201 of SEQ ID NO: 27, wherein in one or two alleles the wild-type nucleotide A is replaced by indicator nucleotide G,
• SNP rs11025990 which is represented by a single polymorphic change at position 201 of SEQ ID NO: 28, wherein in one or two alleles the wild-type nucleotide A is replaced by indicator nucleotide G,
• SNP rs3758562 which is represented by a single polymorphic change at position 201 of SEQ ID NO: 29, wherein in one or two alleles the wild-type nucleotide A is replaced by indicator nucleotide G, and/or
• SNP rs10156056 which is represented by a single polymorphic change at position 201 of SEQ ID NO: 30, wherein in one or two alleles the wild-type nucleotide C is replaced by indicator nucleotide G.

13. CRHR1 antagonist for use according to claim 11 or 12, wherein the set of biomarkers comprises at least 15, at least 20 or at least 25 of the biomarkers as defined in claims 11 or 12.

14. CRHR1 antagonist for use according to any of claims 11 to 13, wherein the set of biomarkers consists of the biomarkers as defined in claims 11 or 12.

15. CRHR1 antagonist for use according to any of claims 11 to 14, wherein the marker is a combination of a biomarker or set of biomarkers as defined in any of claims 11 to 14 with the biomarker REM density.

## Patentansprüche

1. Corticotropin-Releasing-Hormon-Rezeptor-Typ-1 (CRHR1)-Antagonist zur Verwendung bei der Behandlung von depressiven Symptomen und/oder Angstsymptomen bei einem Patienten, wobei der Patient Corticotropin-Releasing-Hormon (CRH)-Überaktivität aufweist und die Behandlung das Feststellen, ob der Patient CRH hat oder nicht, durch Bestimmen des Status eines oder mehrerer Biomarker, die CRH-Überaktivität anzeigen, umfasst, wobei der Biomarker oder ein Satz an Biomarkern durch ein genomweites Screening auf Einzelnukleotid-Polymorphismen bei Patienten mit depressiven und/oder Angstsymptomen erhalten worden ist und/oder der Biomarker REM-Dichte ist,
wobei der CRHR1-Antagonist eine Verbindung der Formel (I) ist 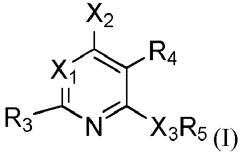 oder ein pharmazeutisch verträgliches Salz davon ist, wobei
X₁ -CR₆ ist;
X₂ -NR₁R₂, -CR₁R₂R₉, -C(=CR₂R₁₀)R₁, -NHCHR₁R₂, -OCHR₁R₂, -SCHR₁R₂, -CHR₂OR₁₀, -CHR₂SR₁₀, -C(S)R₂ oder -C(O)R₂ ist;
X₃ NH, O, S, -N(C₁-C₂-Alkyl) oder -C(R₁₁R₁₂) ist, wobei R₁₁ und R₁₂ jeweils unabhängig voneinander Wasserstoff, Triflourmethyl oder Methyl sind, oder einer von R₁₁ und R₁₂ Cyan ist und der andere Wasserstoff oder Methyl ist;
R₁ C₁-C₆-Alkyl ist, das optional mit einem oder zwei Substituenten R₇ unabhängig voneinander ausgewählt aus der Gruppe bestehend aus Hydroxy-, Fluor-, Chlor-, Brom-, Iod-, CF₃-, C₃-C₈-Cycloalkyl, C₁-C₄-Alkoxy, -O-CO-(C₁-C₄-Alkyl), -O-CO-NH(C₁-C₄-Alkyl), -O-CO-N(C₁-C₄-Alkyl)(C₁-C₂-Alkyl), -NH(C₁-C₄-Alkyl), -N(C₁-C₂-Alkyl)(C₁-C₄-Alkyl), -S(C₁-C₄-Alkyl), -N(C₁-C₄-Alkyl)CO(C₁-C₄-Alkyl), -NHCO(C₁-C₄-Alkyl), -COO(C₁-C₄-Alkyl), -CONH(C₁-C₄-Alkyl), -CON(C₁-C₄-Alkyl)(C₁-C₂-Alkyl), CN, NO₂, -SO(C₁-C₄-Alkyl) und -SO₂(C₁-C₄-Alkyl) substituiert sein kann, und wobei die C₁-C₆-Alkyl und die (C₁-C₄)-Alkylreste in den vorstehenden R₇-Gruppen optional eine Kohlenstoff-Kohlenstoff-Doppel- oder Dreifachbindung enthalten können;
R₂ C₁-C₁₂-Alkyl, C₁-C₁₂-Alkoxyalkyl,
Aryl oder -(C₁-C₄-Alkylen-)Aryl, wobei das Aryl, Phenyl, Naphthyl, Thienyl, Benzothienyl, Pyridyl, Chinolyl, Pyrazinyl, Pyrimidyl, Imidazolyl, Furanyl, Benzofuranyl, Benzothiazolyl, Isothiazolyl, Benzisothiazolyl, Benzisoxazolyl, Benzimidazolyl, Indolyl, Oxadiazolyl oder Benzoxazolyl ist;
3- bis 8-gliedriges Cycloalkyl oder -(C₁-C₆-Alkylen)cycloalkyl ist, wobei ein oder zwei der Ringkohlenstoffe des Cycloalkyls, die mindestens 4 Ringglieder aufweisen, und der Cycloalkylrest des -(C₁-C₆-Alkylen)cycloalkyls, der mindestens 4 Ringglieder aufweist, optional durch ein Sauerstoff- oder Schwefelatom oder durch N-Rs ersetzt werden kann, wobei R₈ Wasserstoff oder C₁-C₄-Alkyl ist;
und wobei jede der vorstehenden R₂-Gruppen optional mit ein bis drei Substituenten, die unabhängig voneinander ausgewählt sind aus Chlor, Fluor und C₁-C₄-Alkyl, oder mit einem Substituenten ausgewählt aus Brom, Iod, C₁-C₆-Alkoxy, -O-CO-(C₁-C₆-Alkyl), -O-CO-N(C₁-C₄-Alkyl)(C₁-C₂-Alkyl), -S(C₁-C₆-Alkyl), CN, NO₂, -SO(C₁-C₄-Alkyl) und -SO₂(C₁-C₄-Alkyl) substituiert sein können, und
wobei das C₁-C₁₂-Alkyl und/oder der C₁-C₄-Alkylenrest des -(C₁-C₄-Alkylen-)-Aryls optional eine Kohlenstoff-Kohlenstoff-Doppel- oder Dreifachbindung enthalten kann;
oder -NR₁R₂ oder -CR₁R₂R₉ einen gesättigten 5- bis 8-gliedrigen Ring bilden kann, der optional eine oder zwei Kohlenstoff-Kohlenstoff-Doppelbindungen enthalten kann und/oder bei dem ein oder zwei der Ringkohlenstoffe optional durch ein Sauerstoff-, Stickstoff-, oder Schwefelatom ersetzt sein können und der mit mindestens einem Substituenten substituiert sein kann;
R₃ Methyl, Ethyl, Fluor, Chlor, Brom, Iod, Cyano, Methoxy, OCF₃, Methylthio, Methylsulfonyl, CH₂OH, oder CH₂OCH₃ ist;
R₄ Wasserstoff, C₁-C₄-Alkyl, Fluor, Chlor, Brom, Iod, C₁-C₄-Alkoxy, Trifluormethoxy,
-CH₂OCH₃, -CH₂OCH₂CH₃, -CH₂CH₂OCH₃, -CH₂OCF3, CF₃, Amino, Nitro, -NH(C₁-C₄-Alkyl), -N(CH₃)₂, -NHCOCH₃ -NHCONHCH₃, -SOₙ(C₁-C₄-Alkyl), wobei n 0, 1 oder 2 ist, Hydroxy, -CO(C₁-C₄-Alkyl), -CHO, Cyano oder - COO(C₁-C₄-Alkyl) ist, wobei das C₁-C₄-Alkyl optional eine Doppel- oder Dreifachbindung enthalten kann und/oder mit einem Substituenten, ausgewählt aus Hydroxy, Amino, -NHCOCH₃, -NH(C₁-C₂-Alkyl), -N(C₁-C₂-Alkyl)₂, - COO(C₁-C₄-Alkyl), -CO(C₁-C₄-Alkyl), C₁-C₃-Alkoxy, C₁-C₃-Thioalkyl, Fluor, Chlor, Cyano und Nitro, substituiert sein kann;
R₅ Phenyl, Naphthyl, Thienyl, Benzothienyl, Pyridyl, Chinolyl, Pyrazinyl, Pyrimidyl, Furanyl, Benzofuranyl, Benzothiazolyl, oder Indolyl ist,
wobei jede der obigen R₅-Gruppen mit ein bis drei Substituenten, die unabhängig ausgewählt sind aus Fluor, Chlor, C₁-C₆-Alkyl und C₁-C₆-Alkoxy oder mit einem Substituenten, ausgewählt aus Hydroxy, Iod, Brom, Formyl, Cyano, Nitro, Trifluormethyl, Amino, (C₁-C₆-Alkyl)O(C₁-C₆)alkyl, -NHCH₃, -N(CH₃)₂, -COOH, -COO(C₁-C₄-Alkyl), -CO(C₁-C₄-Alkyl), -SO₂NH(C₁-C₄-Alkyl), - SO₂N(C₁-C₄-Alkyl)(C₁-C₂-Alkyl), -SO₂NH₂, -NHSO₂(C₁-C₄-Alkyl), -S(C₁-C₆-Alkyl) und SO₂(C₁-C₆-Alkyl) substituiert ist, und wobei das C₁-C₄-Alkyl und die C₁-C₆-Alkylreste der vorstehenden R₅-Gruppen optional mit einer oder zwei Fluorgruppen oder mit einem Substituenten ausgewählt aus Hydroxy, Amino, Methylamino, Dimethylamino und Acetyl substituiert sein können;
R₆ Wasserstoff, Methyl, Fluor, Chlor, Brom, Iod, Cyano, Hydroxy, -O(C₁-C₄-Alkyl), -C(O)(C₁-C₄-Alkyl), -C(O)O(C₁-C₄ -Alkyl), -OCF₃, CF₃, -CH₂OH, -CH₂OCH₃ oder -CH₂OCH₂CH₃ ist;
R₉ Wasserstoff, Hydroxy, Fluor, oder Methoxy ist;
R₁₀ Wasserstoff oder C₁-C₄-Alkyl ist.

2. CRHR1-Antagonist zur Verwendung nach Anspruch 1, wobei X₁ CH ist.

3. CRHR1-Antagonist zur Verwendung nach Anspruch 1 oder 2, wobei der 5-bis 8-gliedrige Ring, gebildet durch -NR₁R₂ oder -CR₁R₂R₉, substituiert ist mit mindestens einem Substituenten ausgewählt aus C₁-C₄-Alkyl oder mit einem 4-8-gliedrigen Ring, der gesättigt sein kann oder ein bis drei Doppelbindungen enthalten kann und bei dem ein Kohlenstoffatom durch CO oder SO₂ ersetzt sein kann und ein bis vier Kohlenstoffatome optional durch Stickstoff ersetzt sein können.

4. CRHR1-Antagonist zur Verwendung nach einem der Ansprüche 1 bis 3, wobei X₂ -NHCHR₁R₂, -OCHR₁R₂ oder -NR₁R₂ ist.

5. CRHR1-Antagonist zur Verwendung nach Anspruch 4, wobei
-NHCHR₁R₂ NHCH(CH₂OCH₃)₂, -NHCH(CH₂OCH₃)(CH₂CH₃), -NHCH(CH₂CH₃)₂, -NHCH(CH₂CH₂OCH₃)₂, -NHCH(CH₃)(CH₂CH₃) oder -NHCHR₁R₂ ist, wobei R₁ Ethyl und R₂ Oxadiazolyl substituiert mit Methyl, Isopropyl, Cyclopropyl, Trifluormethyl, oder Ethyl ist,
oder
-NR₁R₂ -N(CH₂CH₃)(CH₃), -N(CH₂CH₂CH₃)₂, -N(CH₂CH₂CH₃)(CH₂-Cyclopropyl), -N(CH₂CH₃)(CH₂CH₂CH₂CH₃), -N(CH₂CH₂OCH₃)₂, oder -N(CH₂CH₂OCH₃)(CH₂CH₂CH₃) ist
oder
-OCHR₁R₂ -OCH(CH₂CH₃)₂, -OCH(CH₂CH₃)CH₃, -OCH(CH₂CH₃)(CH₂CH₂CH₃), -OCH(CH₂CH₃)(CH₂OCH₃) ist.

6. CRHR1-Antagonist zur Verwendung nach einem der Ansprüche 1 bis 5, wobei R₃ und R₄ Methyl sind.

7. CRHR1-Antagonist zur Verwendung nach einem der Ansprüche 1 bis 6, wobei X₃ O ist.

8. CRHR1-Antagonist zur Verwendung nach einem der Ansprüche 1 bis 7, wobei R₅ Phenyl substituiert mit einem bis drei Substituenten unabhängig voneinander ausgewählt aus der Gruppe CH₃, CH₂CH₃, OCH₃, Cl, F, CF₃ ist.

9. CRHR1-Antagonist zur Verwendung nach einem der Ansprüche 1 bis 8, wobei der CRHR1-Antagonist eine Verbindung der Formel (VI) ist 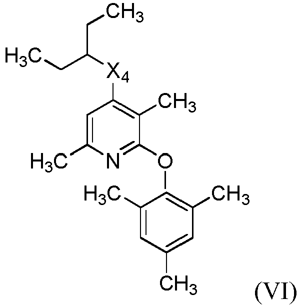 oder ein pharmazeutisch verträgliches Salz davon ist, wobei
X₄ O oder NH ist;

10. CRHR1-Antagonist zur Verwendung nach Anspruch 1, wobei der CRHR1-Antagonist ausgewählt ist aus der Gruppe bestehend aus CP-316,311 und CP-376,395.

11. CRHR1-Antagonist zur Verwendung nach Ansprüchen 1 oder 10, wobei der Biomarker oder der Satz an Biomarkern ausgewählt ist aus der Gruppe umfassend:
• SNP rs6437726,
• SNP rs1986684,
• SNP rs7380830,
• SNP rs3903768,
• SNP rs7325978,
• SNP rs13585,
• SNP rs9368373,
• SNP rs10935354,
• SNP rs8095703,
• SNP rs10206851,
• SNP rs9542977,
• SNP rs4942879,
• SNP rs9542954,
• SNP rs1593478,
• SNP rs9542951,
• SNP rs2188534,
• SNP rs12524124,
• SNP rs4352629,
• SNP rs7448716,
• SNP rs11873533,
• SNP rs10062658,
• SNP rs12547917,
• SNP rs1038268,
• SNP rs2375811,
• SNP rs1352671,
• SNP rs364331,
• SNP rs1924949,
• SNP rs11025990,
• SNP rs3758562, und/oder
• SNP rs10156056.

12. CRHR1-Antagonist zur Verwendung nach einem der Ansprüche 1 bis 11, wobei der Biomarker oder der Satz an Biomarkern, die einen Marker für CRH-Aktivität bilden, ausgewählt ist aus einem oder mehreren Biomarkern aus der Gruppe umfassend:
• SNP rs6437726, das durch eine einzige polymorphe Veränderung an Position 201 von SEQ ID NO: 1 dargestellt ist, wobei in einem oder zwei Allelen das Wildtyp-Nukleotid A durch das Indikator-Nukleotid G ersetzt ist,
• SNP rs1986684, das durch eine einzige polymorphe Veränderung an Position 201 von SEQ ID NO: 2 dargestellt ist, wobei in einem oder zwei Allelen das Wildtyp-Nukleotid A durch das Indikator-Nukleotid G ersetzt ist,
• SNP rs7380830, das durch eine einzige polymorphe Veränderung an Position 201 von SEQ ID NO: 3 dargestellt ist, wobei in einem oder zwei Allelen das Wildtyp-Nukleotid C durch das Indikator-Nukleotid T ersetzt ist,
• SNP rs3903768, das durch eine einzige polymorphe Veränderung an Position 201 von SEQ ID NO: 4 dargestellt ist, wobei in einem oder zwei Allelen das Wildtyp-Nukleotid A durch das Indikator-Nukleotid G ersetzt ist,
• SNP rs7325978, das durch eine einzige polymorphe Veränderung an Position 201 von SEQ ID NO: 5 dargestellt ist, wobei in einem oder zwei Allelen das Wildtyp-Nukleotid C durch das Indikator-Nukleotid T ersetzt ist,
• SNP rs13585, das durch eine einzige polymorphe Veränderung an Position 185 von SEQ ID NO: 6, dargestellt ist, wobei in einem oder zwei Allelen das Wildtyp-Nukleotid C durch das Indikator-Nukleotid T ersetzt ist,
• SNP rs9368373, das durch eine einzige polymorphe Veränderung an Position 201 von SEQ ID NO: 7 dargestellt ist, wobei in einem oder zwei Allelen das Wildtyp-Nukleotid C durch das Indikator-Nukleotid T ersetzt ist,
• SNP rs10935354, das durch eine einzige polymorphe Veränderung an Position 201 von SEQ ID NO: 8 dargestellt ist, wobei in einem oder zwei Allelen das Wildtyp-Nukleotid A durch das Indikator-Nukleotid G ersetzt ist,
• SNP rs8095703, das durch eine einzige polymorphe Veränderung an Position 201 von SEQ ID NO: 9 dargestellt ist, wobei in einem oder zwei Allelen das Wildtyp-Nukleotid A durch das Indikator-Nukleotid G ersetzt ist,
• SNP rs10206851, das durch eine einzige polymorphe Veränderung an Position 201 von SEQ ID NO: 10 dargestellt ist, wobei in einem oder zwei Allelen das Wildtyp-Nukleotid C durch das Indikator-Nukleotid T ersetzt ist,
• SNP rs9542977, das durch eine einzige polymorphe Veränderung an Position 201 von SEQ ID NO: 11 dargestellt ist, wobei in einem oder zwei Allelen das Wildtyp-Nukleotid C durch das Indikator-Nukleotid T ersetzt ist,
• SNP rs4942879, das durch eine einzige polymorphe Veränderung an Position 201 von SEQ ID NO: 12 dargestellt ist, wobei in einem oder zwei Allelen das Wildtyp-Nukleotid A durch das Indikator-Nukleotid G ersetzt ist,
• SNP rs9542954, das durch eine einzige polymorphe Veränderung an Position 201 von SEQ ID NO: 13 dargestellt ist, wobei in einem oder zwei Allelen das Wildtyp-Nukleotid A durch das Indikator-Nukleotid G ersetzt ist,
• SNP rs1593478, das durch eine einzige polymorphe Veränderung an Position 201 von SEQ ID NO: 14 dargestellt ist, wobei in einem oder zwei Allelen das Wildtyp-Nukleotid C durch das Indikator-Nukleotid T ersetzt ist,
• SNP rs9542951, das durch eine einzige polymorphe Veränderung an Position 201 von SEQ ID NO: 15 dargestellt ist, wobei in einem oder zwei Allelen das Wildtyp-Nukleotid A durch das Indikator-Nukleotid G ersetzt ist,
• SNP rs2188534, das durch eine einzige polymorphe Veränderung an Position 200 von SEQ ID NO: 16 dargestellt ist, wobei in einem oder zwei Allelen das Wildtyp-Nukleotid G durch das Indikator-Nukleotid T ersetzt ist,
• SNP rs12524124, das durch eine einzige polymorphe Veränderung an Position 201 von SEQ ID NO: 17 dargestellt ist, wobei in einem oder zwei Allelen das Wildtyp-Nukleotid A durch das Indikator-Nukleotid G ersetzt ist,
• SNP rs4352629, das durch eine einzige polymorphe Veränderung an Position 201 von SEQ ID NO: 18 dargestellt ist, wobei in einem oder zwei Allelen das Wildtyp-Nukleotid C durch das Indikator-Nukleotid T ersetzt ist,
• SNP rs7448716, das durch eine einzige polymorphe Veränderung an Position 201 von SEQ ID NO: 19 dargestellt ist, wobei in einem oder zwei Allelen das Wildtyp-Nukleotid A durch das Indikator-Nukleotid G ersetzt ist,
• SNP rs11873533, das durch eine einzige polymorphe Veränderung an Position 201 von SEQ ID NO: 20 dargestellt ist, wobei in einem oder zwei Allelen das Wildtyp-Nukleotid A durch das Indikator-Nukleotid C ersetzt ist,
• SNP rs10062658, das durch eine einzige polymorphe Veränderung an Position 201 von SEQ ID NO: 21 dargestellt ist, wobei in einem oder zwei Allelen das Wildtyp-Nukleotid A durch das Indikator-Nukleotid G ersetzt ist,
• SNP rs12547917, das durch eine einzige polymorphe Veränderung an Position 201 von SEQ ID NO: 22 dargestellt ist, wobei in einem oder zwei Allelen das Wildtyp-Nukleotid C durch das Indikator-Nukleotid T ersetzt ist,
• SNP rs1038268, das durch eine einzige polymorphe Veränderung an Position 201 von SEQ ID NO: 23 dargestellt ist, wobei in einem oder zwei Allelen das Wildtyp-Nukleotid C durch das Indikator-Nukleotid T ersetzt ist,
• SNP rs2375811, das durch eine einzige polymorphe Veränderung an Position 201 von SEQ ID NO: 24 dargestellt ist, wobei in einem oder zwei Allelen das Wildtyp-Nukleotid A durch das Indikator-Nukleotid G ersetzt ist,
• SNP rs1352671, das durch eine einzige polymorphe Veränderung an Position 201 von SEQ ID NO: 25 dargestellt ist, wobei in einem oder zwei Allelen das Wildtyp-Nukleotid A durch das Indikator-Nukleotid C ersetzt ist,
• SNP rs364331, das durch eine einzige polymorphe Veränderung an Position 201 von SEQ ID NO: 26 dargestellt ist, wobei in einem oder zwei Allelen das Wildtyp-Nukleotid A durch das Indikator-Nukleotid C ersetzt ist,
• SNP rs1924949, das durch eine einzige polymorphe Veränderung an Position 201 von SEQ ID NO: 27 dargestellt ist, wobei in einem oder zwei Allelen das Wildtyp-Nukleotid A durch das Indikator-Nukleotid G ersetzt ist,
• SNP rs11025990, das durch eine einzige polymorphe Veränderung an Position 201 von SEQ ID NO: 28 dargestellt ist, wobei in einem oder zwei Allelen das Wildtyp-Nukleotid A durch das Indikator-Nukleotid G ersetzt ist,
• SNP rs3758562, das durch eine einzige polymorphe Veränderung an Position 201 von SEQ ID NO: 29 dargestellt ist, wobei in einem oder zwei Allelen das Wildtyp-Nukleotid A durch das Indikator-Nukleotid G ersetzt ist, und/oder
• SNP rs10156056, das durch eine einzige polymorphe Veränderung an Position 201 von SEQ ID NO: 30 dargestellt ist, wobei in einem oder zwei Allelen das Wildtyp-Nukleotid C durch das Indikator-Nukleotid G ersetzt ist.

13. CRHR1-Antagonist zur Verwendung nach Anspruch 11 oder 12, wobei der Satz an Biomarkern mindestens 15, mindestens 20, oder mindestens 25 der Biomarker, wie definiert in Anspruch 11 oder 12, umfasst.

14. CRHR1-Antagonist zur Verwendung nach einem der Ansprüche 11 bis 13, wobei der Satz an Biomarkern aus den in Ansprüchen 11 und 12 definierten Biomarkern besteht.

15. CRHR1-Antagonist zur Verwendung nach einem der Ansprüche 11 bis 14, wobei der Marker eine Kombination von einem Biomarker oder einem Satz an Biomarkern, wie definiert in einem der Ansprüche 11 bis 14, mit dem Biomarker REM-Dichte ist.

## Revendications

1. Antagoniste du récepteur de l'hormone de libération de la corticotropine de type 1 (CRHR1) destiné à être utilisé dans le traitement de symptômes dépressifs et/ou de symptômes d'angoisse chez un patient, sachant que le patient présente une hyperactivité de l'hormone de libération de la corticotropine (CRH) et le traitement comprend le fait de détecter si le patient présente une hyperactivité de la CRH ou non en déterminant l'état d'un ou de plusieurs biomarqueurs indicateurs d'hyperactivité de la CRH, sachant que le biomarqueur ou un ensemble de biomarqueurs est obtenu par dépistage sur tout le génome de polymorphismes nucléotidiques simples chez des patients présentant des symptômes dépressifs et/ou des symptômes d'angoisse et/ou le biomarqueur est la densité REM,
sachant que l'antagoniste CRHR1 est un composé de formule (I) 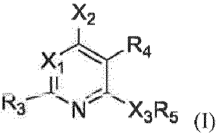 ou un sel pharmaceutiquement acceptable de celui-ci, sachant que
X₁ est -CR₆ ;
X₂ est -NR₁R₂, -CR₁R₂R₉, -C(=CR₂R₁₀)R₁, -NHCHR₁R₂, -OCHR₁R₂, - SCHR₁R₂, -CHR₂OR₁₀, -CHR₂SR₁₀, -C(S)R₂ ou -C(O)R₂ ;
X₃ est NH, O, S, -N(alkyle en C₁-C₂) ou -C(R₁₁R₁₂), sachant que R₁₁ et R₁₂ sont chacun, indépendamment, de l'hydrogène, du trifluorométhyle ou du méthyle, ou l'un de R₁₁ et de R₁₂ est du cyano et l'autre est de l'hydrogène ou du méthyle ;
R₁ est de l'alkyle en C₁-C₆ qui peut facultativement être substitué par un ou deux substituants R₇ indépendamment sélectionnés dans le groupe constitué par de l'hydroxy, du fluoro, du chloro, du bromo, de l'iodo, CF₃, du cycloalkyle en C₃-C₈, de l'alkoxy en C₁-C₄, -O-CO-(alkyle en C₁-C₄), -O-CO-NH(alkyle en C₁-C₄), -O-CO-N(alkyle en C₁-C₄)(alkyle en C₁-C₂), -NH(alkyle en C₁-C₄), -N(alkyle en C₁-C₂)(alkyle en C₁-C₄), -S(alkyle en C₁-C₄), -N(alkyle en C₁-C₄)CO(alkyle en C₁-C₄), - NHCO(alkyle en C₁-C₄), -COO(alkyle en C₁-C₄), -CONH(alkyle en C₁-C₄), - CON(alkyle en C₁-C₄)(alkyle en C₁-C₂), CN, NO₂, -SO(alkyle en C₁-C₄) et - SO₂(alkyle en C₁-C₄), et sachant que ledit alkyle en C₁-C₆ et les groupes fonctionnels alkyles (C₁-C₄) dans les groupes R₇ susmentionnés peuvent facultativement contenir une liaison carbone-carbone double ou triple ;
R₂ est de l'alkyle en C₁-C₁₂, de l'alkoxyalkyle en C₁-C₁₂, de l'aryle ou - (alkylène en C₁-C₄)aryle, sachant que ledit aryle est du phényle, du naphtyle, du thiényle, du benzothiényle, du pyridyle, du quinolyle, du pyrazinyle, du pyrimidyle, de l'imidazolyle, du furanyle, du benzofuranyle, du benzothiazolyle, de l'isothiazolyle, du benzisothiazolyle, du benzisoxazolyle, du benzimidazolyle, de l'indolyle, de l'oxadiazolyle ou du benzoxazolyle ;
du cycloalkyle ou-(alkylène en C₁-C₆)cycloalkyle présentant 3 à 8 chaînons, sachant qu'un ou deux des carbones cycliques dudit cycloalkyle présentant au moins 4 chaînons cycliques et du groupe fonctionnel cycloalkyle dudit -(alkylène en C₁-C₆)cycloalkyle présentant au moins 4 chaînons cycliques peuvent être facultativement remplacés par un atome d'oxygène ou de soufre ou par N-R₈, sachant que Rs est de l'hydrogène ou de l'alkyle en C₁-C₄ ;
et sachant que chacun des groupes R₂ susmentionnés peut facultativement être substitué par un à trois substituants sélectionnés indépendamment parmi du chloro, du fluoro et de l'alkyle en C₁-C₄, ou par un substituant sélectionné parmi du bromo, de l'iodo, de l'alkoxy en C₁-C₆, -O-CO-(alkyle en C₁-C₆), -O-CO-N(alkyle en C₁-C₄)(alkyle en C₁-C₂), -S(alkyle en C₁-C₆), CN, NO₂, -SO(alkyle en C₁-C₄), et - SO₂(alkyle en C₁-C₄), et sachant que ledit alkyle en C₁-C₁₂ et/ou le groupe fonctionnel alkylène en C₁-C₄ dudit -(alkylène en C₁-C₄)aryle peut facultativement contenir une liaison carbone-carbone double ou triple ;
ou -NR₁R₂ ou -CR₁R₂R₉ peut former un cycle saturé présentant 5 à 8 chaînons qui peut facultativement contenir une ou deux liaisons carbone-carbone doubles et/ou dans lequel un ou deux des carbones cycliques peuvent facultativement être remplacés par un atome d'oxygène, d'azote ou de soufre et qui peut être substitué par au moins un substituant ;
R₃ est du méthyle, de l'éthyle, du fluoro, du chloro, du bromo, de l'iodo, du cyano, du méthoxy, OCF₃, du méthylthio, du méthylsulfonyle, CH₂OH, ou CH₂OCH₃ ;
R₄ est de l'hydrogène, de l'alkyle en C₁-C₄, du fluoro, du chloro, du bromo, de l'iodo, de l'alkoxy en C₁-C₄, du trifluorométhoxy, -CH₂OCH₃, -CH₂OCH₂CH₃, - CH₂CH₂OCH₃, -CH₂OCF3, CF₃, de l'amino, du nitro, -NH(alkyle en C₁-C₄), - N(CH₃)₂, -NHCOCH₃ -NHCONHCH₃, -SOₙ(alkyle en C₁-C₄), sachant que n est 0, 1 ou 2, de l'hydroxy, -CO(alkyle en C₁-C₄), -CHO, du cyano ou -COO(alkyle en C₁-C₄), sachant que ledit alkyle en C₁-C₄ peut facultativement contenir une liaison double ou triple et/ou peut facultativement être substitué par un substituant sélectionné parmi de l'hydroxy, de l'amino, -NHCOCH₃, -NH(alkyle en C₁-C₂), - N(alkyle en C₁-C₂), -COO(alkyle en C₁-C₄), -CO(alkyle en C₁-C₄), de l'alkoxy en C₁-C₃, du thioalkyle en C₁-C₃, du fluoro, du chloro, du cyano et du nitro ;
R₅ est du phényle, du naphtyle, du thiényle, du benzothiényle, du pyridyle, du quinolyle, du pyrazinyle, du pyrimidyle, du furanyle, du benzofuranyle, du benzothiazolyle, ou de l'indolyle,
sachant que chacun des groupes R₅ ci-dessus est substitué par un à trois substituants sélectionnés indépendamment parmi du fluoro, du chloro, de l'alkyle en C₁-C₆ et de l'alkoxy en C₁-C₆, ou par un substituant sélectionné parmi de l'hydroxy, de l'iodo, du bromo, du formyle, du cyano, du nitro, du trifluorométhyle, de l'amino, (alkyle en C₁-C₆)O(C₁-C₆)alkyle, -NHCH₃, -N(CH₃)₂, -COOH, -COO(alkyle en C₁-C₄), -CO(alkyle en C₁-C₄), -SO₂NH(alkyle en C₁-C₄), -SO₂N(alkyle en C₁-C₄)(alkyle en C₁-C₂), -SO₂NH₂, -NHSO₂(alkyle en C₁-C₄), -S(alkyle en C₁-C₆) et SO₂(alkyle en C₁-C₆), et sachant que les groupes fonctionnels d'alkyle en C₁-C₄ et d'alkyle en C₁-C₆ des groupes R₅ susmentionnés peuvent facultativement être substitués par un ou deux groupes fluoro ou par un substituant sélectionné parmi de l'hydroxy, de l'amino, du méthylamino, du diméthylamino et de l'acétyle ;
R₆ est de l'hydrogène, du méthyle, du fluoro, du chloro, du bromo, de l'iodo, du cyano, de l'hydroxy, -O(alkyle en C₁-C₄), -C(O)(alkyle en C₁-C₄), -C(O)O(alkyle en C₁-C₄), -OCF₃, CF₃, -CH₂OH, -CH₂OCH₃ ou -CH₂OCH₂CH₃ ;
R₉ est de l'hydrogène, de l'hydroxy, du fluoro, ou du méthoxy ;
R₁₀ est de l'hydrogène ou de l'alkyle en C₁-C₄.

2. Antagoniste CRHR1 destiné à être utilisé selon la revendication 1, sachant que X₁ est CH.

3. Antagoniste CRHR1 destiné à être utilisé selon la revendication 1 ou 2, sachant que le cycle présentant 5 à 8 chaînons formé par -NR₁R₂ ou -CR₁R₂R₉ est substitué par au moins un substituant sélectionné parmi de l'alkyle en C₁-C₄ ou par un cycle présentant 4 à 8 chaînons, lequel peut être saturé ou peut contenir une à trois liaisons doubles et dans lequel un atome de carbone peut être remplacé par CO ou SO₂ et un à quatre atomes de carbone peuvent facultativement être remplacés par de l'azote.

4. Antagoniste CRHR1 destiné à être utilisé selon l'une quelconque des revendications 1 à 3, sachant que X₂ est -NHCHR₁R₂, -OCHR₁R₂ ou -NR₁R₂.

5. Antagoniste CRHR1 destiné à être utilisé selon la revendication 4, sachant que -NHCHR₁R₂ est -NHCH(CH₂OCH₃)₂, -NHCH(CH₂OCH₃)(CH₃CH₂), - NHCH(CH₂CH₃)₂, -NHCH(CH₂CH₂OCH₃)₂, -NHCH(CH₃)(CH₂CH₃) ou - NHCHR₁R₂, sachant que R₁ est de l'éthyle et R₂ est de l'oxadiozolyle substitué par du méthyle, de l'isopropyle, du cyclopropyle, du trifluorométhyle, ou de l'éthyle, ou
-NR₁R₂ est -N(CH₂CH₃)(CH₃), -N(CH₂CH₂CH₃)₂, -N(CH₂CH₂CH₃)(CH₂-cyclopropyle), -N(CH₂CH₃)(CH₂CH₂CH₂CH₃), -N(CH₂CH₂OCH₃)₂, ou - N(CH₂CH₂OCH₃)(CH₂CH₂CH₃), ou
-OCHR₁R₂ est -OCH(CH₂CH₃)₂, -OCH(CH₂CH₃)CH₃, - OCH(CH₂CH₃)(CH₂CH₂CH₃), -OCH(CH₂CH₃)(CH₂OCH₃).

6. Antagoniste CRHR1 destiné à être utilisé selon l'une quelconque des revendications 1 à 5, sachant que R₃ et R₄ sont du méthyle.

7. Antagoniste CRHR1 destiné à être utilisé selon l'une quelconque des revendications 1 à 6, sachant que X₃ est O.

8. Antagoniste CRHR1 destiné à être utilisé selon l'une quelconque des revendications 1 à 7, sachant que R₅ est du phényle substitué par un à trois substituants sélectionnés indépendamment dans le groupe constitué par CH₃, CH₂CH₃, OCH₃, Cl, F, CF₃.

9. Antagoniste CRHR1 destiné à être utilisé selon l'une quelconque des revendications 1 à 8, sachant que l'antagoniste CRHR1 est un composé de la formule (VI) 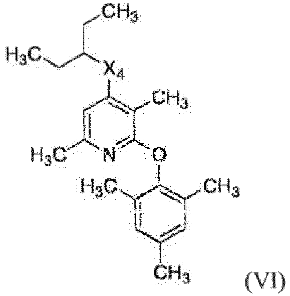 ou un sel pharmaceutiquement acceptable de celui-ci, sachant que X₄ est O ou NH.

10. Antagoniste CRHR1 destiné à être utilisé selon la revendication 1, sachant que l'antagoniste CRHR1 est sélectionné dans le groupe constitué par CP-316,311 et CP-376,395.

11. Antagoniste CRHR1 destiné à être utilisé selon les revendications 1 ou 10, sachant que le biomarqueur ou l'ensemble de biomarqueurs est sélectionné dans le groupe comprenant :
- SNP rs6437726,
- SNP rs1986684,
- SNP rs7380830,
- SNP rs3903768,
- SNP rs7325978,
- SNP rs13585,
- SNP rs9368373,
- SNP rs10935354,
- SNP rs8095703,
- SNP rs10206851,
- SNP rs9542977,
- SNP rs4942879,
- SNP rs9542954,
- SNP rs1593478,
- SNP rs9542951,
- SNP rs2188534,
- SNP rs12524124,
- SNP rs4352629,
- SNP rs7448716,
- SNP rs11873533,
- SNP rs10062658,
- SNP rs12547917,
- SNP rs1038268,
- SNP rs2375811,
- SNP rs1352671,
- SNP rs364331,
- SNP rs1924949,
- SNP rs11025990,
- SNP rs3758562, et/ou
- SNP rs10156056.

12. Antagoniste CRHR1 destiné à être utilisé selon l'une quelconque des revendications 1 à 11, sachant que le biomarqueur ou l'ensemble des biomarqueurs constituant un marqueur de l'activité de la CRH est sélectionné parmi un ou plusieurs biomarqueurs issus du groupe comprenant :
- SNP rs6437726 qui est représenté par un changement polymorphique simple en position 201 de SEQ ID N° 1, sachant que dans un ou deux allèles le nucléotide de type sauvage A est remplacé par un nucléotide indicateur G,
- SNP rs1986684 qui est représenté par un changement polymorphique simple en position 201 de SEQ ID N° 2, sachant que dans un ou deux allèles le nucléotide de type sauvage A est remplacé par un nucléotide indicateur G,
- SNP rs7380830 qui est représenté par un changement polymorphique simple en position 201 de SEQ ID N° 3, sachant que dans un ou deux allèles le nucléotide de type sauvage C est remplacé par un nucléotide indicateur T,
- SNP rs3903768 qui est représenté par un changement polymorphique simple en position 201 de SEQ ID N° 4, sachant que dans un ou deux allèles le nucléotide de type sauvage A est remplacé par un nucléotide indicateur G,
- SNP rs7325978 qui est représenté par un changement polymorphique simple en position 201 de SEQ ID N° 5, sachant que dans un ou deux allèles le nucléotide de type sauvage C est remplacé par un nucléotide indicateur T,
- SNP rs13585 qui est représenté par un changement polymorphique simple en position 185 de SEQ ID N° 6, sachant que dans un ou deux allèles le nucléotide de type sauvage C est remplacé par un nucléotide indicateur T,
- SNP rs9368373 qui est représenté par un changement polymorphique simple en position 201 de SEQ ID N° 7, sachant que dans un ou deux allèles le nucléotide de type sauvage C est remplacé par un nucléotide indicateur T,
- SNP rs 10935354 qui est représenté par un changement polymorphique simple en position 201 de SEQ ID N° 8, sachant que dans un ou deux allèles le nucléotide de type sauvage A est remplacé par un nucléotide indicateur G,
- SNP rs8095703 qui est représenté par un changement polymorphique simple en position 201 de SEQ ID N° 9, sachant que dans un ou deux allèles le nucléotide de type sauvage A est remplacé par un nucléotide indicateur G,
- SNP rs10206851 qui est représenté par un changement polymorphique simple en position 201 de SEQ ID N° 10, sachant que dans un ou deux allèles le nucléotide de type sauvage C est remplacé par un nucléotide indicateur T,
- SNP rs9542977 qui est représenté par un changement polymorphique simple en position 201 de SEQ ID N° 11, sachant que dans un ou deux allèles le nucléotide de type sauvage C est remplacé par un nucléotide indicateur T,
- SNP rs4942879 qui est représenté par un changement polymorphique simple en position 201 de SEQ ID N° 12, sachant que dans un ou deux allèles le nucléotide de type sauvage A est remplacé par un nucléotide indicateur G,
- SNP rs9542954 qui est représenté par un changement polymorphique simple en position 201 de SEQ ID N° 13, sachant que dans un ou deux allèles le nucléotide de type sauvage A est remplacé par un nucléotide indicateur C,
- SNP rs1593478 qui est représenté par un changement polymorphique simple en position 201 de SEQ ID N° 14, sachant que dans un ou deux allèles le nucléotide de type sauvage C est remplacé par un nucléotide indicateur T,
- SNP rs9542951 qui est représenté par un changement polymorphique simple en position 201 de SEQ ID N° 15, sachant que dans un ou deux allèles le nucléotide de type sauvage A est remplacé par un nucléotide indicateur G,
- SNP rs2188534 qui est représenté par un changement polymorphique simple en position 200 de SEQ ID N° 16, sachant que dans un ou deux allèles le nucléotide de type sauvage G est remplacé par un nucléotide indicateur T,
- SNP rs12524124 qui est représenté par un changement polymorphique simple en position 201 de SEQ ID N° 17, sachant que dans un ou deux allèles le nucléotide de type sauvage A est remplacé par un nucléotide indicateur G,
- SNP rs4352629 qui est représenté par un changement polymorphique simple en position 201 de SEQ ID N° 18, sachant que dans un ou deux allèles le nucléotide de type sauvage C est remplacé par un nucléotide indicateur T,
- SNP rs7448716 qui est représenté par un changement polymorphique simple en position 201 de SEQ ID N° 19, sachant que dans un ou deux allèles le nucléotide de type sauvage A est remplacé par un nucléotide indicateur G,
- SNP rs11873533 qui est représenté par un changement polymorphique simple en position 201 de SEQ ID N° 20, sachant que dans un ou deux allèles le nucléotide de type sauvage A est remplacé par un nucléotide indicateur C,
- SNP rs10062658 qui est représenté par un changement polymorphique simple en position 201 de SEQ ID N° 21, sachant que dans un ou deux allèles le nucléotide de type sauvage A est remplacé par un nucléotide indicateur G,
- SNP rs12547917 qui est représenté par un changement polymorphique simple en position 201 de SEQ ID N° 22, sachant que dans un ou deux allèles le nucléotide de type sauvage C est remplacé par un nucléotide indicateur T,
- SNP rs1038268 qui est représenté par un changement polymorphique simple en position 201 de SEQ ID N° 23, sachant que dans un ou deux allèles le nucléotide de type sauvage C est remplacé par un nucléotide indicateur T,
- SNP rs2375811 qui est représenté par un changement polymorphique simple en position 201 de SEQ ID N° 24, sachant que dans un ou deux allèles le nucléotide de type sauvage A est remplacé par un nucléotide indicateur G,
- SNP rs1352671 qui est représenté par un changement polymorphique simple en position 201 de SEQ ID N° 25, sachant que dans un ou deux allèles le nucléotide de type sauvage A est remplacé par un nucléotide indicateur C,
- SNP rs364331 qui est représenté par un changement polymorphique simple en position 201 de SEQ ID N° 26, sachant que dans un ou deux allèles le nucléotide de type sauvage A est remplacé par un nucléotide indicateur C,
- SNP rs1924949 qui est représenté par un changement polymorphique simple en position 201 de SEQ ID N° 27, sachant que dans un ou deux allèles le nucléotide de type sauvage A est remplacé par un nucléotide indicateur G,
- SNP rs11025990 qui est représenté par un changement polymorphique simple en position 201 de SEQ ID N° 28, sachant que dans un ou deux allèles le nucléotide de type sauvage A est remplacé par un nucléotide indicateur G,
- SNP rs3758562 qui est représenté par un changement polymorphique simple en position 201 de SEQ ID N° 29, sachant que dans un ou deux allèles le nucléotide de type sauvage A est remplacé par un nucléotide indicateur G, et/ou
- SNP rs10156056 qui est représenté par un changement polymorphique simple en position 201 de SEQ ID N° 30, sachant que dans un ou deux allèles le nucléotide de type sauvage C est remplacé par un nucléotide indicateur G.

13. Antagoniste CRHR1 destiné à être utilisé selon la revendication 11 ou 12, sachant que l'ensemble de biomarqueurs comprend au moins 15, au moins 20 ou au moins 25 des biomarqueurs tels que définis dans les revendications 11 ou 12.

14. Antagoniste CRHR1 destiné à être utilisé selon l'une quelconque des revendications 11 à 13, sachant que l'ensemble de biomarqueurs est constitué par les biomarqueurs tels que définis dans les revendications 11 ou 12.

15. Antagoniste CRHR1 destiné à être utilisé selon l'une quelconque des revendications 11 à 14, sachant que le marqueur est une combinaison d'un biomarqueur ou d'un ensemble de biomarqueurs tels que définis dans l'une quelconque des revendications 11 à 14 avec le biomarqueur densité REM.
